(19) Europäisches Patentamt
European Patent Office
Office européen des brevets

(11) **EP 2 616 099 B1**

(12) **EUROPEAN PATENT SPECIFICATION**

(45) Date of publication and mention
of the grant of the patent:
**30.05.2018 Bulletin 2018/22**

(51) Int Cl.:
***A61K 39/09*** (2006.01)

(21) Application number: **11768149.4**

(22) Date of filing: **16.09.2011**

(86) International application number:
**PCT/IB2011/054069**

(87) International publication number:
**WO 2012/035519 (22.03.2012 Gazette 2012/12)**

(54) **IMMUNOGENIC COMPOSITIONS**

IMMUNOGENE ZUSAMMENSETZUNGEN

COMPOSITIONS IMMUNOGÈNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2011 GB 201101665**
**16.09.2010 US 383668 P**

(43) Date of publication of application:
**24.07.2013 Bulletin 2013/30**

(73) Proprietor: **GlaxoSmithKline Biologicals SA
1330 Rixensart (BE)**

(72) Inventors:
• **BERTI, Francesco**
**I-53100 Siena (IT)**
• **CONTORNI, Mario**
**I-53100 Siena (IT)**
• **COSTANTINO, Paolo**
**I-53100 Siena (IT)**
• **FINCO, Oretta**
**I-53100 Siena (IT)**
• **GRANDI, Guido**
**I-53100 Siena (IT)**
• **MAIONE, Domenico**
**I-53100 Siena (IT)**
• **TELFORD, John**
**I-53100 Siena (IT)**

(74) Representative: **Courgeon, Antoine et al
GlaxoSmithKline
Global Patents CN925.1
980 Great West Road
Brentford, Middlesex TW8 9GS (GB)**

(56) References cited:
**WO-A1-94/06467          WO-A1-2004/011027
WO-A2-2004/041157**

• **PAOLETTI L C ET AL: "NEONATAL MOUSE
PROTECTION AGAINST INFECTION WITH
MULTIPLE GROUP B STREPTOCOCCAL (GBS)
SEROTYPES BY MATERNAL IMMUNIZATION
WITH A TETRAVALENT GBS
POLYSACCHARIDE-TETANUS TOXOID
CONJUGATE VACCINE", INFECTION AND
IMMUNITY, AMERICAN SOCIETY FOR
MICROBIOLOGY, WASHINGTON, US, vol. 62, no.
8, 1 August 1994 (1994-08-01) , pages 3236-3243,
XP001148813, ISSN: 0019-9567**
• **BAKER CAROL J ET AL: "Safety and
immunogenicity of a bivalent group B
streptococcal conjugate vaccine for serotypes II
and III.", JOURNAL OF INFECTIOUS DISEASES,
vol. 188, no. 1, 1 July 2003 (2003-07-01), pages
66-73, XP002666912, ISSN: 0022-1899**
• **BAKER ET AL: "Dose-response to type V group
B streptococcal polysaccharide-tetanus toxoid
conjugate vaccine in healthy adults", VACCINE,
ELSEVIER LTD, GB, vol. 25, no. 1, 8 December
2006 (2006-12-08), pages 55-63, XP005797684,
ISSN: 0264-410X, DOI:
10.1016/J.VACCINE.2006.07.018**
• **PALAZZI D L ET AL: "Use of type V group B
streptococcal conjugate vaccine in adults 65-85
years old", JOURNAL OF INFECTIOUS
DISEASES, UNIVERSITY OF CHICAGO PRESS,
CHICAGO, IL, vol. 190, 1 August 2004
(2004-08-01), pages 558-564, XP002997497, ISSN:
0022-1899, DOI: 10.1086/422010**

- BAKER C J ET AL: "Immunization of pregnant women with group B streptococcal type III capsular polysaccharide-tetanus toxoid conjugate vaccine", VACCINE, ELSEVIER LTD, GB, vol. 21, no. 24, 28 July 2003 (2003-07-28) , pages 3468-3472, XP004436458, ISSN: 0264-410X, DOI: 10.1016/S0264-410X(03)00353-0
- JOHRI ATUL KUMAR ET AL: "Group B Streptococcus: global incidence and vaccine development", NATURE REVIEWS. MICROBIOLOGY, NATURE PUBLISHING GROUP, GB, vol. 4, no. 12, 1 December 2006 (2006-12-01), pages 932-942, XP008095186, ISSN: 1740-1526, DOI: 10.1038/NRMICRO1552
- BLACK STEVEN ET AL: "Efficacy, safety and immunogenicity of heptavalent pneumococcal conjugate vaccine in children", THE PEDIATRIC INFECTIOUS DISEASE JOURNAL, LIPPINCOTT WILLIAMS & WILKINS, US, vol. 19, no. 3, 1 March 2000 (2000-03-01), pages 187-195, XP009105189, ISSN: 0891-3668, DOI: 10.1097/00006454-200003000-00003
- Lorna E Lancaster, Kevin Markey, Mei M Ho, Fatme Mawas and Michael J Corbel: "Structural and Immunological Characterisation of a Group B Streptococcus Conjugate Vaccine", , November 2009 (2009-11), Retrieved from the Internet: URL:http://www.meningitis.org/assets/x/523 05 [retrieved on 2015-01-23]

Remarks:

The file contains technical information submitted after the application was filed and not included in this specification

ï»¿SEQUENCE LISTING

<110> Novartis AG

<120> Immunogenic compositions

<130> P056312WO

<140> PCT/IB2011/_
<141> 2011-09-16

<150> US 61/383,668
<151> 2010-09-16

<150> GB 1101665.6
<151> 2011-01-31

<160> 26

<170> SeqWin2010, version 1.0

<210> 1
<211> 901
<212> PRT
<213> Streptococcus agalactiae serotype V strain 2603 V/R

<400> 1

```
Met Arg Lys Tyr Gln Lys Phe Ser Lys Ile Leu Thr Leu Ser Leu Phe
1               5               10              15

Cys Leu Ser Gln Ile Pro Leu Asn Thr Asn Val Leu Gly Glu Ser Thr
            20              25              30

Val Pro Glu Asn Gly Ala Lys Gly Lys Leu Val Val Lys Lys Thr Asp
        35              40              45

Asp Gln Asn Lys Pro Leu Ser Lys Ala Thr Phe Val Leu Lys Thr Thr
    50              55              60

Ala His Pro Glu Ser Lys Ile Glu Lys Val Thr Ala Glu Leu Thr Gly
65              70              75              80

Glu Ala Thr Phe Asp Asn Leu Ile Pro Gly Asp Tyr Thr Leu Ser Glu
            85              90              95

Glu Thr Ala Pro Glu Gly Tyr Lys Lys Thr Asn Gln Thr Trp Gln Val
        100             105             110

Lys Val Glu Ser Asn Gly Lys Thr Thr Ile Gln Asn Ser Gly Asp Lys
    115             120             125

Asn Ser Thr Ile Gly Gln Asn Gln Glu Glu Leu Asp Lys Gln Tyr Pro
    130             135             140

Pro Thr Gly Ile Tyr Glu Asp Thr Lys Glu Ser Tyr Lys Leu Glu His
145             150             155             160

Val Lys Gly Ser Val Pro Asn Gly Lys Ser Glu Ala Lys Ala Val Asn
            165             170             175

Pro Tyr Ser Ser Glu Gly Glu His Ile Arg Glu Ile Pro Glu Gly Thr
            180             185             190

Leu Ser Lys Arg Ile Ser Glu Val Gly Asp Leu Ala His Asn Lys Tyr
            195             200             205
```

```
Lys Ile Glu Leu Thr Val Ser Gly Lys Thr Ile Val Lys Pro Val Asp
    210                 215                 220

Lys Gln Lys Pro Leu Asp Val Val Phe Val Leu Asp Asn Ser Asn Ser
    225                 230                 235                 240

Met Asn Asn Asp Gly Pro Asn Phe Gln Arg His Asn Lys Ala Lys Lys
                245                 250                 255

Ala Ala Glu Ala Leu Gly Thr Ala Val Lys Asp Ile Leu Gly Ala Asn
                260                 265                 270

Ser Asp Asn Arg Val Ala Leu Val Thr Tyr Gly Ser Asp Ile Phe Asp
            275                 280                 285

Gly Arg Ser Val Asp Val Val Lys Gly Phe Lys Glu Asp Asp Lys Tyr
    290                 295                 300

Tyr Gly Leu Gln Thr Lys Phe Thr Ile Gln Thr Glu Asn Tyr Ser His
305                 310                 315                 320

Lys Gln Leu Thr Asn Asn Ala Glu Glu Ile Ile Lys Arg Ile Pro Thr
                325                 330                 335

Glu Ala Pro Lys Ala Lys Trp Gly Ser Thr Thr Asn Gly Leu Thr Pro
                340                 345                 350

Glu Gln Gln Lys Glu Tyr Tyr Leu Ser Lys Val Gly Glu Thr Phe Thr
                355                 360                 365

Met Lys Ala Phe Met Glu Ala Asp Asp Ile Leu Ser Gln Val Asn Arg
    370                 375                 380

Asn Ser Gln Lys Ile Ile Val His Val Thr Asp Gly Val Pro Thr Arg
385                 390                 395                 400

Ser Tyr Ala Ile Asn Asn Phe Lys Leu Gly Ala Ser Tyr Glu Ser Gln
                405                 410                 415

Phe Glu Gln Met Lys Lys Asn Gly Tyr Leu Asn Lys Ser Asn Phe Leu
                420                 425                 430

Leu Thr Asp Lys Pro Glu Asp Ile Lys Gly Asn Gly Glu Ser Tyr Phe
                435                 440                 445

Leu Phe Pro Leu Asp Ser Tyr Gln Thr Gln Ile Ile Ser Gly Asn Leu
    450                 455                 460

Gln Lys Leu His Tyr Leu Asp Leu Asn Leu Asn Tyr Pro Lys Gly Thr
465                 470                 475                 480

Ile Tyr Arg Asn Gly Pro Val Lys Glu His Gly Thr Pro Thr Lys Leu
                485                 490                 495

Tyr Ile Asn Ser Leu Lys Gln Lys Asn Tyr Asp Ile Phe Asn Phe Gly
                500                 505                 510

Ile Asp Ile Ser Gly Phe Arg Gln Val Tyr Asn Glu Glu Tyr Lys Lys
    515                 520                 525

Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
```

|  | 545 | | | | 550 | | | | 555 | | | | 560 | | |
|---|---|---|---|---|---|---|---|---|---|---|---|---|---|---|---|
| Glu | Tyr | Tyr | Thr | Pro | Ile | Val | Thr | Ser | Ala | Asp | Thr | Ser | Asn | Asn | Glu |
|  |  |  |  | 565 |  |  |  | 570 |  |  |  |  | 575 |  |  |

Ile Leu Ser Lys Ile Gln Gln Gln Phe Glu Thr Ile Leu Thr Lys Glu
        580                 585                 590

Asn Ser Ile Val Asn Gly Thr Ile Glu Asp Pro Met Gly Asp Lys Ile
        595                 600                 605

Asn Leu Gln Leu Gly Asn Gly Gln Thr Leu Gln Pro Ser Asp Tyr Thr
    610             615                 620

Leu Gln Gly Asn Asp Gly Ser Val Met Lys Asp Gly Ile Ala Thr Gly
625             630                 635                 640

Gly Pro Asn Asn Asp Gly Gly Ile Leu Lys Gly Val Lys Leu Glu Tyr
            645                 650                 655

Ile Gly Asn Lys Leu Tyr Val Arg Gly Leu Asn Leu Gly Glu Gly Gln
            660             665                 670

Lys Val Thr Leu Thr Tyr Asp Val Lys Leu Asp Asp Ser Phe Ile Ser
        675                 680                 685

Asn Lys Phe Tyr Asp Thr Asn Gly Arg Thr Thr Leu Asn Pro Lys Ser
    690                 695                 700

Glu Asp Pro Asn Thr Leu Arg Asp Phe Pro Ile Pro Lys Ile Arg Asp
705             710                 715                 720

Val Arg Glu Tyr Pro Thr Ile Thr Ile Lys Asn Glu Lys Lys Leu Gly
            725                 730                 735

Glu Ile Glu Phe Ile Lys Val Asp Lys Asp Asn Asn Lys Leu Leu Leu
            740                 745                 750

Lys Gly Ala Thr Phe Glu Leu Gln Glu Phe Asn Glu Asp Tyr Lys Leu
        755                 760                 765

Tyr Leu Pro Ile Lys Asn Asn Asn Ser Lys Val Val Thr Gly Glu Asn
    770                 775                 780

Gly Lys Ile Ser Tyr Lys Asp Leu Lys Asp Gly Lys Tyr Gln Leu Ile
785                 790                 795                 800

Glu Ala Val Ser Pro Glu Asp Tyr Gln Lys Ile Thr Asn Lys Pro Ile
            805                 810                 815

Leu Thr Phe Glu Val Val Lys Gly Ser Ile Lys Asn Ile Ile Ala Val
        820                 825                 830

Asn Lys Gln Ile Ser Glu Tyr His Glu Glu Gly Asp Lys His Leu Ile
    835                 840                 845

Thr Asn Thr His Ile Pro Pro Lys Gly Ile Ile Pro Met Thr Gly Gly
    850                 855                 860

Lys Gly Ile Leu Ser Phe Ile Leu Ile Gly Gly Ala Met Met Ser Ile
865                 870                 875                 880

Ala Gly Gly Ile Tyr Ile Trp Lys Arg Tyr Lys Lys Ser Ser Asp Met
            885                 890                 895

```
Ser Ile Lys Lys Asp
              900
```

<210> 2
<211> 554
<212> PRT
<213> Streptococcus agalactiae serotype V strain 2603 V/R

<400> 2

```
Met Lys Leu Ser Lys Lys Leu Leu Phe Ser Ala Ala Val Leu Thr Met
1               5               10              15

Val Ala Gly Ser Thr Val Glu Pro Val Ala Gln Phe Ala Thr Gly Met
        20              25              30

Ser Ile Val Arg Ala Ala Glu Val Ser Gln Glu Arg Pro Ala Lys Thr
        35              40              45

Thr Val Asn Ile Tyr Lys Leu Gln Ala Asp Ser Tyr Lys Ser Glu Ile
        50              55              60

Thr Ser Asn Gly Gly Ile Glu Asn Lys Asp Gly Glu Val Ile Ser Asn
65              70              75              80

Tyr Ala Lys Leu Gly Asp Asn Val Lys Gly Leu Gln Gly Val Gln Phe
            85              90              95

Lys Arg Tyr Lys Val Lys Thr Asp Ile Ser Val Asp Glu Leu Lys Lys
        100             105             110

Leu Thr Thr Val Glu Ala Ala Asp Ala Lys Val Gly Thr Ile Leu Glu
        115             120             125

Glu Gly Val Ser Leu Pro Gln Lys Thr Asn Ala Gln Gly Leu Val Val
    130             135             140

Asp Ala Leu Asp Ser Lys Ser Asn Val Arg Tyr Leu Tyr Val Glu Asp
145             150             155             160

Leu Lys Asn Ser Pro Ser Asn Ile Thr Lys Ala Tyr Ala Val Pro Phe
            165             170             175

Val Leu Glu Leu Pro Val Ala Asn Ser Thr Gly Thr Gly Phe Leu Ser
        180             185             190

Glu Ile Asn Ile Tyr Pro Lys Asn Val Val Thr Asp Glu Pro Lys Thr
        195             200             205

Asp Lys Asp Val Lys Lys Leu Gly Gln Asp Asp Ala Gly Tyr Thr Ile
    210             215             220

Gly Glu Glu Phe Lys Trp Phe Leu Lys Ser Thr Ile Pro Ala Asn Leu
225             230             235             240

Gly Asp Tyr Glu Lys Phe Glu Ile Thr Asp Lys Phe Ala Asp Gly Leu
            245             250             255

Thr Tyr Lys Ser Val Gly Lys Ile Lys Ile Gly Ser Lys Thr Leu Asn
        260             265             270

Arg Asp Glu His Tyr Thr Ile Asp Glu Pro Thr Val Asp Asn Gln Asn
    275             280             285

Thr Leu Lys Ile Thr Phe Lys Pro Glu Lys Phe Lys Glu Ile Ala Glu
    290             295             300
```

```
Leu Leu Lys Gly Met Thr Leu Val Lys Asn Gln Asp Ala Leu Asp Lys
305             310             315             320

Ala Thr Ala Asn Thr Asp Asp Ala Ala Phe Leu Glu Ile Pro Val Ala
            325             330             335

Ser Thr Ile Asn Glu Lys Ala Val Leu Gly Lys Ala Ile Glu Asn Thr
            340             345             350

Phe Glu Leu Gln Tyr Asp His Thr Pro Asp Lys Ala Asp Asn Pro Lys
            355             360             365

Pro Ser Asn Pro Pro Arg Lys Pro Glu Val His Thr Gly Gly Lys Arg
    370             375             380

Phe Val Lys Lys Asp Ser Thr Glu Thr Gln Thr Leu Gly Gly Ala Glu
385             390             395             400

Phe Asp Leu Leu Ala Ser Asp Gly Thr Ala Val Lys Trp Thr Asp Ala
            405             410             415

Leu Ile Lys Ala Asn Thr Asn Lys Asn Tyr Ile Ala Gly Glu Ala Val
            420             425             430

Thr Gly Gln Pro Ile Lys Leu Lys Ser His Thr Asp Gly Thr Phe Glu
            435             440             445

Ile Lys Gly Leu Ala Tyr Ala Val Asp Ala Asn Ala Glu Gly Thr Ala
    450             455             460

Val Thr Tyr Lys Leu Lys Glu Thr Lys Ala Pro Glu Gly Tyr Val Ile
465             470             475             480

Pro Asp Lys Glu Ile Glu Phe Thr Val Ser Gln Thr Ser Tyr Asn Thr
            485             490             495

Lys Pro Thr Asp Ile Thr Val Asp Ser Ala Asp Ala Thr Pro Asp Thr
            500             505             510

Ile Lys Asn Asn Lys Arg Pro Ser Ile Pro Asn Thr Gly Gly Ile Gly
            515             520             525

Thr Ala Ile Phe Val Ala Ile Gly Ala Ala Val Met Ala Phe Ala Val
    530             535             540

Lys Gly Met Lys Arg Arg Thr Lys Asp Asn
545             550
```

<210> 3
<211> 502
<212> PRT
<213> Streptococcus agalactiae serotype III strain COH1

<400> 3

Met Lys Lys Lys Met Ile Gln Ser Leu Leu Val Ala Ser Leu Ala Phe
1                   5                   10                  15

Gly Met Ala Val Ser Pro Val Thr Pro Ile Ala Phe Ala Ala Glu Thr
            20                  25                  30

Gly Thr Ile Thr Val Gln Asp Thr Gln Lys Gly Ala Thr Tyr Lys Ala
        35                  40                  45

Tyr Lys Val Phe Asp Ala Glu Ile Asp Asn Ala Asn Val Ser Asp Ser

EP 2 616 099 B1

```
                50                      55                         60

        Asn Lys Asp Gly Ala Ser Tyr Leu Ile Pro Gln Gly Lys Glu Ala Glu
        65                  70                  75                  80

        Tyr Lys Ala Ser Thr Asp Phe Asn Ser Leu Phe Thr Thr Thr Thr Asn
                        85                  90                  95

        Gly Gly Arg Thr Tyr Val Thr Lys Lys Asp Thr Ala Ser Ala Asn Glu
                    100                 105                 110

        Ile Ala Thr Trp Ala Lys Ser Ile Ser Ala Asn Thr Thr Pro Val Ser
                    115                 120                 125

        Thr Val Thr Glu Ser Asn Asn Asp Gly Thr Glu Val Ile Asn Val Ser
                130                 135                 140

        Gln Tyr Gly Tyr Tyr Tyr Val Ser Ser Thr Val Asn Asn Gly Ala Val
        145                 150                 155                 160

        Ile Met Val Thr Ser Val Thr Pro Asn Ala Thr Ile His Glu Lys Asn
                        165                 170                 175

        Thr Asp Ala Thr Trp Gly Asp Gly Gly Gly Lys Thr Val Asp Gln Lys
                    180                 185                 190

        Thr Tyr Ser Val Gly Asp Thr Val Lys Tyr Thr Ile Thr Tyr Lys Asn
                    195                 200                 205

        Ala Val Asn Tyr His Gly Thr Glu Lys Val Tyr Gln Tyr Val Ile Lys
            210                 215                 220

        Asp Thr Met Pro Ser Ala Ser Val Val Asp Leu Asn Glu Gly Ser Tyr
        225                 230                 235                 240

        Glu Val Thr Ile Thr Asp Gly Ser Gly Asn Ile Thr Thr Leu Thr Gln
                        245                 250                 255

        Gly Ser Glu Lys Ala Thr Gly Lys Tyr Asn Leu Leu Glu Glu Asn Asn
                    260                 265                 270

        Asn Phe Thr Ile Thr Ile Pro Trp Ala Ala Thr Asn Thr Pro Thr Gly
                    275                 280                 285

        Asn Thr Gln Asn Gly Ala Asn Asp Asp Phe Phe Tyr Lys Gly Ile Asn
                    290                 295                 300

        Thr Ile Thr Val Thr Tyr Thr Gly Val Leu Lys Ser Gly Ala Lys Pro
        305                 310                 315                 320

        Gly Ser Ala Asp Leu Pro Glu Asn Thr Asn Ile Ala Thr Ile Asn Pro
                    325                 330                 335

        Asn Thr Ser Asn Asp Asp Pro Gly Gln Lys Val Thr Val Arg Asp Gly
                    340                 345                 350

        Gln Ile Thr Ile Lys Lys Ile Asp Gly Ser Thr Lys Ala Ser Leu Gln
                    355                 360                 365

        Gly Ala Ile Phe Val Leu Lys Asn Ala Thr Gly Gln Phe Leu Asn Phe
                    370                 375                 380

        Asn Asp Thr Asn Asn Val Glu Trp Gly Thr Glu Ala Asn Ala Thr Glu
        385                 390                 395                 400
```

66

```
        Tyr Thr Thr Gly Ala Asp Gly Ile Ile Thr Ile Thr Gly Leu Lys Glu
                        405                 410                 415

        Gly Thr Tyr Tyr Leu Val Glu Lys Lys Ala Pro Leu Gly Tyr Asn Leu
                        420                 425                 430

        Leu Asp Asn Ser Gln Lys Val Ile Leu Gly Asp Gly Ala Thr Asp Thr
                        435                 440                 445

        Thr Asn Ser Asp Asn Leu Leu Val Asn Pro Thr Val Glu Asn Asn Lys
                        450                 455                 460

        Gly Thr Glu Leu Pro Ser Thr Gly Gly Ile Gly Thr Thr Ile Phe Tyr
        465                 470                 475                 480

        Ile Ile Gly Ala Ile Leu Val Ile Gly Ala Gly Ile Val Leu Val Ala
                        485                 490                 495

        Arg Arg Arg Leu Arg Ser
                        500
```

<210> 4
<211> 869
<212> PRT
<213> Artificial Sequence

<220>
<223> Amino acid sequence of GBS67 sequenced from Streptococcus agalactiae
serotype V strain 2603 V/R truncated before C-terminal transmembrane region

<400> 4

```
Met Arg Lys Tyr Gln Lys Phe Ser Lys Ile Leu Thr Leu Ser Leu Phe
1               5               10              15

Cys Leu Ser Gln Ile Pro Leu Asn Thr Asn Val Leu Gly Glu Ser Thr
            20              25              30

Val Pro Glu Asn Gly Ala Lys Gly Lys Leu Val Val Lys Lys Thr Asp
        35              40              45

Asp Gln Asn Lys Pro Leu Ser Lys Ala Thr Phe Val Leu Lys Thr Thr
    50              55              60

Ala His Pro Glu Ser Lys Ile Glu Lys Val Thr Ala Glu Leu Thr Gly
65              70              75              80

Glu Ala Thr Phe Asp Asn Leu Ile Pro Gly Asp Tyr Thr Leu Ser Glu
            85              90              95

Glu Thr Ala Pro Glu Gly Tyr Lys Lys Thr Asn Gln Thr Trp Gln Val
            100             105             110

Lys Val Glu Ser Asn Gly Lys Thr Thr Ile Gln Asn Ser Gly Asp Lys
        115             120             125

Asn Ser Thr Ile Gly Gln Asn Gln Glu Glu Leu Asp Lys Gln Tyr Pro
    130             135             140

Pro Thr Gly Ile Tyr Glu Asp Thr Lys Glu Ser Tyr Lys Leu Glu His
145             150             155             160

Val Lys Gly Ser Val Pro Asn Gly Lys Ser Glu Ala Lys Ala Val Asn
            165             170             175

Pro Tyr Ser Ser Glu Gly Glu His Ile Arg Glu Ile Pro Glu Gly Thr
```

```
                    180                      185                      190

        Leu Ser Lys Arg Ile Ser Glu Val Gly Asp Leu Ala His Asn Lys Tyr
                195                      200                  205

        Lys Ile Glu Leu Thr Val Ser Gly Lys Thr Ile Val Lys Pro Val Asp
                210                      215                  220

        Lys Gln Lys Pro Leu Asp Val Val Phe Val Leu Asp Asn Ser Asn Ser
        225                      230                  235                  240

        Met Asn Asn Asp Gly Pro Asn Phe Gln Arg His Asn Lys Ala Lys Lys
                        245                  250                      255

        Ala Ala Glu Ala Leu Gly Thr Ala Val Lys Asp Ile Leu Gly Ala Asn
                        260                  265                  270

        Ser Asp Asn Arg Val Ala Leu Val Thr Tyr Gly Ser Asp Ile Phe Asp
                    275                  280                  285

        Gly Arg Ser Val Asp Val Val Lys Gly Phe Lys Glu Asp Asp Lys Tyr
                290                  295                  300

        Tyr Gly Leu Gln Thr Lys Phe Thr Ile Gln Thr Glu Asn Tyr Ser His
        305                      310                  315                  320

        Lys Gln Leu Thr Asn Asn Ala Glu Glu Ile Ile Lys Arg Ile Pro Thr
                        325                  330                      335

        Glu Ala Pro Lys Ala Lys Trp Gly Ser Thr Thr Asn Gly Leu Thr Pro
                        340                  345                  350

        Glu Gln Gln Lys Glu Tyr Tyr Leu Ser Lys Val Gly Glu Thr Phe Thr
                    355                  360                  365

        Met Lys Ala Phe Met Glu Ala Asp Asp Ile Leu Ser Gln Val Asn Arg
                370                  375                  380

        Asn Ser Gln Lys Ile Ile Val His Val Thr Asp Gly Val Pro Thr Arg
        385                      390                  395                  400

        Ser Tyr Ala Ile Asn Asn Phe Lys Leu Gly Ala Ser Tyr Glu Ser Gln
                        405                  410                  415

        Phe Glu Gln Met Lys Lys Asn Gly Tyr Leu Asn Lys Ser Asn Phe Leu
                        420                  425                  430

        Leu Thr Asp Lys Pro Glu Asp Ile Lys Gly Asn Gly Glu Ser Tyr Phe
                    435                  440                  445

        Leu Phe Pro Leu Asp Ser Tyr Gln Thr Gln Ile Ile Ser Gly Asn Leu
                450                  455                  460

        Gln Lys Leu His Tyr Leu Asp Leu Asn Leu Asn Tyr Pro Lys Gly Thr
        465                      470                  475                  480

        Ile Tyr Arg Asn Gly Pro Val Lys Glu His Gly Thr Pro Thr Lys Leu
                        485                  490                  495

        Tyr Ile Asn Ser Leu Lys Gln Lys Asn Tyr Asp Ile Phe Asn Phe Gly
                    500                  505                  510

        Ile Asp Ile Ser Gly Phe Arg Gln Val Tyr Asn Glu Glu Tyr Lys Lys
                515                  520                  525
```

```
Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
545                 550                 555                 560

Glu Tyr Tyr Thr Pro Ile Val Thr Ser Ala Asp Thr Ser Asn Asn Glu
            565                 570                 575

Ile Leu Ser Lys Ile Gln Gln Gln Phe Glu Thr Ile Leu Thr Lys Glu
            580                 585                 590

Asn Ser Ile Val Asn Gly Thr Ile Glu Asp Pro Met Gly Asp Lys Ile
        595                 600                 605

Asn Leu Gln Leu Gly Asn Gly Gln Thr Leu Gln Pro Ser Asp Tyr Thr
    610                 615                 620

Leu Gln Gly Asn Asp Gly Ser Val Met Lys Asp Gly Ile Ala Thr Gly
625                 630                 635                 640

Gly Pro Asn Asn Asp Gly Gly Ile Leu Lys Gly Val Lys Leu Glu Tyr
            645                 650                 655

Ile Gly Asn Lys Leu Tyr Val Arg Gly Leu Asn Leu Gly Glu Gly Gln
            660                 665                 670

Lys Val Thr Leu Thr Tyr Asp Val Lys Leu Asp Asp Ser Phe Ile Ser
            675                 680                 685

Asn Lys Phe Tyr Asp Thr Asn Gly Arg Thr Thr Leu Asn Pro Lys Ser
    690                 695                 700

Glu Asp Pro Asn Thr Leu Arg Asp Phe Pro Ile Pro Lys Ile Arg Asp
705                 710                 715                 720

Val Arg Glu Tyr Pro Thr Ile Thr Ile Lys Asn Glu Lys Lys Leu Gly
            725                 730                 735

Glu Ile Glu Phe Ile Lys Val Asp Lys Asp Asn Asn Lys Leu Leu Leu
            740                 745                 750

Lys Gly Ala Thr Phe Glu Leu Gln Glu Phe Asn Glu Asp Tyr Lys Leu
            755                 760                 765

Tyr Leu Pro Ile Lys Asn Asn Asn Ser Lys Val Val Thr Gly Glu Asn
    770                 775                 780

Gly Lys Ile Ser Tyr Lys Asp Leu Lys Asp Gly Lys Tyr Gln Leu Ile
785                 790                 795                 800

Glu Ala Val Ser Pro Glu Asp Tyr Gln Lys Ile Thr Asn Lys Pro Ile
            805                 810                 815

Leu Thr Phe Glu Val Val Lys Gly Ser Ile Lys Asn Ile Ile Ala Val
            820                 825                 830

Asn Lys Gln Ile Ser Glu Tyr His Glu Glu Gly Asp Lys His Leu Ile
    835                 840                 845

Thr Asn Thr His Ile Pro Pro Lys Gly Ile Ile Pro Met Thr Gly Gly
    850                 855                 860

Lys Gly Ile Leu Ser
865
```

<210> 5
<211> 858
<212> PRT
<213> Artificial Sequence

<220>
<223> Amino acid sequence of GBS67 sequenced from Streptococcus agalactiae serotype V strain 2603 V/R lacking transmembrane and cell wall anchor motifs

<400> 5

```
Met Arg Lys Tyr Gln Lys Phe Ser Lys Ile Leu Thr Leu Ser Leu Phe
1             5             10                15

Cys Leu Ser Gln Ile Pro Leu Asn Thr Asn Val Leu Gly Glu Ser Thr
            20            25                30

Val Pro Glu Asn Gly Ala Lys Gly Lys Leu Val Val Lys Lys Thr Asp
        35            40            45

Asp Gln Asn Lys Pro Leu Ser Lys Ala Thr Phe Val Leu Lys Thr Thr
    50            55                60

Ala His Pro Glu Ser Lys Ile Glu Lys Val Thr Ala Glu Leu Thr Gly
65            70            75            80

Glu Ala Thr Phe Asp Asn Leu Ile Pro Gly Asp Tyr Thr Leu Ser Glu
            85            90                95

Glu Thr Ala Pro Glu Gly Tyr Lys Lys Thr Asn Gln Thr Trp Gln Val
        100           105           110

Lys Val Glu Ser Asn Gly Lys Thr Thr Ile Gln Asn Ser Gly Asp Lys
        115           120           125

Asn Ser Thr Ile Gly Gln Asn Gln Glu Glu Leu Asp Lys Gln Tyr Pro
    130           135           140

Pro Thr Gly Ile Tyr Glu Asp Thr Lys Glu Ser Tyr Lys Leu Glu His
145           150           155           160

Val Lys Gly Ser Val Pro Asn Gly Lys Ser Glu Ala Lys Ala Val Asn
            165           170           175

Pro Tyr Ser Ser Glu Gly Glu His Ile Arg Glu Ile Pro Glu Gly Thr
            180           185           190

Leu Ser Lys Arg Ile Ser Glu Val Gly Asp Leu Ala His Asn Lys Tyr
            195           200           205

Lys Ile Glu Leu Thr Val Ser Gly Lys Thr Ile Val Lys Pro Val Asp
    210           215           220

Lys Gln Lys Pro Leu Asp Val Val Phe Val Leu Asp Asn Ser Asn Ser
225           230           235           240

Met Asn Asn Asp Gly Pro Asn Phe Gln Arg His Asn Lys Ala Lys Lys
            245           250           255

Ala Ala Glu Ala Leu Gly Thr Ala Val Lys Asp Ile Leu Gly Ala Asn
            260           265           270

Ser Asp Asn Arg Val Ala Leu Val Thr Tyr Gly Ser Asp Ile Phe Asp
            275           280           285
```

```
Gly Arg Ser Val Asp Val Val Lys Gly Phe Lys Glu Asp Asp Lys Tyr
    290                 295                 300

Tyr Gly Leu Gln Thr Lys Phe Thr Ile Gln Thr Glu Asn Tyr Ser His
305                 310                 315                 320

Lys Gln Leu Thr Asn Asn Ala Glu Glu Ile Ile Lys Arg Ile Pro Thr
                325                 330                 335

Glu Ala Pro Lys Ala Lys Trp Gly Ser Thr Thr Asn Gly Leu Thr Pro
                340                 345                 350

Glu Gln Gln Lys Glu Tyr Tyr Leu Ser Lys Val Gly Glu Thr Phe Thr
                355                 360                 365

Met Lys Ala Phe Met Glu Ala Asp Asp Ile Leu Ser Gln Val Asn Arg
    370                 375                 380

Asn Ser Gln Lys Ile Ile Val His Val Thr Asp Gly Val Pro Thr Arg
385                 390                 395                 400

Ser Tyr Ala Ile Asn Asn Phe Lys Leu Gly Ala Ser Tyr Glu Ser Gln
                405                 410                 415

Phe Glu Gln Met Lys Lys Asn Gly Tyr Leu Asn Lys Ser Asn Phe Leu
                420                 425                 430

Leu Thr Asp Lys Pro Glu Asp Ile Lys Gly Asn Gly Glu Ser Tyr Phe
                435                 440                 445

Leu Phe Pro Leu Asp Ser Tyr Gln Thr Gln Ile Ile Ser Gly Asn Leu
    450                 455                 460

Gln Lys Leu His Tyr Leu Asp Leu Asn Leu Asn Tyr Pro Lys Gly Thr
465                 470                 475                 480

Ile Tyr Arg Asn Gly Pro Val Lys Glu His Gly Thr Pro Thr Lys Leu
                485                 490                 495

Tyr Ile Asn Ser Leu Lys Gln Lys Asn Tyr Asp Ile Phe Asn Phe Gly
                500                 505                 510

Ile Asp Ile Ser Gly Phe Arg Gln Val Tyr Asn Glu Glu Tyr Lys Lys
                515                 520                 525

Asn Gln Asp Gly Thr Phe Gln Lys Leu Lys Glu Glu Ala Phe Lys Leu
    530                 535                 540

Ser Asp Gly Glu Ile Thr Glu Leu Met Arg Ser Phe Ser Ser Lys Pro
545                 550                 555                 560

Glu Tyr Tyr Thr Pro Ile Val Thr Ser Ala Asp Thr Ser Asn Asn Glu
                565                 570                 575

Ile Leu Ser Lys Ile Gln Gln Gln Phe Glu Thr Ile Leu Thr Lys Glu
                580                 585                 590

Asn Ser Ile Val Asn Gly Thr Ile Glu Asp Pro Met Gly Asp Lys Ile
    595                 600                 605

Asn Leu Gln Leu Gly Asn Gly Gln Thr Leu Gln Pro Ser Asp Tyr Thr
    610                 615                 620

Leu Gln Gly Asn Asp Gly Ser Val Met Lys Asp Gly Ile Ala Thr Gly
625                 630                 635                 640
```

```
Gly Pro Asn Asn Asp Gly Gly Ile Leu Lys Gly Val Lys Leu Glu Tyr
                645             650             655

Ile Gly Asn Lys Leu Tyr Val Arg Gly Leu Asn Leu Gly Glu Gly Gln
                660             665             670

Lys Val Thr Leu Thr Tyr Asp Val Lys Leu Asp Asp Ser Phe Ile Ser
        675             680             685

Asn Lys Phe Tyr Asp Thr Asn Gly Arg Thr Thr Leu Asn Pro Lys Ser
    690             695             700

Glu Asp Pro Asn Thr Leu Arg Asp Phe Pro Ile Pro Lys Ile Arg Asp
705             710             715             720

Val Arg Glu Tyr Pro Thr Ile Thr Ile Lys Asn Glu Lys Lys Leu Gly
                725             730             735

Glu Ile Glu Phe Ile Lys Val Asp Lys Asp Asn Asn Lys Leu Leu Leu
            740             745             750

Lys Gly Ala Thr Phe Glu Leu Gln Glu Phe Asn Glu Asp Tyr Lys Leu
            755             760             765

Tyr Leu Pro Ile Lys Asn Asn Asn Ser Lys Val Val Thr Gly Glu Asn
    770             775             780

Gly Lys Ile Ser Tyr Lys Asp Leu Lys Asp Gly Lys Tyr Gln Leu Ile
785             790             795             800

Glu Ala Val Ser Pro Glu Asp Tyr Gln Lys Ile Thr Asn Lys Pro Ile
            805             810             815

Leu Thr Phe Glu Val Val Lys Gly Ser Ile Lys Asn Ile Ile Ala Val
            820             825             830

Asn Lys Gln Ile Ser Glu Tyr His Glu Glu Gly Asp Lys His Leu Ile
    835             840             845

Thr Asn Thr His Ile Pro Pro Lys Gly Ile
    850             855
```

<210> 6
<211> 517
<212> PRT
<213> Artificial Sequence

<220>
<223> Amino acid sequence of GBS80 sequenced from Streptococcus agalactiae
serotype V strain 2603 V/R lacking the N-terminal leader or signal sequence region

<400> 6

```
Ala Glu Val Ser Gln Glu Arg Pro Ala Lys Thr Thr Val Asn Ile Tyr
1               5               10                      15

Lys Leu Gln Ala Asp Ser Tyr Lys Ser Glu Ile Thr Ser Asn Gly Gly
            20              25                      30

Ile Glu Asn Lys Asp Gly Glu Val Ile Ser Asn Tyr Ala Lys Leu Gly
        35              40                      45

Asp Asn Val Lys Gly Leu Gln Gly Val Gln Phe Lys Arg Tyr Lys Val
    50              55                      60
```

Lys Thr Asp Ile Ser Val Asp Glu Leu Lys Lys Leu Thr Thr Val Glu
65 70 75 80

Ala Ala Asp Ala Lys Val Gly Thr Ile Leu Glu Glu Gly Val Ser Leu
85 90 95

Pro Gln Lys Thr Asn Ala Gln Gly Leu Val Val Asp Ala Leu Asp Ser
100 105 110

Lys Ser Asn Val Arg Tyr Leu Tyr Val Glu Asp Leu Lys Asn Ser Pro
115 120 125

Ser Asn Ile Thr Lys Ala Tyr Ala Val Pro Phe Val Leu Glu Leu Pro
130 135 140

Val Ala Asn Ser Thr Gly Thr Gly Phe Leu Ser Glu Ile Asn Ile Tyr
145 150 155 160

Pro Lys Asn Val Val Thr Asp Glu Pro Lys Thr Asp Lys Asp Val Lys
165 170 175

Lys Leu Gly Gln Asp Asp Ala Gly Tyr Thr Ile Gly Glu Glu Phe Lys
180 185 190

Trp Phe Leu Lys Ser Thr Ile Pro Ala Asn Leu Gly Asp Tyr Glu Lys
195 200 205

Phe Glu Ile Thr Asp Lys Phe Ala Asp Gly Leu Thr Tyr Lys Ser Val
210 215 220

Gly Lys Ile Lys Ile Gly Ser Lys Thr Leu Asn Arg Asp Glu His Tyr
225 230 235 240

Thr Ile Asp Glu Pro Thr Val Asp Asn Gln Asn Thr Leu Lys Ile Thr
245 250 255

Phe Lys Pro Glu Lys Phe Lys Glu Ile Ala Glu Leu Leu Lys Gly Met
260 265 270

Thr Leu Val Lys Asn Gln Asp Ala Leu Asp Lys Ala Thr Ala Asn Thr
275 280 285

Asp Asp Ala Ala Phe Leu Glu Ile Pro Val Ala Ser Thr Ile Asn Glu
290 295 300

Lys Ala Val Leu Gly Lys Ala Ile Glu Asn Thr Phe Glu Leu Gln Tyr
305 310 315 320

Asp His Thr Pro Asp Lys Ala Asp Asn Pro Lys Pro Ser Asn Pro Pro
325 330 335

Arg Lys Pro Glu Val His Thr Gly Gly Lys Arg Phe Val Lys Lys Asp
340 345 350

Ser Thr Glu Thr Gln Thr Leu Gly Gly Ala Glu Phe Asp Leu Leu Ala
355 360 365

Ser Asp Gly Thr Ala Val Lys Trp Thr Asp Ala Leu Ile Lys Ala Asn
370 375 380

Thr Asn Lys Asn Tyr Ile Ala Gly Glu Ala Val Thr Gly Gln Pro Ile
385 390 395 400

Lys Leu Lys Ser His Thr Asp Gly Thr Phe Glu Ile Lys Gly Leu Ala

```
                      405                    410                    415
      Tyr Ala Val Asp Ala Asn Ala Glu Gly Thr Ala Val Thr Tyr Lys Leu
                  420                    425                    430

      Lys Glu Thr Lys Ala Pro Glu Gly Tyr Val Ile Pro Asp Lys Glu Ile
                  435                    440                    445

      Glu Phe Thr Val Ser Gln Thr Ser Tyr Asn Thr Lys Pro Thr Asp Ile
          450                    455                    460

      Thr Val Asp Ser Ala Asp Ala Thr Pro Asp Thr Ile Lys Asn Asn Lys
      465                    470                    475                    480

      Arg Pro Ser Ile Pro Asn Thr Gly Gly Ile Gly Thr Ala Ile Phe Val
                  485                    490                    495

      Ala Ile Gly Ala Ala Val Met Ala Phe Ala Val Lys Gly Met Lys Arg
                  500                    505                    510

      Arg Thr Lys Asp Asn
                  515
```

<210> 7
<211> 525
<212> PRT
<213> Artificial Sequence

<220>
<223> Amino acid sequence of GBS80 sequenced from Streptococcus agalactiae
serotype V strain 2603 V/R lacking the C-terminal transmembrane region and downstream amino acids

<400> 7

Met Lys Leu Ser Lys Lys Leu Leu Phe Ser Ala Ala Val Leu Thr Met
1               5               10              15

Val Ala Gly Ser Thr Val Glu Pro Val Ala Gln Phe Ala Thr Gly Met
          20              25              30

Ser Ile Val Arg Ala Ala Glu Val Ser Gln Glu Arg Pro Ala Lys Thr
          35              40              45

Thr Val Asn Ile Tyr Lys Leu Gln Ala Asp Ser Tyr Lys Ser Glu Ile
          50              55              60

Thr Ser Asn Gly Gly Ile Glu Asn Lys Asp Gly Glu Val Ile Ser Asn
65              70              75              80

Tyr Ala Lys Leu Gly Asp Asn Val Lys Gly Leu Gln Gly Val Gln Phe
              85              90              95

Lys Arg Tyr Lys Val Lys Thr Asp Ile Ser Val Asp Glu Leu Lys Lys
          100             105             110

Leu Thr Thr Val Glu Ala Ala Asp Ala Lys Val Gly Thr Ile Leu Glu
          115             120             125

Glu Gly Val Ser Leu Pro Gln Lys Thr Asn Ala Gln Gly Leu Val Val
      130             135             140

Asp Ala Leu Asp Ser Lys Ser Asn Val Arg Tyr Leu Tyr Val Glu Asp
145             150             155             160

Leu Lys Asn Ser Pro Ser Asn Ile Thr Lys Ala Tyr Ala Val Pro Phe

|     |     | 165 |     |     |     |     |     | 170 |     |     |     |     |     | 175 |     |
| --- | --- | --- | --- | --- | --- | --- | --- | --- | --- | --- | --- | --- | --- | --- | --- |

Val Leu Glu Leu Pro Val Ala Asn Ser Thr Gly Thr Gly Phe Leu Ser
                180               185               190

Glu Ile Asn Ile Tyr Pro Lys Asn Val Val Thr Asp Glu Pro Lys Thr
        195               200               205

Asp Lys Asp Val Lys Lys Leu Gly Gln Asp Asp Ala Gly Tyr Thr Ile
    210               215               220

Gly Glu Glu Phe Lys Trp Phe Leu Lys Ser Thr Ile Pro Ala Asn Leu
225               230               235               240

Gly Asp Tyr Glu Lys Phe Glu Ile Thr Asp Lys Phe Ala Asp Gly Leu
                245               250               255

Thr Tyr Lys Ser Val Gly Lys Ile Lys Ile Gly Ser Lys Thr Leu Asn
                260               265               270

Arg Asp Glu His Tyr Thr Ile Asp Glu Pro Thr Val Asp Asn Gln Asn
                275               280               285

Thr Leu Lys Ile Thr Phe Lys Pro Glu Lys Phe Lys Glu Ile Ala Glu
    290               295               300

Leu Leu Lys Gly Met Thr Leu Val Lys Asn Gln Asp Ala Leu Asp Lys
305               310               315               320

Ala Thr Ala Asn Thr Asp Asp Ala Ala Phe Leu Glu Ile Pro Val Ala
                325               330               335

Ser Thr Ile Asn Glu Lys Ala Val Leu Gly Lys Ala Ile Glu Asn Thr
                340               345               350

Phe Glu Leu Gln Tyr Asp His Thr Pro Asp Lys Ala Asp Asn Pro Lys
        355               360               365

Pro Ser Asn Pro Pro Arg Lys Pro Glu Val His Thr Gly Gly Lys Arg
    370               375               380

Phe Val Lys Lys Asp Ser Thr Glu Thr Gln Thr Leu Gly Gly Ala Glu
385               390               395               400

Phe Asp Leu Leu Ala Ser Asp Gly Thr Ala Val Lys Trp Thr Asp Ala
                405               410               415

Leu Ile Lys Ala Asn Thr Asn Lys Asn Tyr Ile Ala Gly Glu Ala Val
                420               425               430

Thr Gly Gln Pro Ile Lys Leu Lys Ser His Thr Asp Gly Thr Phe Glu
        435               440               445

Ile Lys Gly Leu Ala Tyr Ala Val Asp Ala Asn Ala Glu Gly Thr Ala
    450               455               460

Val Thr Tyr Lys Leu Lys Glu Thr Lys Ala Pro Glu Gly Tyr Val Ile
465               470               475               480

Pro Asp Lys Glu Ile Glu Phe Thr Val Ser Gln Thr Ser Tyr Asn Thr
                485               490               495

Lys Pro Thr Asp Ile Thr Val Asp Ser Ala Asp Ala Thr Pro Asp Thr
                500               505               510

```
Ile Lys Asn Asn Lys Arg Pro Ser Ile Pro Asn Thr Gly
          515                 520                 525
```

<210> 8
<211> 520
<212> PRT
<213> Artificial Sequence

<220>
<223> Amino acid sequence of GBS80 sequenced from Streptococcus agalactiae
serotype V strain 2603 V/R lacking the transmembrane and cytoplasmic regions and the cell wall anchor motif

<400> 8

```
Met Lys Leu Ser Lys Lys Leu Leu Phe Ser Ala Ala Val Leu Thr Met
1               5               10              15

Val Ala Gly Ser Thr Val Glu Pro Val Ala Gln Phe Ala Thr Gly Met
        20              25              30

Ser Ile Val Arg Ala Ala Glu Val Ser Gln Glu Arg Pro Ala Lys Thr
        35              40              45

Thr Val Asn Ile Tyr Lys Leu Gln Ala Asp Ser Tyr Lys Ser Glu Ile
        50              55              60

Thr Ser Asn Gly Gly Ile Glu Asn Lys Asp Gly Glu Val Ile Ser Asn
65              70              75              80

Tyr Ala Lys Leu Gly Asp Asn Val Lys Gly Leu Gln Gly Val Gln Phe
                85              90              95

Lys Arg Tyr Lys Val Lys Thr Asp Ile Ser Val Asp Glu Leu Lys Lys
            100             105             110

Leu Thr Thr Val Glu Ala Ala Asp Ala Lys Val Gly Thr Ile Leu Glu
        115             120             125

Glu Gly Val Ser Leu Pro Gln Lys Thr Asn Ala Gln Gly Leu Val Val
    130             135             140

Asp Ala Leu Asp Ser Lys Ser Asn Val Arg Tyr Leu Tyr Val Glu Asp
145             150             155             160

Leu Lys Asn Ser Pro Ser Asn Ile Thr Lys Ala Tyr Ala Val Pro Phe
                165             170             175

Val Leu Glu Leu Pro Val Ala Asn Ser Thr Gly Thr Gly Phe Leu Ser
        180             185             190

Glu Ile Asn Ile Tyr Pro Lys Asn Val Val Thr Asp Glu Pro Lys Thr
        195             200             205

Asp Lys Asp Val Lys Lys Leu Gly Gln Asp Asp Ala Gly Tyr Thr Ile
    210             215             220

Gly Glu Glu Phe Lys Trp Phe Leu Lys Ser Thr Ile Pro Ala Asn Leu
225             230             235             240

Gly Asp Tyr Glu Lys Phe Glu Ile Thr Asp Lys Phe Ala Asp Gly Leu
            245             250             255

Thr Tyr Lys Ser Val Gly Lys Ile Lys Ile Gly Ser Lys Thr Leu Asn
            260             265             270
```

```
Arg Asp Glu His Tyr Thr Ile Asp Glu Pro Thr Val Asp Asn Gln Asn
        275             280             285

Thr Leu Lys Ile Thr Phe Lys Pro Glu Lys Phe Lys Glu Ile Ala Glu
    290             295             300

Leu Leu Lys Gly Met Thr Leu Val Lys Asn Gln Asp Ala Leu Asp Lys
305             310             315             320

Ala Thr Ala Asn Thr Asp Asp Ala Ala Phe Leu Glu Ile Pro Val Ala
            325             330             335

Ser Thr Ile Asn Glu Lys Ala Val Leu Gly Lys Ala Ile Glu Asn Thr
        340             345             350

Phe Glu Leu Gln Tyr Asp His Thr Pro Asp Lys Ala Asp Asn Pro Lys
        355             360             365

Pro Ser Asn Pro Pro Arg Lys Pro Glu Val His Thr Gly Gly Lys Arg
    370             375             380

Phe Val Lys Lys Asp Ser Thr Glu Thr Gln Thr Leu Gly Gly Ala Glu
385             390             395             400

Phe Asp Leu Leu Ala Ser Asp Gly Thr Ala Val Lys Trp Thr Asp Ala
            405             410             415

Leu Ile Lys Ala Asn Thr Asn Lys Asn Tyr Ile Ala Gly Glu Ala Val
            420             425             430

Thr Gly Gln Pro Ile Lys Leu Lys Ser His Thr Asp Gly Thr Phe Glu
            435             440             445

Ile Lys Gly Leu Ala Tyr Ala Val Asp Ala Asn Ala Glu Gly Thr Ala
    450             455             460

Val Thr Tyr Lys Leu Lys Glu Thr Lys Ala Pro Glu Gly Tyr Val Ile
465             470             475             480

Pro Asp Lys Glu Ile Glu Phe Thr Val Ser Gln Thr Ser Tyr Asn Thr
            485             490             495

Lys Pro Thr Asp Ile Thr Val Asp Ser Ala Asp Ala Thr Pro Asp Thr
            500             505             510

Ile Lys Asn Asn Lys Arg Pro Ser
            515             520
```

<210> 9
<211> 483
<212> PRT
<213> Artificial Sequence

<220>
<223> Amino acid sequence of GBS80 sequenced from Streptococcus agalactiae serotype V strain 2603 V/R lacking the leader or signal sequence region, the transmembrane and cytoplasmic regions and the cell wall anchor motif

<400> 9

Ala Glu Val Ser Gln Glu Arg Pro Ala Lys Thr Thr Val Asn Ile Tyr
1               5                   10                  15

Lys Leu Gln Ala Asp Ser Tyr Lys Ser Glu Ile Thr Ser Asn Gly Gly
            20                  25                  30

```
Ile Glu Asn Lys Asp Gly Glu Val Ile Ser Asn Tyr Ala Lys Leu Gly
         35                  40                  45

Asp Asn Val Lys Gly Leu Gln Gly Val Gln Phe Lys Arg Tyr Lys Val
         50                  55                  60

Lys Thr Asp Ile Ser Val Asp Glu Leu Lys Lys Leu Thr Thr Val Glu
65                  70                  75                  80

Ala Ala Asp Ala Lys Val Gly Thr Ile Leu Glu Glu Gly Val Ser Leu
              85                  90                  95

Pro Gln Lys Thr Asn Ala Gln Gly Leu Val Val Asp Ala Leu Asp Ser
             100                 105                 110

Lys Ser Asn Val Arg Tyr Leu Tyr Val Glu Asp Leu Lys Asn Ser Pro
             115                 120                 125

Ser Asn Ile Thr Lys Ala Tyr Ala Val Pro Phe Val Leu Glu Leu Pro
         130                 135                 140

Val Ala Asn Ser Thr Gly Thr Gly Phe Leu Ser Glu Ile Asn Ile Tyr
145                 150                 155                 160

Pro Lys Asn Val Val Thr Asp Glu Pro Lys Thr Asp Lys Asp Val Lys
                 165                 170                 175

Lys Leu Gly Gln Asp Asp Ala Gly Tyr Thr Ile Gly Glu Glu Phe Lys
             180                 185                 190

Trp Phe Leu Lys Ser Thr Ile Pro Ala Asn Leu Gly Asp Tyr Glu Lys
             195                 200                 205

Phe Glu Ile Thr Asp Lys Phe Ala Asp Gly Leu Thr Tyr Lys Ser Val
         210                 215                 220

Gly Lys Ile Lys Ile Gly Ser Lys Thr Leu Asn Arg Asp Glu His Tyr
225                 230                 235                 240

Thr Ile Asp Glu Pro Thr Val Asp Asn Gln Asn Thr Leu Lys Ile Thr
                 245                 250                 255

Phe Lys Pro Glu Lys Phe Lys Glu Ile Ala Glu Leu Leu Lys Gly Met
             260                 265                 270

Thr Leu Val Lys Asn Gln Asp Ala Leu Asp Lys Ala Thr Ala Asn Thr
             275                 280                 285

Asp Asp Ala Ala Phe Leu Glu Ile Pro Val Ala Ser Thr Ile Asn Glu
         290                 295                 300

Lys Ala Val Leu Gly Lys Ala Ile Glu Asn Thr Phe Glu Leu Gln Tyr
305                 310                 315                 320

Asp His Thr Pro Asp Lys Ala Asp Asn Pro Lys Pro Ser Asn Pro Pro
             325                 330                 335

Arg Lys Pro Glu Val His Thr Gly Gly Lys Arg Phe Val Lys Lys Asp
             340                 345                 350

Ser Thr Glu Thr Gln Thr Leu Gly Gly Ala Glu Phe Asp Leu Leu Ala
             355                 360                 365

Ser Asp Gly Thr Ala Val Lys Trp Thr Asp Ala Leu Ile Lys Ala Asn
         370                 375                 380
```

```
Thr Asn Lys Asn Tyr Ile Ala Gly Glu Ala Val Thr Gly Gln Pro Ile
385                 390                 395                 400

Lys Leu Lys Ser His Thr Asp Gly Thr Phe Glu Ile Lys Gly Leu Ala
            405                 410                 415

Tyr Ala Val Asp Ala Asn Ala Glu Gly Thr Ala Val Thr Tyr Lys Leu
            420                 425                 430

Lys Glu Thr Lys Ala Pro Glu Gly Tyr Val Ile Pro Asp Lys Glu Ile
            435                 440                 445

Glu Phe Thr Val Ser Gln Thr Ser Tyr Asn Thr Lys Pro Thr Asp Ile
    450                 455                 460

Thr Val Asp Ser Ala Asp Ala Thr Pro Asp Thr Ile Lys Asn Asn Lys
465                 470                 475                 480

Arg Pro Ser
```

<210> 10
<211> 271
<212> PRT
<213> Artificial Sequence

<220>
<223> A particularly immunogenic fragment of GBS80 located towards the N-terminus of the protein

<400> 10

```
Ala Glu Val Ser Gln Glu Arg Pro Ala Lys Thr Thr Val Asn Ile Tyr
1               5               10                  15

Lys Leu Gln Ala Asp Ser Tyr Lys Ser Glu Ile Thr Ser Asn Gly Gly
        20              25                  30

Ile Glu Asn Lys Asp Gly Glu Val Ile Ser Asn Tyr Ala Lys Leu Gly
        35              40                  45

Asp Asn Val Lys Gly Leu Gln Gly Val Gln Phe Lys Arg Tyr Lys Val
    50              55              60

Lys Thr Asp Ile Ser Val Asp Glu Leu Lys Lys Leu Thr Thr Val Glu
65              70              75                  80

Ala Ala Asp Ala Lys Val Gly Thr Ile Leu Glu Glu Gly Val Ser Leu
            85              90                  95

Pro Gln Lys Thr Asn Ala Gln Gly Leu Val Val Asp Ala Leu Asp Ser
        100             105             110

Lys Ser Asn Val Arg Tyr Leu Tyr Val Glu Asp Leu Lys Asn Ser Pro
    115             120             125

Ser Asn Ile Thr Lys Ala Tyr Ala Val Pro Phe Val Leu Glu Leu Pro
    130             135             140

Val Ala Asn Ser Thr Gly Thr Gly Phe Leu Ser Glu Ile Asn Ile Tyr
145             150             155                 160

Pro Lys Asn Val Val Thr Asp Glu Pro Lys Thr Asp Lys Asp Val Lys
            165             170             175

Lys Leu Gly Gln Asp Asp Ala Gly Tyr Thr Ile Gly Glu Glu Phe Lys
        180             185             190

Trp Phe Leu Lys Ser Thr Ile Pro Ala Asn Leu Gly Asp Tyr Glu Lys
        195             200             205

Phe Glu Ile Thr Asp Lys Phe Ala Asp Gly Leu Thr Tyr Lys Ser Val
    210             215             220

Gly Lys Ile Lys Ile Gly Ser Lys Thr Leu Asn Arg Asp Glu His Tyr
225             230             235                 240

Thr Ile Asp Glu Pro Thr Val Asp Asn Gln Asn Thr Leu Lys Ile Thr
            245             250             255

Phe Lys Pro Glu Lys Phe Lys Glu Ile Ala Glu Leu Leu Lys Gly
            260             265             270
```

<210> 11

<211> 473

<212> PRT

<213> Artificial Sequence

<220>

<223> Amino acid sequence of SpbI from Streptococcus agalactiae serotype III strain COH1 lacking the N-terminal leader or signal sequence region

<400> 11

```
Ala Glu Thr Gly Thr Ile Thr Val Gln Asp Thr Gln Lys Gly Ala Thr
1               5               10              15

Tyr Lys Ala Tyr Lys Val Phe Asp Ala Glu Ile Asp Asn Ala Asn Val
        20              25              30

Ser Asp Ser Asn Lys Asp Gly Ala Ser Tyr Leu Ile Pro Gln Gly Lys
        35              40              45

Glu Ala Glu Tyr Lys Ala Ser Thr Asp Phe Asn Ser Leu Phe Thr Thr
    50              55              60

Thr Thr Asn Gly Gly Arg Thr Tyr Val Thr Lys Lys Asp Thr Ala Ser
65              70              75              80

Ala Asn Glu Ile Ala Thr Trp Ala Lys Ser Ile Ser Ala Asn Thr Thr
            85              90              95

Pro Val Ser Thr Val Thr Glu Ser Asn Asn Asp Gly Thr Glu Val Ile
        100             105             110

Asn Val Ser Gln Tyr Gly Tyr Tyr Tyr Val Ser Ser Thr Val Asn Asn
        115             120             125

Gly Ala Val Ile Met Val Thr Ser Val Thr Pro Asn Ala Thr Ile His
    130             135             140

Glu Lys Asn Thr Asp Ala Thr Trp Gly Asp Gly Gly Lys Thr Val
145             150             155             160

Asp Gln Lys Thr Tyr Ser Val Gly Asp Thr Val Lys Tyr Thr Ile Thr
            165             170             175

Tyr Lys Asn Ala Val Asn Tyr His Gly Thr Glu Lys Val Tyr Gln Tyr
            180             185             190

Val Ile Lys Asp Thr Met Pro Ser Ala Ser Val Val Asp Leu Asn Glu
```

EP 2 616 099 B1

195                    200                    205

Gly Ser Tyr Glu Val Thr Ile Thr Asp Gly Ser Gly Asn Ile Thr Thr
    210                215            220

Leu Thr Gln Gly Ser Glu Lys Ala Thr Gly Lys Tyr Asn Leu Leu Glu
225            230                235                    240

Glu Asn Asn Asn Phe Thr Ile Thr Ile Pro Trp Ala Ala Thr Asn Thr
            245                250                    255

Pro Thr Gly Asn Thr Gln Asn Gly Ala Asn Asp Asp Phe Phe Tyr Lys
            260                265                270

Gly Ile Asn Thr Ile Thr Val Thr Tyr Thr Gly Val Leu Lys Ser Gly
            275                280                285

Ala Lys Pro Gly Ser Ala Asp Leu Pro Glu Asn Thr Asn Ile Ala Thr
    290                295                300

Ile Asn Pro Asn Thr Ser Asn Asp Asp Pro Gly Gln Lys Val Thr Val
305                310                315                320

Arg Asp Gly Gln Ile Thr Ile Lys Lys Ile Asp Gly Ser Thr Lys Ala
            325                330                335

Ser Leu Gln Gly Ala Ile Phe Val Leu Lys Asn Ala Thr Gly Gln Phe
            340                345                350

Leu Asn Phe Asn Asp Thr Asn Asn Val Glu Trp Gly Thr Glu Ala Asn
    355                360                365

Ala Thr Glu Tyr Thr Thr Gly Ala Asp Gly Ile Ile Thr Ile Thr Gly
    370                375                380

Leu Lys Glu Gly Thr Tyr Tyr Leu Val Glu Lys Lys Ala Pro Leu Gly
385                390                395                400

Tyr Asn Leu Leu Asp Asn Ser Gln Lys Val Ile Leu Gly Asp Gly Ala
            405                410                415

Thr Asp Thr Thr Asn Ser Asp Asn Leu Leu Val Asn Pro Thr Val Glu
            420                425                430

Asn Asn Lys Gly Thr Glu Leu Pro Ser Thr Gly Gly Ile Gly Thr Thr
            435                440                445

Ile Phe Tyr Ile Ile Gly Ala Ile Leu Val Ile Gly Ala Gly Ile Val
            450                455                460

Leu Val Ala Arg Arg Arg Leu Arg Ser
465                470

<210> 12
<211> 467
<212> PRT
<213> Artificial Sequence

<220>
<223> Amino acid sequence of SpbI from Streptococcus agalactiae serotype
III strain COH1 lacking the cell wall anchor motif and sequence C-terminal to this motif

<400> 12

Met Lys Lys Lys Met Ile Gln Ser Leu Leu Val Ala Ser Leu Ala Phe

```
      1                   5                   10                  15
      Gly Met Ala Val Ser Pro Val Thr Pro Ile Ala Phe Ala Ala Glu Thr
                  20                  25                  30

      Gly Thr Ile Thr Val Gln Asp Thr Gln Lys Gly Ala Thr Tyr Lys Ala
                  35                  40                  45

      Tyr Lys Val Phe Asp Ala Glu Ile Asp Asn Ala Asn Val Ser Asp Ser
          50                  55                  60

      Asn Lys Asp Gly Ala Ser Tyr Leu Ile Pro Gln Gly Lys Glu Ala Glu
      65                  70                  75                  80

      Tyr Lys Ala Ser Thr Asp Phe Asn Ser Leu Phe Thr Thr Thr Thr Asn
                  85                  90                  95

      Gly Gly Arg Thr Tyr Val Thr Lys Lys Asp Thr Ala Ser Ala Asn Glu
                  100                 105                 110

      Ile Ala Thr Trp Ala Lys Ser Ile Ser Ala Asn Thr Thr Pro Val Ser
                  115                 120                 125

      Thr Val Thr Glu Ser Asn Asn Asp Gly Thr Glu Val Ile Asn Val Ser
                  130                 135                 140

      Gln Tyr Gly Tyr Tyr Tyr Val Ser Ser Thr Val Asn Asn Gly Ala Val
      145                 150                 155                 160

      Ile Met Val Thr Ser Val Thr Pro Asn Ala Thr Ile His Glu Lys Asn
                  165                 170                 175

      Thr Asp Ala Thr Trp Gly Asp Gly Gly Gly Lys Thr Val Asp Gln Lys
                  180                 185                 190

      Thr Tyr Ser Val Gly Asp Thr Val Lys Tyr Thr Ile Thr Tyr Lys Asn
                  195                 200                 205

      Ala Val Asn Tyr His Gly Thr Glu Lys Val Tyr Gln Tyr Val Ile Lys
          210                 215                 220

      Asp Thr Met Pro Ser Ala Ser Val Val Asp Leu Asn Glu Gly Ser Tyr
      225                 230                 235                 240

      Glu Val Thr Ile Thr Asp Gly Ser Gly Asn Ile Thr Thr Leu Thr Gln
                  245                 250                 255

      Gly Ser Glu Lys Ala Thr Gly Lys Tyr Asn Leu Leu Glu Glu Asn Asn
                  260                 265                 270

      Asn Phe Thr Ile Thr Ile Pro Trp Ala Ala Thr Asn Thr Pro Thr Gly
                  275                 280                 285

      Asn Thr Gln Asn Gly Ala Asn Asp Asp Phe Phe Tyr Lys Gly Ile Asn
                  290                 295                 300

      Thr Ile Thr Val Thr Tyr Thr Gly Val Leu Lys Ser Gly Ala Lys Pro
      305                 310                 315                 320

      Gly Ser Ala Asp Leu Pro Glu Asn Thr Asn Ile Ala Thr Ile Asn Pro
                  325                 330                 335

      Asn Thr Ser Asn Asp Asp Pro Gly Gln Lys Val Thr Val Arg Asp Gly
                  340                 345                 350
```

```
        Gln Ile Thr Ile Lys Lys Ile Asp Gly Ser Thr Lys Ala Ser Leu Gln
                355                 360                 365

        Gly Ala Ile Phe Val Leu Lys Asn Ala Thr Gly Gln Phe Leu Asn Phe
                370                 375                 380

        Asn Asp Thr Asn Asn Val Glu Trp Gly Thr Glu Ala Asn Ala Thr Glu
        385                 390                 395                 400

        Tyr Thr Thr Gly Ala Asp Gly Ile Ile Thr Ile Thr Gly Leu Lys Glu
                        405                 410                 415

        Gly Thr Tyr Tyr Leu Val Glu Lys Lys Ala Pro Leu Gly Tyr Asn Leu
                        420                 425                 430

        Leu Asp Asn Ser Gln Lys Val Ile Leu Gly Asp Gly Ala Thr Asp Thr
                435                 440                 445

        Thr Asn Ser Asp Asn Leu Leu Val Asn Pro Thr Val Glu Asn Asn Lys
                450                 455                 460

        Gly Thr Glu
        465
```

<210> 13
<211> 438
<212> PRT
<213> Artificial Sequence

<220>
<223> Amino acid sequence of SpbI from Streptococcus agalactiae serotype
III strain COH1 lacking the leader or signal sequence region, the cell wall anchor motif and sequence C terminal to this motif

<400> 13

```
Ala Glu Thr Gly Thr Ile Thr Val Gln Asp Thr Gln Lys Gly Ala Thr
1               5                   10              15

Tyr Lys Ala Tyr Lys Val Phe Asp Ala Glu Ile Asp Asn Ala Asn Val
            20              25              30

Ser Asp Ser Asn Lys Asp Gly Ala Ser Tyr Leu Ile Pro Gln Gly Lys
            35              40              45

Glu Ala Glu Tyr Lys Ala Ser Thr Asp Phe Asn Ser Leu Phe Thr Thr
    50              55              60

Thr Thr Asn Gly Gly Arg Thr Tyr Val Thr Lys Lys Asp Thr Ala Ser
65              70              75              80

Ala Asn Glu Ile Ala Thr Trp Ala Lys Ser Ile Ser Ala Asn Thr Thr
            85              90              95

Pro Val Ser Thr Val Thr Glu Ser Asn Asn Asp Gly Thr Glu Val Ile
            100             105             110

Asn Val Ser Gln Tyr Gly Tyr Tyr Tyr Val Ser Ser Thr Val Asn Asn
            115             120             125

Gly Ala Val Ile Met Val Thr Ser Val Thr Pro Asn Ala Thr Ile His
    130             135             140

Glu Lys Asn Thr Asp Ala Thr Trp Gly Asp Gly Gly Gly Lys Thr Val
145             150             155             160
```

```
Asp Gln Lys Thr Tyr Ser Val Gly Asp Thr Val Lys Tyr Thr Ile Thr
            165             170             175

Tyr Lys Asn Ala Val Asn Tyr His Gly Thr Glu Lys Val Tyr Gln Tyr
        180             185             190

Val Ile Lys Asp Thr Met Pro Ser Ala Ser Val Val Asp Leu Asn Glu
        195             200             205

Gly Ser Tyr Glu Val Thr Ile Thr Asp Gly Ser Gly Asn Ile Thr Thr
    210             215             220

Leu Thr Gln Gly Ser Glu Lys Ala Thr Gly Lys Tyr Asn Leu Leu Glu
225             230             235             240

Glu Asn Asn Asn Phe Thr Ile Thr Ile Pro Trp Ala Ala Thr Asn Thr
            245             250             255

Pro Thr Gly Asn Thr Gln Asn Gly Ala Asn Asp Asp Phe Phe Tyr Lys
        260             265             270

Gly Ile Asn Thr Ile Thr Val Thr Tyr Thr Gly Val Leu Lys Ser Gly
        275             280             285

Ala Lys Pro Gly Ser Ala Asp Leu Pro Glu Asn Thr Asn Ile Ala Thr
    290             295             300

Ile Asn Pro Asn Thr Ser Asn Asp Asp Pro Gly Gln Lys Val Thr Val
305             310             315             320

Arg Asp Gly Gln Ile Thr Ile Lys Lys Ile Asp Gly Ser Thr Lys Ala
            325             330             335

Ser Leu Gln Gly Ala Ile Phe Val Leu Lys Asn Ala Thr Gly Gln Phe
        340             345             350

Leu Asn Phe Asn Asp Thr Asn Asn Val Glu Trp Gly Thr Glu Ala Asn
        355             360             365

Ala Thr Glu Tyr Thr Thr Gly Ala Asp Gly Ile Ile Thr Ile Thr Gly
    370             375             380

Leu Lys Glu Gly Thr Tyr Tyr Leu Val Glu Lys Lys Ala Pro Leu Gly
385             390             395             400

Tyr Asn Leu Leu Asp Asn Ser Gln Lys Val Ile Leu Gly Asp Gly Ala
            405             410             415

Thr Asp Thr Thr Asn Ser Asp Asn Leu Leu Val Asn Pro Thr Val Glu
        420             425             430

Asn Asn Lys Gly Thr Glu
        435
```

<210> 14
<211> 12
<212> DNA
<213> Artificial Sequence

<220>
<223> Nucleotide sequence upstream of internal methionine codon of

wild-type SpbI, containing core Shine-Dalgarno sequence

<400> 14
taatggagct gt        12

<210> 15
<211> 4
<212> PRT
<213> Artificial Sequence

<220>
<223> Linker

<400> 15

                    Gly Gly Gly Gly
                    1

<210> 16
<211> 14
<212> PRT
<213> Artificial Sequence

<220>
<223> Linker

<400> 16

Gly Gly Gly Gly Ser Gly Gly Gly Gly Ser Gly Gly Gly Gly
1                   5                   10

<210> 17
<211> 17
<212> PRT
<213> Artificial Sequence

<220>
<223> Linker

<400> 17

Gly Gly Gly Gly Ser Gly Gly Gly Gly Ser Gly Gly Gly Gly Ser Glu
1                   5                   10                  15

Leu

<210> 18
<211> 6
<212> PRT
<213> Artificial Sequence

<220>
<223> Linker

<400> 18

Gly Ser Gly Gly Gly Gly
1               5

<210> 19
<211> 3
<212> PRT
<213> Artificial Sequence

<220>
<223> W moiety

<400> 19

```
                                    Met Ala Ser
                                    1
```

<210> 20
<211> 6
<212> PRT
<213> Artificial Sequence

<220>
<223> Z moiety

<400> 20

```
                            His His His His His His
                            1                   5
```

<210> 21
<211> 941
<212> PRT
<213> Artificial Sequence

<220>
<223> Combination of W, X, L and Y moieties

<400> 21

```
Met Ala Ser Ala Glu Thr Gly Thr Ile Thr Val Gln Asp Thr Gln Lys
1               5               10              15

Gly Ala Thr Tyr Lys Ala Tyr Lys Val Phe Asp Ala Glu Ile Asp Asn
            20              25              30

Ala Asn Val Ser Asp Ser Asn Lys Asp Gly Ala Ser Tyr Leu Ile Pro
        35              40              45

Gln Gly Lys Glu Ala Glu Tyr Lys Ala Ser Thr Asp Phe Asn Ser Leu
    50              55              60

Phe Thr Thr Thr Thr Asn Gly Gly Arg Thr Tyr Val Thr Lys Lys Asp
65              70              75              80

Thr Ala Ser Ala Asn Glu Ile Ala Thr Trp Ala Lys Ser Ile Ser Ala
            85              90              95

Asn Thr Thr Pro Val Ser Thr Val Thr Glu Ser Asn Asn Asp Gly Thr
            100             105             110

Glu Val Ile Asn Val Ser Gln Tyr Gly Tyr Tyr Tyr Val Ser Ser Thr
        115             120             125

Val Asn Asn Gly Ala Val Ile Met Val Thr Ser Val Thr Pro Asn Ala
    130             135             140

Thr Ile His Glu Lys Asn Thr Asp Ala Thr Trp Gly Asp Gly Gly Gly
145             150             155             160

Lys Thr Val Asp Gln Lys Thr Tyr Ser Val Gly Asp Thr Val Lys Tyr
            165             170             175

Thr Ile Thr Tyr Lys Asn Ala Val Asn Tyr His Gly Thr Glu Lys Val
            180             185             190

Tyr Gln Tyr Val Ile Lys Asp Thr Met Pro Ser Ala Ser Val Val Asp
        195             200             205

Leu Asn Glu Gly Ser Tyr Glu Val Thr Ile Thr Asp Gly Ser Gly Asn
    210             215             220

Ile Thr Thr Leu Thr Gln Gly Ser Glu Lys Ala Thr Gly Lys Tyr Asn
225             230             235             240
```

```
Leu Leu Glu Glu Asn Asn Asn Phe Thr Ile Thr Ile Pro Trp Ala Ala
            245             250             255

Thr Asn Thr Pro Thr Gly Asn Thr Gln Asn Gly Ala Asn Asp Asp Phe
            260             265             270

Phe Tyr Lys Gly Ile Asn Thr Ile Thr Val Thr Tyr Thr Gly Val Leu
            275             280             285

Lys Ser Gly Ala Lys Pro Gly Ser Ala Asp Leu Pro Glu Asn Thr Asn
            290             295             300

Ile Ala Thr Ile Asn Pro Asn Thr Ser Asn Asp Asp Pro Gly Gln Lys
305             310             315             320

Val Thr Val Arg Asp Gly Gln Ile Thr Ile Lys Lys Ile Asp Gly Ser
            325             330             335

Thr Lys Ala Ser Leu Gln Gly Ala Ile Phe Val Leu Lys Asn Ala Thr
            340             345             350

Gly Gln Phe Leu Asn Phe Asn Asp Thr Asn Asn Val Glu Trp Gly Thr
            355             360             365

Glu Ala Asn Ala Thr Glu Tyr Thr Thr Gly Ala Asp Gly Ile Ile Thr
370             375             380

Ile Thr Gly Leu Lys Glu Gly Thr Tyr Tyr Leu Val Glu Lys Lys Ala
385             390             395             400

Pro Leu Gly Tyr Asn Leu Leu Asp Asn Ser Gln Lys Val Ile Leu Gly
            405             410             415

Asp Gly Ala Thr Asp Thr Thr Asn Ser Asp Asn Leu Leu Val Asn Pro
            420             425             430

Thr Val Glu Asn Asn Lys Gly Thr Glu Gly Gly Gly Gly Ser Gly Gly
            435             440             445

Gly Gly Ser Gly Gly Gly Gly Ser Glu Leu Ala Glu Val Ser Gln Glu
            450             455             460

Arg Pro Ala Lys Thr Thr Val Asn Ile Tyr Lys Leu Gln Ala Asp Ser
465             470             475             480

Tyr Lys Ser Glu Ile Thr Ser Asn Gly Gly Ile Glu Asn Lys Asp Gly
            485             490             495

Glu Val Ile Ser Asn Tyr Ala Lys Leu Gly Asp Asn Val Lys Gly Leu
            500             505             510

Gln Gly Val Gln Phe Lys Arg Tyr Lys Val Lys Thr Asp Ile Ser Val
            515             520             525

Asp Glu Leu Lys Lys Leu Thr Thr Val Glu Ala Ala Asp Ala Lys Val
            530             535             540

Gly Thr Ile Leu Glu Glu Gly Val Ser Leu Pro Gln Lys Thr Asn Ala
545             550             555             560

Gln Gly Leu Val Val Asp Ala Leu Asp Ser Lys Ser Asn Val Arg Tyr
            565             570             575

Leu Tyr Val Glu Asp Leu Lys Asn Ser Pro Ser Asn Ile Thr Lys Ala
            580             585             590
```

97

```
Tyr Ala Val Pro Phe Val Leu Glu Leu Pro Val Ala Asn Ser Thr Gly
        595             600             605

Thr Gly Phe Leu Ser Glu Ile Asn Ile Tyr Pro Lys Asn Val Val Thr
        610             615             620

Asp Glu Pro Lys Thr Asp Lys Asp Val Lys Lys Leu Gly Gln Asp Asp
625             630             635             640

Ala Gly Tyr Thr Ile Gly Glu Glu Phe Lys Trp Phe Leu Lys Ser Thr
            645             650             655

Ile Pro Ala Asn Leu Gly Asp Tyr Glu Lys Phe Glu Ile Thr Asp Lys
            660             665             670

Phe Ala Asp Gly Leu Thr Tyr Lys Ser Val Gly Lys Ile Lys Ile Gly
            675             680             685

Ser Lys Thr Leu Asn Arg Asp Glu His Tyr Thr Ile Asp Glu Pro Thr
        690             695             700

Val Asp Asn Gln Asn Thr Leu Lys Ile Thr Phe Lys Pro Glu Lys Phe
705             710             715             720

Lys Glu Ile Ala Glu Leu Leu Lys Gly Met Thr Leu Val Lys Asn Gln
            725             730             735

Asp Ala Leu Asp Lys Ala Thr Ala Asn Thr Asp Asp Ala Ala Phe Leu
            740             745             750

Glu Ile Pro Val Ala Ser Thr Ile Asn Glu Lys Ala Val Leu Gly Lys
        755             760             765

Ala Ile Glu Asn Thr Phe Glu Leu Gln Tyr Asp His Thr Pro Asp Lys
    770             775             780

Ala Asp Asn Pro Lys Pro Ser Asn Pro Pro Arg Lys Pro Glu Val His
785             790             795             800

Thr Gly Gly Lys Arg Phe Val Lys Lys Asp Ser Thr Glu Thr Gln Thr
            805             810             815

Leu Gly Gly Ala Glu Phe Asp Leu Leu Ala Ser Asp Gly Thr Ala Val
            820             825             830

Lys Trp Thr Asp Ala Leu Ile Lys Ala Asn Thr Asn Lys Asn Tyr Ile
            835             840             845

Ala Gly Glu Ala Val Thr Gly Gln Pro Ile Lys Leu Lys Ser His Thr
    850             855             860

Asp Gly Thr Phe Glu Ile Lys Gly Leu Ala Tyr Ala Val Asp Ala Asn
865             870             875             880

Ala Glu Gly Thr Ala Val Thr Tyr Lys Leu Lys Glu Thr Lys Ala Pro
            885             890             895

Glu Gly Tyr Val Ile Pro Asp Lys Glu Ile Glu Phe Thr Val Ser Gln
            900             905             910

Thr Ser Tyr Asn Thr Lys Pro Thr Asp Ile Thr Val Asp Ser Ala Asp
            915             920             925

Ala Thr Pro Asp Thr Ile Lys Asn Asn Lys Arg Pro Ser
```

98

930 935 940

<210> 22
<211> 947
<212> PRT
<213> Artificial Sequence

<220>
<223> Combination of W, X, L, Y and Z moieties

<400> 22

```
Met Ala Ser Ala Glu Thr Gly Thr Ile Thr Val Gln Asp Thr Gln Lys
1                   5                   10                  15

Gly Ala Thr Tyr Lys Ala Tyr Lys Val Phe Asp Ala Glu Ile Asp Asn
            20                  25                  30

Ala Asn Val Ser Asp Ser Asn Lys Asp Gly Ala Ser Tyr Leu Ile Pro
            35                  40                  45

Gln Gly Lys Glu Ala Glu Tyr Lys Ala Ser Thr Asp Phe Asn Ser Leu
        50                  55                  60

Phe Thr Thr Thr Thr Asn Gly Gly Arg Thr Tyr Val Thr Lys Lys Asp
65                  70                  75                  80

Thr Ala Ser Ala Asn Glu Ile Ala Thr Trp Ala Lys Ser Ile Ser Ala
            85                  90                  95

Asn Thr Thr Pro Val Ser Thr Val Thr Glu Ser Asn Asn Asp Gly Thr
            100                 105                 110

Glu Val Ile Asn Val Ser Gln Tyr Gly Tyr Tyr Tyr Val Ser Ser Thr
            115                 120                 125

Val Asn Asn Gly Ala Val Ile Met Val Thr Ser Val Thr Pro Asn Ala
    130                 135                 140

Thr Ile His Glu Lys Asn Thr Asp Ala Thr Trp Gly Asp Gly Gly Gly
145                 150                 155                 160

Lys Thr Val Asp Gln Lys Thr Tyr Ser Val Gly Asp Thr Val Lys Tyr
                165                 170                 175

Thr Ile Thr Tyr Lys Asn Ala Val Asn Tyr His Gly Thr Glu Lys Val
            180                 185                 190

Tyr Gln Tyr Val Ile Lys Asp Thr Met Pro Ser Ala Ser Val Val Asp
        195                 200                 205

Leu Asn Glu Gly Ser Tyr Glu Val Thr Ile Thr Asp Gly Ser Gly Asn
    210                 215                 220

Ile Thr Thr Leu Thr Gln Gly Ser Glu Lys Ala Thr Gly Lys Tyr Asn
225                 230                 235                 240

Leu Leu Glu Glu Asn Asn Asn Phe Thr Ile Thr Ile Pro Trp Ala Ala
            245                 250                 255

Thr Asn Thr Pro Thr Gly Asn Thr Gln Asn Gly Ala Asn Asp Asp Phe
            260                 265                 270

Phe Tyr Lys Gly Ile Asn Thr Ile Thr Val Thr Tyr Thr Gly Val Leu
        275                 280                 285
```

```
Lys Ser Gly Ala Lys Pro Gly Ser Ala Asp Leu Pro Glu Asn Thr Asn
    290             295             300

Ile Ala Thr Ile Asn Pro Asn Thr Ser Asn Asp Asp Pro Gly Gln Lys
305             310             315             320

Val Thr Val Arg Asp Gly Gln Ile Thr Ile Lys Lys Ile Asp Gly Ser
            325             330             335

Thr Lys Ala Ser Leu Gln Gly Ala Ile Phe Val Leu Lys Asn Ala Thr
            340             345             350

Gly Gln Phe Leu Asn Phe Asn Asp Thr Asn Asn Val Glu Trp Gly Thr
            355             360             365

Glu Ala Asn Ala Thr Glu Tyr Thr Thr Gly Ala Asp Gly Ile Ile Thr
    370             375             380

Ile Thr Gly Leu Lys Glu Gly Thr Tyr Tyr Leu Val Glu Lys Lys Ala
385             390             395             400

Pro Leu Gly Tyr Asn Leu Leu Asp Asn Ser Gln Lys Val Ile Leu Gly
            405             410             415

Asp Gly Ala Thr Asp Thr Thr Asn Ser Asp Asn Leu Leu Val Asn Pro
            420             425             430

Thr Val Glu Asn Asn Lys Gly Thr Glu Gly Gly Gly Gly Ser Gly Gly
            435             440             445

Gly Gly Ser Gly Gly Gly Gly Ser Glu Leu Ala Glu Val Ser Gln Glu
    450             455             460

Arg Pro Ala Lys Thr Thr Val Asn Ile Tyr Lys Leu Gln Ala Asp Ser
465             470             475             480

Tyr Lys Ser Glu Ile Thr Ser Asn Gly Gly Ile Glu Asn Lys Asp Gly
            485             490             495

Glu Val Ile Ser Asn Tyr Ala Lys Leu Gly Asp Asn Val Lys Gly Leu
            500             505             510

Gln Gly Val Gln Phe Lys Arg Tyr Lys Val Lys Thr Asp Ile Ser Val
            515             520             525

Asp Glu Leu Lys Lys Leu Thr Thr Val Glu Ala Ala Asp Ala Lys Val
    530             535             540

Gly Thr Ile Leu Glu Glu Gly Val Ser Leu Pro Gln Lys Thr Asn Ala
545             550             555             560

Gln Gly Leu Val Val Asp Ala Leu Asp Ser Lys Ser Asn Val Arg Tyr
            565             570             575

Leu Tyr Val Glu Asp Leu Lys Asn Ser Pro Ser Asn Ile Thr Lys Ala
            580             585             590

Tyr Ala Val Pro Phe Val Leu Glu Leu Pro Val Ala Asn Ser Thr Gly
            595             600             605

Thr Gly Phe Leu Ser Glu Ile Asn Ile Tyr Pro Lys Asn Val Val Thr
    610             615             620

Asp Glu Pro Lys Thr Asp Lys Asp Val Lys Lys Leu Gly Gln Asp Asp
625             630             635             640
```

```
Ala Gly Tyr Thr Ile Gly Glu Glu Phe Lys Trp Phe Leu Lys Ser Thr
            645             650             655

Ile Pro Ala Asn Leu Gly Asp Tyr Glu Lys Phe Glu Ile Thr Asp Lys
        660             665             670

Phe Ala Asp Gly Leu Thr Tyr Lys Ser Val Gly Lys Ile Lys Ile Gly
        675             680             685

Ser Lys Thr Leu Asn Arg Asp Glu His Tyr Thr Ile Asp Glu Pro Thr
    690             695             700

Val Asp Asn Gln Asn Thr Leu Lys Ile Thr Phe Lys Pro Glu Lys Phe
705             710             715             720

Lys Glu Ile Ala Glu Leu Leu Lys Gly Met Thr Leu Val Lys Asn Gln
            725             730             735

Asp Ala Leu Asp Lys Ala Thr Ala Asn Thr Asp Asp Ala Ala Phe Leu
            740             745             750

Glu Ile Pro Val Ala Ser Thr Ile Asn Glu Lys Ala Val Leu Gly Lys
        755             760             765

Ala Ile Glu Asn Thr Phe Glu Leu Gln Tyr Asp His Thr Pro Asp Lys
    770             775             780

Ala Asp Asn Pro Lys Pro Ser Asn Pro Pro Arg Lys Pro Glu Val His
785             790             795             800

Thr Gly Gly Lys Arg Phe Val Lys Lys Asp Ser Thr Glu Thr Gln Thr
            805             810             815

Leu Gly Gly Ala Glu Phe Asp Leu Leu Ala Ser Asp Gly Thr Ala Val
            820             825             830

Lys Trp Thr Asp Ala Leu Ile Lys Ala Asn Thr Asn Lys Asn Tyr Ile
            835             840             845

Ala Gly Glu Ala Val Thr Gly Gln Pro Ile Lys Leu Lys Ser His Thr
    850             855             860

Asp Gly Thr Phe Glu Ile Lys Gly Leu Ala Tyr Ala Val Asp Ala Asn
865             870             875             880

Ala Glu Gly Thr Ala Val Thr Tyr Lys Leu Lys Glu Thr Lys Ala Pro
            885             890             895

Glu Gly Tyr Val Ile Pro Asp Lys Glu Ile Glu Phe Thr Val Ser Gln
        900             905             910

Thr Ser Tyr Asn Thr Lys Pro Thr Asp Ile Thr Val Asp Ser Ala Asp
        915             920             925

Ala Thr Pro Asp Thr Ile Lys Asn Asn Lys Arg Pro Ser His His His
930             935             940

His His His
945
```

<210> 23

<211> 932
<212> PRT
<213> Artificial Sequence

<220>
<223> Combination of W, X, L and Y moieties

<400> 23

```
Met Ala Ser Ala Glu Thr Gly Thr Ile Thr Val Gln Asp Thr Gln Lys
1               5               10              15

Gly Ala Thr Tyr Lys Ala Tyr Lys Val Phe Asp Ala Glu Ile Asp Asn
        20              25              30

Ala Asn Val Ser Asp Ser Asn Lys Asp Gly Ala Ser Tyr Leu Ile Pro
        35              40              45

Gln Gly Lys Glu Ala Glu Tyr Lys Ala Ser Thr Asp Phe Asn Ser Leu
    50              55              60

Phe Thr Thr Thr Thr Asn Gly Gly Arg Thr Tyr Val Thr Lys Lys Asp
65              70              75              80

Thr Ala Ser Ala Asn Glu Ile Ala Thr Trp Ala Lys Ser Ile Ser Ala
            85              90              95

Asn Thr Thr Pro Val Ser Thr Val Thr Glu Ser Asn Asn Asp Gly Thr
            100             105             110

Glu Val Ile Asn Val Ser Gln Tyr Gly Tyr Tyr Tyr Val Ser Ser Thr
        115             120             125

Val Asn Asn Gly Ala Val Ile Met Val Thr Ser Val Thr Pro Asn Ala
    130             135             140

Thr Ile His Glu Lys Asn Thr Asp Ala Thr Trp Gly Asp Gly Gly Gly
145             150             155             160

Lys Thr Val Asp Gln Lys Thr Tyr Ser Val Gly Asp Thr Val Lys Tyr
            165             170             175

Thr Ile Thr Tyr Lys Asn Ala Val Asn Tyr His Gly Thr Glu Lys Val
        180             185             190

Tyr Gln Tyr Val Ile Lys Asp Thr Met Pro Ser Ala Ser Val Val Asp
        195             200             205

Leu Asn Glu Gly Ser Tyr Glu Val Thr Ile Thr Asp Gly Ser Gly Asn
    210             215             220

Ile Thr Thr Leu Thr Gln Gly Ser Glu Lys Ala Thr Gly Lys Tyr Asn
225             230             235             240

Leu Leu Glu Glu Asn Asn Asn Phe Thr Ile Thr Ile Pro Trp Ala Ala
            245             250             255

Thr Asn Thr Pro Thr Gly Asn Thr Gln Asn Gly Ala Asn Asp Asp Phe
            260             265             270

Phe Tyr Lys Gly Ile Asn Thr Ile Thr Val Thr Tyr Thr Gly Val Leu
    275             280             285

Lys Ser Gly Ala Lys Pro Gly Ser Ala Asp Leu Pro Glu Asn Thr Asn
    290             295             300

Ile Ala Thr Ile Asn Pro Asn Thr Ser Asn Asp Asp Pro Gly Gln Lys
305             310             315             320
```

Val Thr Val Arg Asp Gly Gln Ile Thr Ile Lys Lys Ile Asp Gly Ser
325 330 335

Thr Lys Ala Ser Leu Gln Gly Ala Ile Phe Val Leu Lys Asn Ala Thr
340 345 350

Gly Gln Phe Leu Asn Phe Asn Asp Thr Asn Asn Val Glu Trp Gly Thr
355 360 365

Glu Ala Asn Ala Thr Glu Tyr Thr Thr Gly Ala Asp Gly Ile Ile Thr
370 375 380

Ile Thr Gly Leu Lys Glu Gly Thr Tyr Tyr Leu Val Glu Lys Lys Ala
385 390 395 400

Pro Leu Gly Tyr Asn Leu Leu Asp Asn Ser Gln Lys Val Ile Leu Gly
405 410 415

Asp Gly Ala Thr Asp Thr Thr Asn Ser Asp Asn Leu Leu Val Asn Pro
420 425 430

Thr Val Glu Asn Asn Lys Gly Thr Glu Gly Ser Gly Gly Gly Gly Glu
435 440 445

Leu Ala Glu Val Ser Gln Glu Arg Pro Ala Lys Thr Thr Val Asn Ile
450 455 460

Tyr Lys Leu Gln Ala Asp Ser Tyr Lys Ser Glu Ile Thr Ser Asn Gly
465 470 475 480

Gly Ile Glu Asn Lys Asp Gly Glu Val Ile Ser Asn Tyr Ala Lys Leu
485 490 495

Gly Asp Asn Val Lys Gly Leu Gln Gly Val Gln Phe Lys Arg Tyr Lys
500 505 510

Val Lys Thr Asp Ile Ser Val Asp Glu Leu Lys Lys Leu Thr Thr Val
515 520 525

Glu Ala Ala Asp Ala Lys Val Gly Thr Ile Leu Glu Glu Gly Val Ser
530 535 540

Leu Pro Gln Lys Thr Asn Ala Gln Gly Leu Val Val Asp Ala Leu Asp
545 550 555 560

Ser Lys Ser Asn Val Arg Tyr Leu Tyr Val Glu Asp Leu Lys Asn Ser
565 570 575

Pro Ser Asn Ile Thr Lys Ala Tyr Ala Val Pro Phe Val Leu Glu Leu
580 585 590

Pro Val Ala Asn Ser Thr Gly Thr Gly Phe Leu Ser Glu Ile Asn Ile
595 600 605

Tyr Pro Lys Asn Val Val Thr Asp Glu Pro Lys Thr Asp Lys Asp Val
610 615 620

Lys Lys Leu Gly Gln Asp Asp Ala Gly Tyr Thr Ile Gly Glu Glu Phe
625 630 635 640

Lys Trp Phe Leu Lys Ser Thr Ile Pro Ala Asn Leu Gly Asp Tyr Glu
645 650 655

Lys Phe Glu Ile Thr Asp Lys Phe Ala Asp Gly Leu Thr Tyr Lys Ser
660 665 670

```
Val Gly Lys Ile Lys Ile Gly Ser Lys Thr Leu Asn Arg Asp Glu His
        675             680             685

Tyr Thr Ile Asp Glu Pro Thr Val Asp Asn Gln Asn Thr Leu Lys Ile
    690             695             700

Thr Phe Lys Pro Glu Lys Phe Lys Glu Ile Ala Glu Leu Leu Lys Gly
705             710             715             720

Met Thr Leu Val Lys Asn Gln Asp Ala Leu Asp Lys Ala Thr Ala Asn
            725             730             735

Thr Asp Asp Ala Ala Phe Leu Glu Ile Pro Val Ala Ser Thr Ile Asn
        740             745             750

Glu Lys Ala Val Leu Gly Lys Ala Ile Glu Asn Thr Phe Glu Leu Gln
        755             760             765

Tyr Asp His Thr Pro Asp Lys Ala Asp Asn Pro Lys Pro Ser Asn Pro
    770             775             780

Pro Arg Lys Pro Glu Val His Thr Gly Gly Lys Arg Phe Val Lys Lys
785             790             795             800

Asp Ser Thr Glu Thr Gln Thr Leu Gly Gly Ala Glu Phe Asp Leu Leu
            805             810             815

Ala Ser Asp Gly Thr Ala Val Lys Trp Thr Asp Ala Leu Ile Lys Ala
            820             825             830

Asn Thr Asn Lys Asn Tyr Ile Ala Gly Glu Ala Val Thr Gly Gln Pro
        835             840             845

Ile Lys Leu Lys Ser His Thr Asp Gly Thr Phe Glu Ile Lys Gly Leu
    850             855             860

Ala Tyr Ala Val Asp Ala Asn Ala Glu Gly Thr Ala Val Thr Tyr Lys
865             870             875             880

Leu Lys Glu Thr Lys Ala Pro Glu Gly Tyr Val Ile Pro Asp Lys Glu
            885             890             895

Ile Glu Phe Thr Val Ser Gln Thr Ser Tyr Asn Thr Lys Pro Thr Asp
            900             905             910

Ile Thr Val Asp Ser Ala Asp Ala Thr Pro Asp Thr Ile Lys Asn Asn
            915             920             925

Lys Arg Pro Ser
        930
```

<210> 24

<211> 896

<212> PRT

<213> Streptococcus agalactiae serotype Ib strain H36B

<400> 24

106

Met Arg Lys Tyr Gln Lys Phe Ser Lys Ile Leu Thr Leu Ser Leu Phe
1                   5                   10                  15

Cys Leu Ser Gln Ile Pro Leu Asn Thr Asn Val Leu Gly Glu Ser Thr
                20                  25                  30

Val Pro Glu Asn Gly Ala Lys Gly Lys Leu Val Val Lys Lys Thr Asp

```
                    35                      40                      45

        Asp Gln Asn Lys Pro Leu Ser Lys Ala Thr Phe Val Leu Lys Pro Thr
            50                  55                  60
        Ser His Ser Glu Ser Lys Val Glu Lys Val Thr Thr Glu Val Thr Gly
        65                  70                  75                  80
        Glu Ala Thr Phe Asp Asn Leu Thr Pro Gly Asp Tyr Thr Leu Ser Glu
                        85                  90                  95
        Glu Thr Ala Pro Glu Gly Tyr Lys Lys Thr Thr Gln Thr Trp Gln Val
                        100                 105                 110
        Lys Val Glu Ser Asn Gly Lys Thr Thr Ile Gln Asn Ser Asp Asp Lys
                        115                 120                 125
        Lys Ser Ile Ile Glu Gln Arg Gln Glu Glu Leu Asp Lys Gln Tyr Pro
            130                 135                 140
        Leu Thr Gly Ala Tyr Glu Asp Thr Lys Glu Ser Tyr Asn Leu Glu His
        145                 150                 155                 160
        Val Lys Asn Ser Ile Pro Asn Gly Lys Leu Glu Ala Lys Ala Val Asn
                        165                 170                 175
        Pro Tyr Ser Ser Glu Gly Glu His Ile Arg Glu Ile Gln Glu Gly Thr
                        180                 185                 190
        Leu Ser Lys Arg Ile Ser Glu Val Asn Asp Leu Asp His Asn Lys Tyr
                        195                 200                 205
        Lys Ile Glu Leu Thr Val Ser Gly Lys Ser Ile Ile Lys Thr Ile Asn
            210                 215                 220
        Lys Asp Glu Pro Leu Asp Val Val Phe Val Leu Asp Asn Ser Asn Ser
        225                 230                 235                 240
        Met Lys Asn Asn Gly Lys Asn Asn Lys Ala Lys Lys Ala Gly Glu Ala
                        245                 250                 255
        Val Glu Thr Ile Ile Lys Asp Val Leu Gly Ala Asn Val Glu Asn Arg
                        260                 265                 270
        Ala Ala Leu Val Thr Tyr Gly Ser Asp Ile Phe Asp Gly Arg Thr Val
                        275                 280                 285
        Lys Val Ile Lys Gly Phe Lys Glu Asp Pro Tyr Tyr Gly Leu Glu Thr
            290                 295                 300
        Ser Phe Thr Val Gln Thr Asn Asp Tyr Ser Tyr Lys Lys Phe Thr Asn
        305                 310                 315                 320
        Ile Ala Ala Asp Ile Ile Lys Lys Ile Pro Lys Glu Ala Pro Glu Ala
                        325                 330                 335
        Lys Trp Gly Gly Thr Ser Leu Gly Leu Thr Pro Glu Lys Lys Arg Glu
                        340                 345                 350
        Tyr Asp Leu Ser Lys Val Gly Glu Thr Phe Thr Met Lys Ala Phe Met
                        355                 360                 365
        Glu Ala Asp Thr Leu Leu Ser Ser Ile Gln Arg Lys Ser Arg Lys Ile
            370                 375                 380
```

```
Ile Val His Leu Thr Asp Gly Val Pro Thr Arg Ser Tyr Ala Ile Asn
385             390         395             400

Ser Phe Val Lys Gly Ser Thr Tyr Ala Asn Gln Phe Glu Arg Ile Lys
            405             410             415

Glu Lys Gly Tyr Leu Asp Lys Asn Asn Tyr Phe Ile Thr Asp Asp Pro
        420             425             430

Glu Lys Ile Lys Gly Asn Gly Glu Ser Tyr Phe Leu Phe Pro Leu Asp
        435             440             445

Ser Tyr Gln Thr Gln Ile Ile Ser Gly Asn Leu Gln Lys Leu His Tyr
    450             455             460

Leu Asp Leu Asn Leu Asn Tyr Pro Lys Gly Thr Ile Tyr Arg Asn Gly
465             470             475             480

Pro Val Arg Glu His Gly Thr Pro Thr Lys Leu Tyr Ile Asn Ser Leu
            485             490             495

Lys Gln Lys Asn Tyr Asp Ile Phe Asn Phe Gly Ile Asp Ile Ser Gly
            500             505             510

Phe Arg Gln Val Tyr Asn Glu Asp Tyr Lys Lys Asn Gln Asp Gly Thr
            515             520             525

Phe Gln Lys Leu Lys Glu Glu Ala Phe Glu Leu Ser Asp Gly Glu Ile
    530             535             540

Thr Glu Leu Met Asn Ser Phe Ser Ser Lys Pro Glu Tyr Tyr Thr Pro
545             550             555             560

Ile Val Thr Ser Ala Asp Val Ser Asn Asn Glu Ile Leu Ser Lys Ile
            565             570             575

Gln Gln Gln Phe Glu Lys Ile Leu Thr Lys Glu Asn Ser Ile Val Asn
            580             585             590

Gly Thr Ile Glu Asp Pro Met Gly Asp Lys Ile Asn Leu His Leu Gly
    595             600             605

Asn Gly Gln Thr Leu Gln Pro Ser Asp Tyr Thr Leu Gln Gly Asn Asp
    610             615             620

Gly Ser Ile Met Lys Asp Ser Ile Ala Thr Gly Gly Pro Asn Asn Asp
625             630             635             640

Gly Gly Ile Leu Lys Gly Val Lys Leu Glu Tyr Ile Lys Asn Lys Leu
            645             650             655

Tyr Val Arg Gly Leu Asn Leu Gly Glu Gly Gln Lys Val Thr Leu Thr
            660             665             670

Tyr Asp Val Lys Leu Asp Asp Ser Phe Ile Ser Asn Lys Phe Tyr Asp
    675             680             685

Thr Asn Gly Arg Thr Thr Leu Asn Pro Lys Ser Glu Glu Pro Asp Thr
    690             695             700

Leu Arg Asp Phe Pro Ile Pro Lys Ile Arg Asp Val Arg Glu Tyr Pro
705             710             715             720

Thr Ile Thr Ile Lys Asn Glu Lys Lys Leu Gly Glu Ile Glu Phe Thr
            725             730             735
```

```
Lys Val Asp Lys Asp Asn Asn Lys Leu Leu Leu Lys Gly Ala Thr Phe
            740                 745                 750

Glu Leu Gln Glu Phe Asn Glu Asp Tyr Lys Leu Tyr Leu Pro Ile Lys
            755                 760                 765

Asn Asn Asn Ser Lys Val Val Thr Gly Glu Asn Gly Lys Ile Ser Tyr
            770                 775                 780

Lys Asp Leu Lys Asp Gly Lys Tyr Gln Leu Ile Glu Ala Val Ser Pro
785                 790                 795                 800

Lys Asp Tyr Gln Lys Ile Thr Asn Lys Pro Ile Leu Thr Phe Glu Val
                805                 810                 815

Val Lys Gly Ser Ile Gln Asn Ile Ile Ala Val Asn Lys Gln Ile Ser
            820                 825                 830

Glu Tyr His Glu Glu Gly Asp Lys His Leu Ile Thr Asn Thr His Ile
        835                 840                 845

Pro Pro Lys Gly Ile Ile Pro Met Thr Gly Gly Lys Gly Ile Leu Ser
    850                 855                 860

Phe Ile Leu Ile Gly Gly Ala Met Met Ser Ile Ala Gly Gly Ile Tyr
865                 870                 875                 880

Ile Trp Lys Arg His Lys Lys Ser Ser Asp Ala Ser Ile Glu Lys Asp
                885                 890                 895
```

<210> 25
<211> 864
<212> PRT
<213> Artificial Sequence

<220>
<223> Amino acid sequence of GBS67 sequenced from Streptococcus agalactiae
serotype Ib strain H36B truncated before C-terminal transmembrane region

<400> 25

Met Arg Lys Tyr Gln Lys Phe Ser Lys Ile Leu Thr Leu Ser Leu Phe
1         5             10                15

Cys Leu Ser Gln Ile Pro Leu Asn Thr Asn Val Leu Gly Glu Ser Thr
        20             25                30

Val Pro Glu Asn Gly Ala Lys Gly Lys Leu Val Val Lys Lys Thr Asp
        35             40                45

Asp Gln Asn Lys Pro Leu Ser Lys Ala Thr Phe Val Leu Lys Pro Thr
        50             55                60

Ser His Ser Glu Ser Lys Val Glu Lys Val Thr Thr Glu Val Thr Gly
65             70                75                80

Glu Ala Thr Phe Asp Asn Leu Thr Pro Gly Asp Tyr Thr Leu Ser Glu
                85                90                95

Glu Thr Ala Pro Glu Gly Tyr Lys Lys Thr Thr Gln Thr Trp Gln Val
        100            105               110

Lys Val Glu Ser Asn Gly Lys Thr Thr Ile Gln Asn Ser Asp Asp Lys
        115            120               125

```
Lys Ser Ile Ile Glu Gln Arg Gln Glu Glu Leu Asp Lys Gln Tyr Pro
130             135             140
Leu Thr Gly Ala Tyr Glu Asp Thr Lys Glu Ser Tyr Asn Leu Glu His
145             150             155             160
Val Lys Asn Ser Ile Pro Asn Gly Lys Leu Glu Ala Lys Ala Val Asn
            165             170             175
Pro Tyr Ser Ser Glu Gly Glu His Ile Arg Glu Ile Gln Glu Gly Thr
            180             185             190
Leu Ser Lys Arg Ile Ser Glu Val Asn Asp Leu Asp His Asn Lys Tyr
            195             200             205
Lys Ile Glu Leu Thr Val Ser Gly Lys Ser Ile Ile Lys Thr Ile Asn
    210             215             220
Lys Asp Glu Pro Leu Asp Val Val Phe Val Leu Asp Asn Ser Asn Ser
225             230             235             240
Met Lys Asn Asn Gly Lys Asn Asn Lys Ala Lys Lys Ala Gly Glu Ala
            245             250             255
Val Glu Thr Ile Ile Lys Asp Val Leu Gly Ala Asn Val Glu Asn Arg
            260             265             270
Ala Ala Leu Val Thr Tyr Gly Ser Asp Ile Phe Asp Gly Arg Thr Val
            275             280             285
Lys Val Ile Lys Gly Phe Lys Glu Asp Pro Tyr Tyr Gly Leu Glu Thr
290             295             300
Ser Phe Thr Val Gln Thr Asn Asp Tyr Ser Tyr Lys Lys Phe Thr Asn
305             310             315             320
Ile Ala Ala Asp Ile Ile Lys Lys Ile Pro Lys Glu Ala Pro Glu Ala
            325             330             335
Lys Trp Gly Gly Thr Ser Leu Gly Leu Thr Pro Glu Lys Lys Arg Glu
            340             345             350
Tyr Asp Leu Ser Lys Val Gly Glu Thr Phe Thr Met Lys Ala Phe Met
            355             360             365
Glu Ala Asp Thr Leu Leu Ser Ser Ile Gln Arg Lys Ser Arg Lys Ile
            370             375             380
Ile Val His Leu Thr Asp Gly Val Pro Thr Arg Ser Tyr Ala Ile Asn
385             390             395             400
Ser Phe Val Lys Gly Ser Thr Tyr Ala Asn Gln Phe Glu Arg Ile Lys
            405             410             415
Glu Lys Gly Tyr Leu Asp Lys Asn Asn Tyr Phe Ile Thr Asp Asp Pro
            420             425             430
Glu Lys Ile Lys Gly Asn Gly Glu Ser Tyr Phe Leu Phe Pro Leu Asp
            435             440             445
Ser Tyr Gln Thr Gln Ile Ile Ser Gly Asn Leu Gln Lys Leu His Tyr
            450             455             460
Leu Asp Leu Asn Leu Asn Tyr Pro Lys Gly Thr Ile Tyr Arg Asn Gly
465             470             475             480
```

112

Pro Val Arg Glu His Gly Thr Pro Thr Lys Leu Tyr Ile Asn Ser Leu
                485                   490                   495

Lys Gln Lys Asn Tyr Asp Ile Phe Asn Phe Gly Ile Asp Ile Ser Gly
                500                   505                   510

Phe Arg Gln Val Tyr Asn Glu Asp Tyr Lys Lys Asn Gln Asp Gly Thr
            515                   520                   525

Phe Gln Lys Leu Lys Glu Glu Ala Phe Glu Leu Ser Asp Gly Glu Ile
            530                   535                   540

Thr Glu Leu Met Asn Ser Phe Ser Ser Lys Pro Glu Tyr Tyr Thr Pro
545                   550                   555                   560

Ile Val Thr Ser Ala Asp Val Ser Asn Asn Glu Ile Leu Ser Lys Ile
                565                   570                   575

Gln Gln Gln Phe Glu Lys Ile Leu Thr Lys Glu Asn Ser Ile Val Asn
                580                   585                   590

Gly Thr Ile Glu Asp Pro Met Gly Asp Lys Ile Asn Leu His Leu Gly
                595                   600                   605

Asn Gly Gln Thr Leu Gln Pro Ser Asp Tyr Thr Leu Gln Gly Asn Asp
            610                   615                   620

Gly Ser Ile Met Lys Asp Ser Ile Ala Thr Gly Gly Pro Asn Asn Asp
625                   630                   635                   640

Gly Gly Ile Leu Lys Gly Val Lys Leu Glu Tyr Ile Lys Asn Lys Leu
                645                   650                   655

Tyr Val Arg Gly Leu Asn Leu Gly Glu Gly Gln Lys Val Thr Leu Thr
                660                   665                   670

Tyr Asp Val Lys Leu Asp Asp Ser Phe Ile Ser Asn Lys Phe Tyr Asp
            675                   680                   685

Thr Asn Gly Arg Thr Thr Leu Asn Pro Lys Ser Glu Glu Pro Asp Thr
            690                   695                   700

Leu Arg Asp Phe Pro Ile Pro Lys Ile Arg Asp Val Arg Glu Tyr Pro
705                   710                   715                   720

Thr Ile Thr Ile Lys Asn Glu Lys Lys Leu Gly Glu Ile Glu Phe Thr
                725                   730                   735

Lys Val Asp Lys Asp Asn Asn Lys Leu Leu Leu Lys Gly Ala Thr Phe
                740                   745                   750

Glu Leu Gln Glu Phe Asn Glu Asp Tyr Lys Leu Tyr Leu Pro Ile Lys
            755                   760                   765

Asn Asn Asn Ser Lys Val Val Thr Gly Glu Asn Gly Lys Ile Ser Tyr
            770                   775                   780

Lys Asp Leu Lys Asp Gly Lys Tyr Gln Leu Ile Glu Ala Val Ser Pro
785                   790                   795                   800

Lys Asp Tyr Gln Lys Ile Thr Asn Lys Pro Ile Leu Thr Phe Glu Val
                805                   810                   815

Val Lys Gly Ser Ile Gln Asn Ile Ile Ala Val Asn Lys Gln Ile Ser

```
                   820                    825                       830

        Glu Tyr His Glu Glu Gly Asp Lys His Leu Ile Thr Asn Thr His Ile
                835                 840                 845

        Pro Pro Lys Gly Ile Ile Pro Met Thr Gly Gly Lys Gly Ile Leu Ser
            850                 855                 860
```

<210> 26
<211> 853
<212> PRT
<213> Artificial Sequence

<220>
<223> Amino acid sequence of GBS67 sequenced from Streptococcus agalactiae
serotype Ib strain H36B lacking transmembrane and cell wall anchor motifs

<400> 26

```
Met Arg Lys Tyr Gln Lys Phe Ser Lys Ile Leu Thr Leu Ser Leu Phe
1                 5                 10                15

Cys Leu Ser Gln Ile Pro Leu Asn Thr Asn Val Leu Gly Glu Ser Thr
            20                25                30

Val Pro Glu Asn Gly Ala Lys Gly Lys Leu Val Val Lys Lys Thr Asp
        35                40                45

Asp Gln Asn Lys Pro Leu Ser Lys Ala Thr Phe Val Leu Lys Pro Thr
        50                55                60

Ser His Ser Glu Ser Lys Val Glu Lys Val Thr Thr Glu Val Thr Gly
65                70                75                80

Glu Ala Thr Phe Asp Asn Leu Thr Pro Gly Asp Tyr Thr Leu Ser Glu
                85                90                95

Glu Thr Ala Pro Glu Gly Tyr Lys Lys Thr Thr Gln Thr Trp Gln Val
            100               105               110

Lys Val Glu Ser Asn Gly Lys Thr Thr Ile Gln Asn Ser Asp Asp Lys
        115               120               125

Lys Ser Ile Ile Glu Gln Arg Gln Glu Glu Leu Asp Lys Gln Tyr Pro
    130               135               140

Leu Thr Gly Ala Tyr Glu Asp Thr Lys Glu Ser Tyr Asn Leu Glu His
145               150               155               160

Val Lys Asn Ser Ile Pro Asn Gly Lys Leu Glu Ala Lys Ala Val Asn
            165               170               175

Pro Tyr Ser Ser Glu Gly Glu His Ile Arg Glu Ile Gln Glu Gly Thr
        180               185               190

Leu Ser Lys Arg Ile Ser Glu Val Asn Asp Leu Asp His Asn Lys Tyr
    195               200               205

Lys Ile Glu Leu Thr Val Ser Gly Lys Ser Ile Ile Lys Thr Ile Asn
    210               215               220

Lys Asp Glu Pro Leu Asp Val Val Phe Val Leu Asp Asn Ser Asn Ser
225               230               235               240

Met Lys Asn Asn Gly Lys Asn Asn Lys Ala Lys Lys Ala Gly Glu Ala
            245               250               255
```

Val Glu Thr Ile Ile Lys Asp Val Leu Gly Ala Asn Val Glu Asn Arg
        260             265             270

Ala Ala Leu Val Thr Tyr Gly Ser Asp Ile Phe Asp Gly Arg Thr Val
        275             280             285

Lys Val Ile Lys Gly Phe Lys Glu Asp Pro Tyr Tyr Gly Leu Glu Thr
        290             295             300

Ser Phe Thr Val Gln Thr Asn Asp Tyr Ser Tyr Lys Lys Phe Thr Asn
305             310             315             320

Ile Ala Ala Asp Ile Ile Lys Lys Ile Pro Lys Glu Ala Pro Glu Ala
        325             330             335

Lys Trp Gly Gly Thr Ser Leu Gly Leu Thr Pro Glu Lys Lys Arg Glu
        340             345             350

Tyr Asp Leu Ser Lys Val Gly Glu Thr Phe Thr Met Lys Ala Phe Met
        355             360             365

Glu Ala Asp Thr Leu Leu Ser Ser Ile Gln Arg Lys Ser Arg Lys Ile
370             375             380

Ile Val His Leu Thr Asp Gly Val Pro Thr Arg Ser Tyr Ala Ile Asn
385             390             395             400

Ser Phe Val Lys Gly Ser Thr Tyr Ala Asn Gln Phe Glu Arg Ile Lys
        405             410             415

Glu Lys Gly Tyr Leu Asp Lys Asn Asn Tyr Phe Ile Thr Asp Asp Pro
        420             425             430

Glu Lys Ile Lys Gly Asn Gly Glu Ser Tyr Phe Leu Phe Pro Leu Asp
        435             440             445

Ser Tyr Gln Thr Gln Ile Ile Ser Gly Asn Leu Gln Lys Leu His Tyr
        450             455             460

Leu Asp Leu Asn Leu Asn Tyr Pro Lys Gly Thr Ile Tyr Arg Asn Gly
465             470             475             480

Pro Val Arg Glu His Gly Thr Pro Thr Lys Leu Tyr Ile Asn Ser Leu
        485             490             495

Lys Gln Lys Asn Tyr Asp Ile Phe Asn Phe Gly Ile Asp Ile Ser Gly
        500             505             510

Phe Arg Gln Val Tyr Asn Glu Asp Tyr Lys Lys Asn Gln Asp Gly Thr
        515             520             525

Phe Gln Lys Leu Lys Glu Glu Ala Phe Glu Leu Ser Asp Gly Glu Ile
        530             535             540

Thr Glu Leu Met Asn Ser Phe Ser Ser Lys Pro Glu Tyr Tyr Thr Pro
545             550             555             560

Ile Val Thr Ser Ala Asp Val Ser Asn Asn Glu Ile Leu Ser Lys Ile
        565             570             575

Gln Gln Gln Phe Glu Lys Ile Leu Thr Lys Glu Asn Ser Ile Val Asn
        580             585             590

Gly Thr Ile Glu Asp Pro Met Gly Asp Lys Ile Asn Leu His Leu Gly

```
                595                     600                      605

        Asn Gly Gln Thr Leu Gln Pro Ser Asp Tyr Thr Leu Gln Gly Asn Asp
            610                 615                 620

        Gly Ser Ile Met Lys Asp Ser Ile Ala Thr Gly Gly Pro Asn Asn Asp
        625                 630                 635                     640

        Gly Gly Ile Leu Lys Gly Val Lys Leu Glu Tyr Ile Lys Asn Lys Leu
                        645                 650                     655

        Tyr Val Arg Gly Leu Asn Leu Gly Glu Gly Gln Lys Val Thr Leu Thr
                    660                 665                 670

        Tyr Asp Val Lys Leu Asp Asp Ser Phe Ile Ser Asn Lys Phe Tyr Asp
                    675                 680                 685

        Thr Asn Gly Arg Thr Thr Leu Asn Pro Lys Ser Glu Glu Pro Asp Thr
            690                 695                 700

        Leu Arg Asp Phe Pro Ile Pro Lys Ile Arg Asp Val Arg Glu Tyr Pro
        705                 710                 715                     720

        Thr Ile Thr Ile Lys Asn Glu Lys Lys Leu Gly Glu Ile Glu Phe Thr
                        725                 730                 735

        Lys Val Asp Lys Asp Asn Asn Lys Leu Leu Leu Lys Gly Ala Thr Phe
                    740                 745                 750

        Glu Leu Gln Glu Phe Asn Glu Asp Tyr Lys Leu Tyr Leu Pro Ile Lys
                755                 760                 765

        Asn Asn Asn Ser Lys Val Val Thr Gly Glu Asn Gly Lys Ile Ser Tyr
                770                 775                 780

        Lys Asp Leu Lys Asp Gly Lys Tyr Gln Leu Ile Glu Ala Val Ser Pro
        785                 790                 795                     800

        Lys Asp Tyr Gln Lys Ile Thr Asn Lys Pro Ile Leu Thr Phe Glu Val
                    805                 810                 815

        Val Lys Gly Ser Ile Gln Asn Ile Ile Ala Val Asn Lys Gln Ile Ser
                    820                 825                 830

        Glu Tyr His Glu Glu Gly Asp Lys His Leu Ile Thr Asn Thr His Ile
                835                 840                 845

        Pro Pro Lys Gly Ile
            850
```

**Description**

**TECHNICAL FIELD**

This invention is in the field of immunogenic compositions comprising conjugates of *Streptococcus agalactiae* capsular saccharides and carrier proteins. The compositions are useful for immunisation.

**BACKGROUND ART**

The capsular saccharides of bacteria have been used for many years in vaccines against capsulated bacteria. As saccharides are T-independent antigens, however, they are poorly immunogenic. Conjugation to a carrier can convert T-independent antigens into T-dependent antigens, thereby enhancing memory responses and allowing protective immunity to develop. The most effective saccharide vaccines are therefore based on glycoconjugates, and the prototype conjugate vaccine was against *Haemophilus influenzae* type b ('Hib') [*e.g.* see chapter 14 of ref. 84].

Another bacterium for which conjugate vaccines have been described is *Streptococcus agalactiae,* also known as 'group B streptococcus', or simply as 'GBS'. Much of this work has been performed by Dennis Kasper and colleagues, and is described in documents such as references 1 to 9. Conjugate vaccines for each of GBS serotypes Ia, Ib, II, III, and V have been shown to be safe and immunogenic in humans [10]. Lancaster *et al.* [283] discloses testing in mice of a GBS conjugate vaccine which includes polysaccharides from GBS serotypes Ia, Ib and III. Paoletti *et al.* [203] describes a tetravalent composition comprising capsular polysaccharides from GBS serotypes Ia, Ib, II and III conjugated to tetanus toxoid. However, there remains a need for further and improved GBS conjugate vaccines.

**DISCLOSURE OF THE INVENTION**

In a first aspect, the invention provides an immunogenic composition comprising (a) a conjugate that is a capsular saccharide from GBS serotype Ia conjugated to a carrier protein; (b) a conjugate that is a capsular saccharide from GBS serotype Ib conjugated to a carrier protein; and (c) a conjugate that is a capsular polysaccharide from GBS serotype III conjugated to a carrier protein wherein (i) each GBS capsular saccharide is present at an amount of about 5 µg, 10 µg or 20 µg per unit dose, (ii) the carrier protein in (a), (b) and (c) is diphtheria toxoid or CRM197 and (iii) the immunogenic composition does not contain an aluminium salt adjuvant. The compositions may be used as vaccines for preventing infection by these GBS serotypes.

In a second aspect, the invention provides immunogenic compositions of the first aspect for use as a medicament, vaccine and in a method for immunising a patient against infection by GBS comprising the step of administering to the patient , wherein the patient has been pre-immunised with a diphtheria toxoid or derivative thereof..

*Immunogenic compositions*

Embodiments of the invention comprising three or four conjugates are described below. Of these compositions, the inventors have found that compositions comprising a conjugate that is a capsular saccharide from GBS serotype Ib conjugated to a carrier protein may confer protection against GBS serotype Ia in addition to GBS serotype Ib. This observation is in contrast to the teaching of reference 11, which suggests that type Ib conjugates are not capable of inducing antibodies that can kill type Ia bacteria. Accordingly, the embodiments described below that comprise a conjugate that is a capsular saccharide from GBS serotype Ib conjugated to a carrier protein may be advantageous in that they provide enhanced protection against serotype Ia (when the composition also comprises a conjugate that is a capsular saccharide from GBS serotype Ia conjugated to a carrier protein).

The immunogenic compositions comprise three conjugates. The first conjugate is a capsular saccharide from GBS serotype Ia conjugated to a carrier protein, while the second conjugate is a capsular saccharide from GBS serotype Ib conjugated to a carrier protein and the third conjugate is a capsular saccharide from GBS serotype III conjugated to a carrier protein. The inventors have found that such compositions (e.g. as exemplified below) are particularly suitable for use as vaccines to prevent infection by GBS.

The immunogenic compositions may comprise four conjugates. In one embodiment, the first conjugate is a capsular saccharide from GBS serotype Ia conjugated to a carrier protein, while the second conjugate is a capsular saccharide from GBS serotype Ib conjugated to a carrier protein, the third conjugate is a capsular saccharide from GBS serotype III conjugated to a carrier protein and the fourth conjugate is a capsular saccharide from GBS serotype V conjugated to a carrier protein.

Typically, the immunogenic compositions described above will not comprise any conjugates other than those specifically mentioned, particularly conjugates comprising capsular saccharides from GBS serotypes other than those specifically mentioned. However, in some embodiments, the compositions may comprise other conjugates, including conjugates comprising capsular saccharides from other GBS serotypes. For example, the compositions may comprise a conjugate that is a capsular saccharide from GBS serotype II conjugated to a carrier protein. Similarly, the compositions may comprise a conjugate that is a capsular saccharide from GBS serotype VI conjugated to a carrier protein. In another possibility, the compositions may comprise a conjugate that is a capsular saccharide from GBS serotype VIII conjugated to a carrier protein.

Also described are immunogenic compositions comprising amounts of the capsular saccharide(s) from 0.1 to 50 μg per unit dose. Typically, each GBS capsular saccharide is present at an amount from 1 to 30μg, for example from 2 to 25 μg, and in particular from 5 to 20 μg. Suitable amounts of the capsular saccharide(s) may include 5, 10 and 20 μg per unit dose, particularly when the immunogenic composition comprises capsular saccharides from GBS serotypes Ia, Ib and III. Suitable amounts per unit dose of each capsular saccharide may therefore be selected from the numbered options in the following tables, wherein the relevant embodiment is indicated by reference to the serotype(s) from which the capsular saccharide(s) in the composition are derived:

Table C - Immunogenic compositions comprising three conjugates

| Dosing option | Embodiment | | | |
| --- | --- | --- | --- | --- |
| | Ia, Ib & III | Ia, Ib & V | Ia, III & V | Ib, III & V |
| 1 | Ia: 5 μg<br>Ib: 5 μg<br>III: 5μg | Ia: 5 μg<br>Ib: 5 μg<br>V: 5μg | Ia: 5 μg<br>III: 5μg<br>V: 5μg | Ib: 5 μg<br>III: 5μg<br>V: 5μg |
| 2 | Ia: 5 μg<br>Ib: 5 μg<br>III: 10 μg | Ia: 5 μg<br>Ib: 5 μg<br>V: 10 μg | Ia: 5 μg<br>III: 5μg<br>V: 10 μg | Ib: 5 μg<br>III: 5 μg<br>V: 10 μg |
| 3 | Ia: 5 μg<br>Ib: 5 μg<br>III: 20μg | Ia: 5 μg<br>Ib: 5 μg<br>V: 20μg | Ia: 5 μg<br>III: 5 μg<br>V: 20μg | Ib: 5 μg<br>III: 5 μg<br>V: 20μg |
| 4 | Ia: 5 μg<br>Ib: 10 μg<br>III: 5μg | Ia: 5 μg<br>Ib: 10 μg<br>V: 5μg | Ia: 5 μg<br>III: 10 μg<br>V: 5μg | Ib: 5 μg<br>III: 10 μg<br>V: 5μg |
| 5 | Ia: 5 μg<br>Ib: 10 μg<br>III: 10 μg | Ia: 5 μg<br>Ib: 10 μg<br>V: 10 μg | Ia: 5 μg<br>III: 10 μg<br>V: 10 μg | Ib: 5 μg<br>III: 10 μg<br>V: 10 μg |
| 6 | Ia: 5 μg<br>Ib: 10 μg<br>III: 20 μg | Ia: 5 μg<br>Ib: 10 μg<br>V: 20 μg | Ia: 5 μg<br>III: 10 μg<br>V: 20 μg | Ib: 5 μg<br>III: 10 μg<br>V: 20 μg |
| 7 | Ia: 5 μg<br>Ib: 20 μg<br>III: 5 μg | Ia: 5 μg<br>Ib: 20 μg<br>V: 5 μg | Ia: 5 μg<br>III: 20 μg<br>V: 5 μg | Ib: 5 μg<br>III: 20 μg<br>V: 5 μg |
| 8 | Ia: 5 μg<br>Ib: 20 μg<br>III: 10 μg | Ia: 5 μg<br>Ib: 20 μg<br>V: 10 μg | Ia: 5 μg<br>III: 20 μg<br>V: 10 μg | Ib: 5 μg<br>III: 20 μg<br>V: 10 μg |
| 9 | Ia: 5 μg<br>Ib: 20 μg<br>III: 20 μg | Ia: 5 μg<br>Ib: 20 μg<br>V: 20 | Ia: 5 μg<br>III: 20 μg<br>V: 20 | Ib: 5 μg<br>III: 20 μg<br>V: 20 μg |
| 10 | Ia: 10 μg<br>Ib: 5 μg<br>III: 5 μg | Ia: 10 μg<br>Ib: 5 μg<br>V: 5 μg | Ia: 10 μg<br>III: 5 μg<br>V: 5 μg | Ib: 10 μg<br>III: 5 μg<br>V: 5 μg |
| 11 | Ia: 10 μg<br>Ib: 5 μg<br>III: 10 μg | Ia: 10 μg<br>Ib: 5 μg<br>V: 10 μg | Ia: 10 μg<br>III: 5 μg<br>V: 10 μg | Ib: 10 μg<br>III: 5 μg<br>V: 10 μg |

(continued)

| Dosing option | Embodiment | | | |
|---|---|---|---|---|
| | Ia, Ib & III | Ia, Ib & V | Ia, III & V | Ib, III & V |
| 12 | Ia: 10 μg<br>Ib: 5 μg<br>III: 20 μg | Ia: 10 μg<br>Ib: 5 μg<br>V: 20 μg | Ia: 10 μg<br>III: 5 μg<br>V: 20 μg | Ib: 10 μg<br>III: 5 μg<br>V: 20 μg |
| 13 | Ia: 10 μg<br>Ib: 10 μg<br>III: 5 μg | Ia: 10 μg<br>Ib: 10 μg<br>V: 5 μg | Ia: 10 μg<br>III: 10 μg<br>V: 5 μg | Ib: 10 μg<br>III: 10 μg<br>V: 5 μg |
| 14 | Ia: 10 μg<br>Ib: 10 μg<br>III: 10 μg | Ia: 10 μg<br>Ib: 10 μg<br>V: 10 μg | Ia: 10 μg<br>III: 10 μg<br>V: 10 μg | Ib: 10 μg<br>III: 10 μg<br>V: 10 μg |
| 15 | Ia: 10 μg<br>Ib: 10 μg<br>III: 20 μg | Ia: 10 μg<br>Ib: 10 μg<br>V: 20 | Ia: 10 μg<br>III: 10 μg<br>V: 20 μg | Ib: 10 μg<br>III: 10 μg<br>V: 20 μg |
| 16 | Ia: 10 μg<br>Ib: 20 μg<br>III: 5 μg | Ia: 10 μg<br>Ib: 20 μg<br>V: 5 μg | Ia: 10 μg<br>III: 20 μg<br>V: 5 μg | Ib: 10 μg<br>III: 20 μg<br>V: 5 μg |
| 17 | Ia: 10 μg<br>Ib: 20 μg<br>III: 10 μg | Ia: 10 μg<br>Ib: 20 μg<br>V: 10 μg | Ia: 10 μg<br>III: 20 μg<br>V: 10 μg | Ib: 10 μg<br>III: 20 μg<br>V: 10 μg |
| 18 | Ia: 10 μg<br>Ib: 20 μg<br>III: 20 μg | Ia: 10 μg<br>Ib: 20 μg<br>V: 20 μg | Ia: 10 μg<br>III: 20 μg<br>V: 20 μg | Ib: 10 μg<br>III: 20 μg<br>V: 20 μg |
| 19 | Ia: 20 μg<br>Ib: 5 μg<br>III: 5 μg | Ia: 20 μg<br>Ib: 5 μg<br>V: 5 μg | Ia: 20 μg<br>III: 5 μg<br>V: 5 μg | Ib: 20 μg<br>III: 5 μg<br>V: 5 μg |
| 20 | Ia: 20 μg<br>Ib: 5 μg<br>III: 10 μg | Ia: 20 μg<br>Ib: 5 μg<br>V: 10 μg | Ia: 20 μg<br>III: 5 μg<br>V: 10 μg | Ib: 20 μg<br>III: 5 μg<br>V: 10 μg |
| 21 | Ia: 20 μg<br>Ib: 5 μg<br>III: 20 μg | Ia: 20 μg<br>Ib: 5 μg<br>V: 20 μg | Ia: 20 μg<br>III: 5 μg<br>V: 20 μg | Ib: 20 μg<br>III: 5 μg<br>V: 20 μg |
| 22 | Ia: 20 μg<br>Ib: 10 μg<br>III: 5 μg | Ia: 20 μg<br>Ib: 10 μg<br>V: 5 μg | Ia: 20 μg<br>III: 10 μg<br>V: 5 μg | Ib: 20 μg<br>III: 10 μg<br>V: 5 μg |
| 23 | Ia: 20 μg<br>Ib: 10 μg<br>III: 10 μg | Ia: 20 μg<br>Ib: 10 μg<br>V: 10 μg | Ia: 20 μg<br>III: 10 μg<br>V: 10 μg | Ib: 20 μg<br>III: 10 μg<br>V: 10 μg |
| 24 | Ia: 20 μg<br>Ib: 10 μg<br>III: 20 μg | Ia: 20 μg<br>Ib: 10 μg<br>V: 20 μg | Ia: 20 μg<br>III: 10 μg<br>V: 20 μg | Ib: 20 μg<br>III: 10 μg<br>V: 20 μg |
| 25 | Ia: 20 μg<br>Ib: 20 μg<br>III: 5 μg | Ia: 20 μg<br>Ib: 20 μg<br>V: 5 μg | Ia: 20 μg<br>III: 20 μg<br>V: 5 μg | Ib: 20 μg<br>III: 20 μg<br>V: 5 μg |

(continued)

| Dosing option | Embodiment | | | |
|---|---|---|---|---|
| | Ia, Ib & III | Ia, Ib & V | Ia, III & V | Ib, III & V |
| 26 | Ia: 20 μg<br>Ib: 20 μg<br>III: 10 μg | Ia: 20 μg<br>Ib: 20 μg<br>V: 10 μg | Ia: 20 μg<br>III: 20 μg<br>V: 10 μg | Ib: 20 μg<br>III: 20 μg<br>V: 10 μg |
| 27 | Ia: 20 μg<br>Ib: 20 μg<br>III: 20 μg | Ia: 20 μg<br>Ib: 20 μg<br>V: 20 μg | Ia: 20 μg<br>III: 20 μg<br>V: 20 μg | Ib: 20 μg<br>III: 20 μg<br>V: 20 μg |

Of the dosing options described in Table C, the inventors have found that options 1, 14 and 27 are effective, when the immunogenic composition comprises: a) a conjugate that is a capsular saccharide from GBS serotype Ia conjugated to a carrier protein; b) a conjugate that is a capsular saccharide from GBS serotype Ib conjugated to a carrier protein; and c) a conjugate that is a capsular saccharide from GBS serotype III conjugated to a carrier protein. These dosing options are therefore preferred for use in the invention, particularly for this embodiment. It may be advantageous to minimise the total amount of capsular saccharide(s) per unit dose in order to reduce potential toxicity. Accordingly, dosing option 1 is particularly preferred.

It may be possible to further minimise the amount of capsular saccharide(s) per unit dose. Therefore, also described are amounts of the capsular saccharide(s) from 0.1 to 5 μg per unit dose. For example, an amount from 0.1 to 5 μg, *e.g.* 0.5, 2.5 or 5 μg, per unit dose, for example, each GBS capsular saccharide may be present at an amount from 0.5 to 5 μg, 1 to 4 μg, 2 to 3 μg, or about 2.5 μg per unit dose. Such amounts per unit dose of each capsular saccharide are described in the table below:

Table C' - Immunogenic compositions comprising capsular saccharides from GBS serotypes Ia, Ib and III

| Dosing option | Amount of capsular saccharide per unit dose (μg) | | |
|---|---|---|---|
| | Ia | Ib | III |
| 1 | 0.5 | 0.5 | 0.5 |
| 2 | 0.5 | 0.5 | 2.5 |
| 3 | 0.5 | 0.5 | 5 |
| 4 | 0.5 | 2.5 | 0.5 |
| 5 | 0.5 | 2.5 | 2.5 |
| 6 | 0.5 | 2.5 | 5 |
| 7 | 0.5 | 5 | 0.5 |
| 8 | 0.5 | 5 | 2.5 |
| 9 | 0.5 | 5 | 5 |
| 10 | 2.5 | 0.5 | 0.5 |
| 11 | 2.5 | 0.5 | 2.5 |
| 12 | 2.5 | 0.5 | 5 |
| 13 | 2.5 | 2.5 | 0.5 |
| 14 | 2.5 | 2.5 | 2.5 |
| 15 | 2.5 | 2.5 | 5 |
| 16 | 2.5 | 5 | 0.5 |
| 17 | 2.5 | 5 | 2.5 |
| 18 | 2.5 | 5 | 5 |

(continued)

| Dosing option | Amount of capsular saccharide per unit dose (μg) | | |
|---|---|---|---|
| | Ia | Ib | III |
| 19 | 5 | 0.5 | 0.5 |
| 20 | 5 | 0.5 | 2.5 |
| 21 | 5 | 0.5 | 5 |
| 22 | 5 | 2.5 | 0.5 |
| 23 | 5 | 2.5 | 2.5 |
| 24 | 5 | 2.5 | 5 |
| 25 | 5 | 5 | 0.5 |
| 26 | 5 | 5 | 2.5 |
| 27 | 5 | 5 | 5 |

Of the dosing options described in Table C', the inventors particulary envisage options 1, 14 and 27. In these options, the amount of each GBS capsular saccharide is the same (e.g. as in the higher dose compositions exemplified below).

Table D - Immunogenic compositions comprising four conjugates

| Embodiment - Ia, Ib, III & V | | | | | |
|---|---|---|---|---|---|
| Dosing option | | Dosing option | | Dosing option | |
| 1 | Ia: 5 μg<br>Ib: 5 μg<br>III: 5 μg<br>V: 5 μg | 28 | Ia: 10 μg<br>Ib: 5 μg<br>III: 5 μg<br>V: 5 μg | 55 | Ia: 20 μg<br>Ib: 5 μg<br>III: 5 μg<br>V: 5 μg |
| 2 | Ia: 5 μg<br>Ib: 5 μg<br>III: 5 μg<br>V: 10 μg | 29 | Ia: 10 μg<br>Ib: 5 μg<br>III: 5 μg<br>V: 10 μg | 56 | Ia: 20 μg<br>Ib: 5 μg<br>III: 5 μg<br>V: 10 μg |
| 3 | Ia: 5 μg<br>Ib: 5 μg<br>III: 5 μg<br>V: 20 μg | 30 | Ia: 10 μg<br>Ib: 5 μg<br>III: 5 μg<br>V: 20 μg | 57 | Ia: 20 μg<br>Ib: 5 μg<br>III: 5 μg<br>V: 20 μg |
| 4 | Ia: 5 μg<br>Ib: 5 μg<br>III: 10 μg<br>V: 5 μg | 31 | Ia: 10 μg<br>Ib: 5 μg<br>III: 10 μg<br>V: 5 μg | 58 | Ia: 20 μg<br>Ib: 5 μg<br>III: 10 μg<br>V: 5 μg |
| 5 | Ia: 5 μg<br>Ib: 5 μg<br>III: 10 μg<br>V: 10 μg | 32 | Ia: 10 μg<br>Ib: 5 μg<br>III: 10 μg<br>V: 10 μg | 59 | Ia: 20 μg<br>Ib: 5 μg<br>III: 10 μg<br>V: 10 μg |
| 6 | Ia: 5 μg<br>Ib: 5 μg<br>III: 10 μg<br>V: 20 μg | 33 | Ia: 10 μg<br>Ib: 5 μg<br>III: 10 μg<br>V: 20 μg | 60 | Ia: 20 μg<br>Ib: 5 μg<br>III: 10 μg<br>V: 20 μg |

(continued)

| Embodiment - Ia, Ib, III & V | | | | | |
|---|---|---|---|---|---|
| **Dosing option** | | **Dosing option** | | **Dosing option** | |
| 7 | Ia: 5 μg<br>Ib: 5 μg<br>III: 20 μg<br>V: 5 μg | 34 | Ia: 10 μg<br>Ib: 5 μg<br>III: 20 μg<br>V: 5 μg | 61 | Ia: 20 μg<br>Ib: 5 μg<br>III: 20 μg<br>V: 5 μg |
| 8 | Ia: 5 μg<br>Ib: 5 μg<br>III: 20 μg<br>V: 10 μg | 35 | Ia: 10 μg<br>Ib: 5 μg<br>III: 20 μg<br>V: 10 μg | 62 | Ia: 20 μg<br>Ib: 5 μg<br>III: 20 μg<br>V: 10 μg |
| 9 | Ia: 5 μg<br>Ib: 5 μg<br>III: 20 μg<br>V: 20 μg | 36 | Ia: 10 μg<br>Ib: 5 μg<br>III: 20 μg<br>V: 20 μg | 63 | Ia: 20 μg<br>Ib: 5 μg<br>III: 20 μg<br>V: 20 μg |
| 10 | Ia: 5 μg<br>Ib: 10 μg<br>III: 5 μg<br>V: 5 μg | 37 | Ia: 10 μg<br>Ib: 10 μg<br>III: 5 μg<br>V: 5 μg | 64 | Ia: 20 μg<br>Ib: 10 μg<br>III: 5 μg<br>V: 5 μg |
| 11 | Ia: 5 μg<br>Ib: 10 μg<br>III: 5 μg<br>V: 10 μg | 38 | Ia: 10 μg<br>Ib: 10 μg<br>III: 5 μg<br>V: 10 μg | 65 | Ia: 20 μg<br>Ib: 10 μg<br>III: 5 μg<br>V: 10 μg |
| 12 | Ia: 5 μg<br>Ib: 10 μg<br>III: 5 μg<br>V: 20 μg | 39 | Ia: 10 μg<br>Ib: 10 μg<br>III: 5 μg<br>V: 20 μg | 66 | Ia: 20 μg<br>Ib: 10 μg<br>III: 5 μg<br>V: 20 μg |
| 13 | Ia: 5 μg<br>Ib: 10 μg<br>III: 10 μg<br>V: 5 μg | 40 | Ia: 10 μg<br>Ib: 10 μg<br>III: 10 μg<br>V: 5 μg | 67 | Ia: 20 μg<br>Ib: 10 μg<br>III: 10 μg<br>V: 5 μg |
| 14 | Ia: 5 μg<br>Ib: 10 μg<br>III: 10 μg<br>V: 10 μg | 41 | Ia: 10 μg<br>Ib: 10 μg<br>III: 10 μg<br>V: 10 μg | 68 | Ia: 20 μg<br>Ib: 10 μg<br>III: 10 μg<br>V: 10 μg |
| 15 | Ia: 5 μg<br>Ib: 10 μg<br>III: 10 μg<br>V: 20 μg | 42 | Ia: 10 μg<br>Ib: 10 μg<br>III: 10 μg<br>V: 20 μg | 69 | Ia: 20 μg<br>Ib: 10 μg<br>III: 10 μg<br>V: 20 μg |
| 16 | Ia: 5 μg<br>Ib: 10 μg<br>III: 20 μg<br>V: 5 μg | 43 | Ia: 10 μg<br>Ib: 10 μg<br>III: 20 μg<br>V: 5 μg | 70 | Ia: 20 μg<br>Ib: 10 μg<br>III: 20 μg<br>V: 5 μg |
| 17 | Ia: 5 μg<br>Ib: 10 μg<br>III: 20 μg<br>V: 10 μg | 44 | Ia: 10 μg<br>Ib: 10 μg<br>III: 20 μg<br>V: 10 μg | 71 | Ia: 20 μg<br>Ib: 10 μg<br>III: 20 μg<br>V: 10 μg |

(continued)

| Embodiment - Ia, Ib, III & V | | | | | |
|---|---|---|---|---|---|
| Dosing option | | Dosing option | | Dosing option | |
| 18 | Ia: 5 μg<br>Ib: 10 μg<br>III: 20 μg<br>V: 20 μg | 45 | Ia: 10 μg<br>Ib: 10 μg<br>III: 20 μg<br>V: 20 μg | 72 | Ia: 20 μg<br>Ib: 10 μg<br>III: 20 μg<br>V: 20 μg |
| 19 | Ia: 5 μg<br>Ib: 20 μg<br>III: 5 μg<br>V: 5 μg | 46 | Ia: 10 μg<br>Ib: 20 μg<br>III: 5 μg<br>V: 5 μg | 73 | Ia: 20 μg<br>Ib: 20 μg<br>III: 5 μg<br>V: 5 μg |
| 20 | Ia: 5 μg<br>Ib: 20 μg<br>III: 5 μg<br>V: 10 μg | 47 | Ia: 10 μg<br>Ib: 20 μg<br>III: 5 μg<br>V: 10 μg | 74 | Ia: 20 μg<br>Ib: 20 μg<br>III: 5 μg<br>V: 10 μg |
| 21 | Ia: 5 μg<br>Ib: 20 μg<br>III: 5 μg<br>V: 20 μg | 48 | Ia: 10 μg<br>Ib: 20 μg<br>III: 5 μg<br>V: 20 μg | 75 | Ia: 20 μg<br>Ib: 20 μg<br>III: 5 μg<br>V: 20 μg |
| 22 | Ia: 5 μg<br>Ib: 20 μg<br>III: 10 μg<br>V: 5 μg | 49 | Ia: 10 μg<br>Ib: 20 μg<br>III: 10 μg<br>V: 5 μg | 76 | Ia: 20 μg<br>Ib: 20 μg<br>III: 10 μg<br>V: 5 μg |
| 23 | Ia: 5 μg<br>Ib: 20 μg<br>III: 10 μg<br>V: 10 μg | 50 | Ia: 10 μg<br>Ib: 20 μg<br>III: 10 μg<br>V: 10 μg | 77 | Ia: 20 μg<br>Ib: 20 μg<br>III: 10 μg<br>V: 10 μg |
| 24 | Ia: 5 μg<br>Ib: 20 μg<br>III: 10 μg<br>V: 20 μg | 51 | Ia: 10 μg<br>Ib: 20 μg<br>III: 10 μg<br>V: 20 μg | 78 | Ia: 20 μg<br>Ib: 20 μg<br>III: 10 μg<br>V: 20 μg |
| 25 | Ia: 5 μg<br>Ib: 20 μg<br>III: 20 μg<br>V: 5 μg | 52 | Ia: 10 μg<br>Ib: 20 μg<br>III: 20 μg<br>V: 5 μg | 79 | Ia: 20 μg<br>Ib: 20 μg<br>III: 20 μg<br>V: 5 μg |
| 26 | Ia: 5 μg<br>Ib: 20 μg<br>III: 20 μg<br>V: 10 μg | 53 | Ia: 10 μg<br>Ib: 20 μg<br>III: 20 μg<br>V: 10 μg | 80 | Ia: 20 μg<br>Ib: 20 μg<br>III: 20 μg<br>V: 10 μg |
| 27 | Ia: 5 μg<br>Ib: 20 μg<br>III: 20 μg<br>V: 20 μg | 54 | Ia: 10 μg<br>Ib: 20 μg<br>III: 20 μg<br>V: 20 μg | 81 | Ia: 20 μg<br>Ib: 20 μg<br>III: 20 μg<br>V: 20 μg |

The ratio of the mass of a given capsular saccharide to the mass of the other capsular saccharide(s) may vary. Suitable ratios (w/w) for each capsular saccharide are disclosed as numbered options in the following tables:

Table F - Immunogenic compositions comprising three conjugates

| Ratio option | Embodiment | | | |
|---|---|---|---|---|
| | Ia : Ib : III | | | |
| 1 | 1:1:1 | | | |
| 2 | 1:1:2 | | | |
| 3 | 1:1:4 | | | |
| 4 | 1:2:1 | | | |
| 5 | 1:2:2 | | | |
| 6 | 1:2:4 | | | |
| 7 | 1:4:1 | | | |
| 8 | 1:4:2 | | | |
| 9 | 1:4:4 | | | |
| 10 | 2:1:1 | | | |
| 11 | 2:1:2 | | | |
| 12 | 2:1:4 | | | |
| 13 | 2:2:1 | | | |
| 14 | 2:4:1 | | | |
| 15 | 4:1:1 | | | |
| 16 | 4:1:2 | | | |
| 17 | 4:1:4 | | | |
| 18 | 4:2:1 | | | |
| 19 | 4:4:1 | | | |

Of the ratio options described in Table F, the inventors have found that option 1 is effective, when the immunogenic composition comprises: a) a conjugate that is a capsular saccharide from GBS serotype Ia conjugated to a carrier protein; b) a conjugate that is a capsular saccharide from GBS serotype Ib conjugated to a carrier protein; and c) a conjugate that is a capsular saccharide from GBS serotype III conjugated to a carrier protein. This ratio option is therefore preferred for use in the invention, particularly for this embodiment.

Table G - Immunogenic compositions comprising four conjugates

| Embodiment - Ia, Ib, III & V | | | | | |
|---|---|---|---|---|---|
| Ratio option | Ia : Ib : III: V | Ratio option | Ia : Ib : III: V | Ratio option | Ia : Ib : III: V |
| 1 | 1:1:1:1 | 23 | 1:4:2:2 | 45 | 2:4:2:1 |
| 2 | 1:1:1:2 | 24 | 1:4:2:4 | 46 | 2:4:4:1 |
| 3 | 1:1:1:4 | 25 | 1:4:4:1 | 47 | 4:1:1:1 |
| 4 | 1:1:2:1 | 26 | 1:4:4:2 | 48 | 4:1:1:2 |
| 5 | 1:1:2:2 | 27 | 1:4:4:4 | 49 | 4:1:1:4 |
| 6 | 1:1:2:4 | 28 | 2:1:1:1 | 50 | 4:1:2:1 |
| 7 | 1:1:4:1 | 29 | 2:1:1:2 | 51 | 4:1:2:2 |
| 8 | 1:1:4:2 | 30 | 2:1:1:4 | 52 | 4:1:2:4 |
| 9 | 1:1:4:4 | 31 | 2:1:2:1 | 53 | 4:1:4:1 |
| 10 | 1:2:1:1 | 32 | 2:1:2:2 | 54 | 4:1:4:2 |

(continued)

| Embodiment - Ia, Ib, III & V | | | | | |
|---|---|---|---|---|---|
| Ratio option | Ia : Ib : III: V | Ratio option | Ia : Ib : III: V | Ratio option | Ia : Ib : III: V |
| 11 | 1:2:1:2 | 33 | 2:1:2:4 | 55 | 4:1:4:4 |
| 12 | 1:2:1:4 | 34 | 2:1:4:1 | 56 | 4:2:1:1 |
| 13 | 1:2:2:1 | 35 | 2:1:4:2 | 57 | 4:2:1:2 |
| 14 | 1:2:2:2 | 36 | 2:1:4:4 | 58 | 4:2:1:4 |
| 15 | 1:2:2:4 | 37 | 2:2:1:1 | 59 | 4:2:2:1 |
| 16 | 1:2:4:1 | 38 | 2:2:1:2 | 60 | 4:2:4:1 |
| 17 | 1:2:4:2 | 39 | 2:2:1:4 | 61 | 4:4:1:1 |
| 18 | 1:2:4:4 | 40 | 2:2:2:1 | 62 | 4:4:1:2 |
| 19 | 1:4:1:1 | 41 | 2:2:4:1 | 63 | 4:4:1:4 |
| 20 | 1:4:1:2 | 42 | 2:4:1:1 | 64 | 4:4:2:1 |
| 21 | 1:4:1:4 | 43 | 2:4:1:2 | 65 | 4:4:4:1 |
| 22 | 1:4:2:1 | 44 | 2:4:1:4 | | |

As discussed above, the invention relates in part to immunogenic compositions further comprising a conjugate that is a capsular saccharide from GBS serotype V conjugated to a carrier protein. The inventors have found that the immune response to the capsular saccharide from GBS serotype V in these compositions may be diminished if the immunogenic composition comprises one or more further antigen(s). Without wishing to be bound by theory, it is thought that the presence of the further antigen(s) results in "immune interference", with the response to the capsular saccharide from GBS serotype V being diminished.

The inventors have found that the response to the capsular saccharide from GBS serotype V in these immunogenic compositions may be improved if the composition comprises an adjuvant. This observation is in contrast to the teaching of reference 12, which suggests that adjuvants may not improve the immune response to GBS conjugates.. The skilled person would be capable of identifying adjuvants that may be used in these compositions.

The inventors have also found that the response to the capsular saccharide from GBS serotype V may be improved if the dose of this capsular saccharide is increased. In particular, the response to the type V capsular saccharide may be improved if the dose of the type V capsular saccharide is greater than the dose of the capsular saccharide from the other GBS serotype. Accordingly, in another embodiment the present invention provides an immunogenic composition comprising: a) a conjugate that is a capsular saccharide from GBS serotype V conjugated to a carrier protein; wherein the dose of the type V capsular saccharide is greater than the total dose(s) of the capulsular saccharides from the other GBS serotypes, or is greater than at least one of the doses or the mean dose of the capsular saccharides from the other GBS serotypes. The dose of the type V capsular saccharide may be 1.1,2, 3, 4, 5, 6, 7, 8, 9 or 10 times greater. It is typical for the dose of the type V capsular saccharide to be greater than the mean dose of the capsular saccharides from the other GBS serotypes. Accordingly, this embodiment of the invention encompasses any of the immunogenic compositions described herein that comprise a conjugate that is a capsular saccharide from GBS serotype V conjugated to a carrier protein and further comprises conjugates that are capsular saccharides from GBS serotypes Ia, Ib and III conjugated to carrier proteins; wherein the dose of the type V capsular saccharide is greater than the total dose(s) of the capulsular saccharide(s) from the other GBS serotype(s), or is greater than at least one of the doses or the mean dose of the capsular sacchardes from the other GBS serotypes .

Methods of administering the immunogenic compositions of the invention are discussed below. Briefly, the immunogenic compositions of the invention may be administered in single or multiple doses. The inventors have found that the administration of a single dose of the immunogenic compositions of the invention is effective, particularly when the immunogenic composition comprises capsular saccharides from GBS serotypes Ia, Ib and III; and more particularly when the immunogenic composition comprises: a) a conjugate that is a capsular saccharide from GBS serotype Ia conjugated to a carrier protein; b) a conjugate that is a capsular saccharide from GBS serotype Ib conjugated to a carrier protein; and c) a conjugate that is a capsular saccharide from GBS serotype III conjugated to a carrier protein. Administration of a single dose is therefore preferred in the invention, particularly for these embodiments.

Alternatively, one unit dose followed by a second unit dose may be effective. Typically, the second (or third, fourth, fifth *etc*.) unit dose is identical to the first unit dose. The second unit dose may be administered at any suitable time after the first unit dose, in particular after 1, 2 or 3 months. For example, when the immunogenic composition comprises capsular saccharides from GBS serotypes Ia, Ib and III, then the second unit dose may be administered 3 months after the first unit dose. In another example, if the immunogenic composition also comprises capsular saccharides from GBS serotypes V, then the second unit dose may be administered 1 month after the first unit dose. Typically, the the immunogenic compositions of the invention will be administered intramuscularly, e.g. by intramuscular administration to the thigh or the upper arm as described below.

The inventors have found that the use of unadjuvanted compositions is effective, particularly when the immunogenic composition comprises capsular saccharides from GBS serotypes Ia, Ib and III; and more particularly when the immunogenic composition comprises: a) a conjugate that is a capsular saccharide from GBS serotype Ia conjugated to a carrier protein; b) a conjugate that is a capsular saccharide from GBS serotype Ib conjugated to a carrier protein; and c) a conjugate that is a capsular saccharide from GBS serotype III conjugated to a carrier protein wherein (i) each GBS capsular saccharide is present at an amount of 5 μg, 10 μg or 20 μg per unit dose and (ii) the carrier protein in (a), (b) and (c) is diphtheria toxoid or CRM197. It may be advantageous to omit adjuvants in order to reduce potential toxicity. Accordingly, immunogenic compositions that do not contain any adjuvant (especially that do not contain any aluminium salt adjuvant) are preferred for use in the invention, particularly for these embodiments.

***The capsular saccharide***

The invention is based on the capsular saccharide of *Streptococcus agalactiae.* The capsular saccharide is covalently linked to the peptidoglycan backbone of GBS, and is distinct from the group B antigen, which is another saccharide that is attached to the peptidoglycan backbone.

The GBS capsular saccharides are chemically related, but are antigenically very different. All GBS capsular psaccharides share the following trisaccharide core:

β-D-Gl*cp*NAc(1→3)β-D-Gal*p*(1→4)β-D-Glc*p*

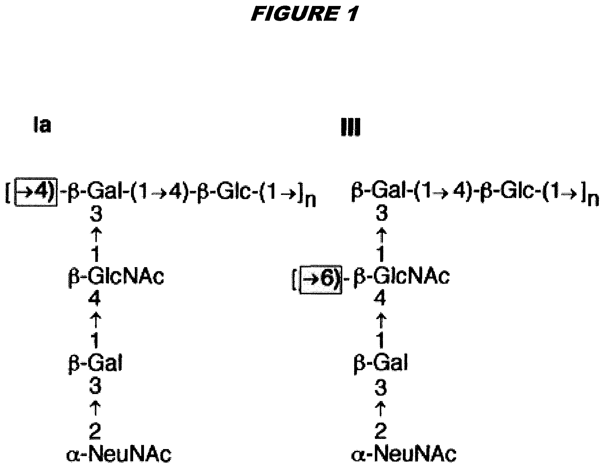

The various GBS serotypes differ by the way in which this core is modified. The difference between serotypes Ia and III, for instance, arises from the use of either the GlcNAc (Ia) or the Gal (III) in this core for linking consecutive trisaccharide cores (Figure 1). Serotypes Ia and Ib both have a [α-D-Neu*p*NAc(2→3)β-D-Gal*p*-(1→] disaccharide linked to the GlcNAc in the core, but the linkage is either 1→4 (Ia) or 1→3 (Ib).

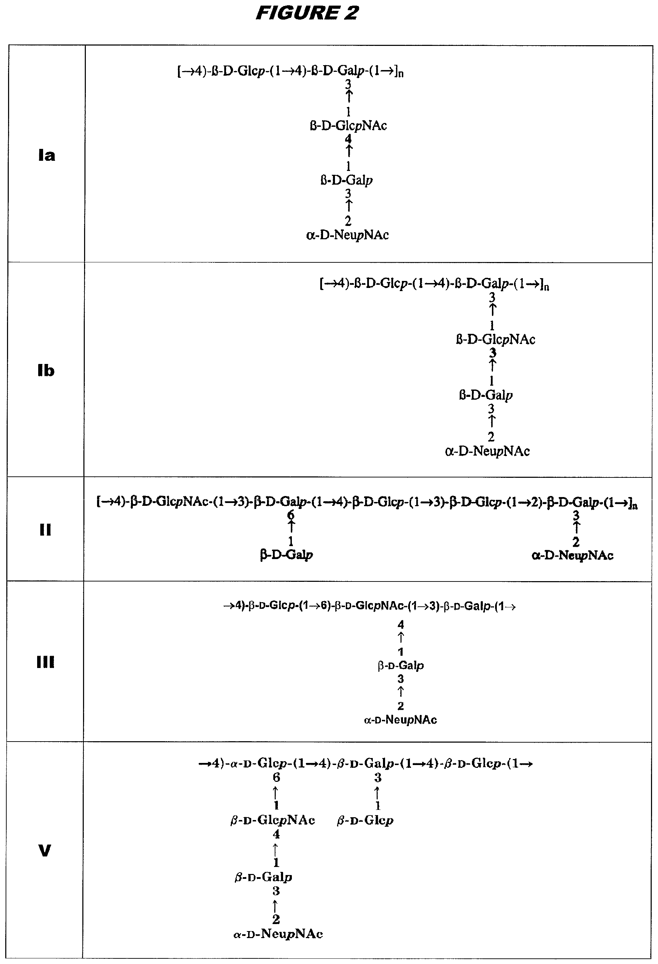

GBS-related disease arises primarily from serotypes Ia, Ib, II, III, IV, V, VI, VII, and VIII, with over 85% being caused by five serotypes: Ia, Ib, III & V. The invention preferably uses a saccharide from three or more of these four serotypes, particularly from serotypes: Ia, Ib & III. As shown in Figure 2, the capsular saccharides of each of these four serotypes include: (a) a terminal *N*-acetyl-neuraminic acid (NeuNAc) residue (commonly referred to as sialic acid), which in all cases is linked 2→3 to a galactose residue; and (b) a *N*-acetyl-glucosamine residue (GlcNAc) within the trisaccharide core.

All four saccharides include galactose residues within the trisaccharide core, but serotypes Ia, Ib, II & III also contain additional galactose residues in each repeating unit.

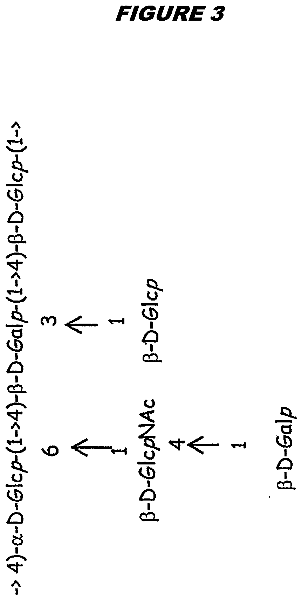

Saccharides used according to the invention may be in their native form, or may have been modified. For example, the saccharide may be shorter than the native capsular saccharide, or may be chemically modified. In particular, the serotype V capsular saccharide used in the invention may be modified as described in refs. 13 and 14. For example, a serotype V capsular saccharide that has been substantially desialylated (Figure 3) as described in refs. 13 and 14 is specifically envisaged for use in the present invention. Desialylated GBS serotype V capsular saccharide may be prepared by treating purified GBS serotype V capsular saccharide under mildly acidic conditions (e.g. 0.1M sulphuric acid at 80°C for 60 minutes) or by treatment with neuraminidase, as described in reference 13. A preferred method for preparing desialylated GBS serotype V capsular saccharide is by treating the purified saccharide with 1M acetic acid at 81°C +/- 3C° for 2h. Thus the saccharide used according to the invention may be a substantially full-length capsular polysaccharide, as found in nature, or it may be shorter than the natural length. Full-length polysaccharides may be depolymerised to give shorter fragments for use with the invention *e.g.* by hydrolysis in mild acid, by heating, by sizing chromatography, *etc.* Chain length has been reported to affect immunogenicity of GBS saccharides in rabbits [4]. In particular, the serotype II and/or III capsular saccharides used in the invention may be depolymerised as described in refs. 15 and 16. These documents describe the partial depolymerization of type II and type III capsular saccharides by mild deaminative cleavage to antigenic fragments with reducing-terminal 2,5-anhydro-D-mannose residues. Briefly, the capsular saccharide is dissolved in 0.5 N NaOH and heated at 70°C for between about 1-4h. The length of this incubation controls the degree of depolymerisation, which may be determined by standard methods (e.g. by HPLC as described in reference 15). The sample is chilled in an ice-water bath before glacial acetic acid is added to bring the pH to 4. The partially N-deacylated product is then deaminated by the addition of 5% (wt/vol) NaNO$_2$ with stirring at 4°C for 2h. The free aldehydes of the newly formed 2,5-anhydro-D-mannose residues may be used for conjugation to a carrier protein, as described below.

Depolymerisation of the serotype III capsular saccharide by endo-β-galactosidase has been reported [refs. 1 & 4-6], including using the depolymerised material to form conjugates with a tetanus toxoid carrier. Ozonolysis of capsular polysaccharides from GBS serotypes III and VIII has also been used for depolymerisation [17]. It is preferred to use saccharides with MW>30kDa, and substantially full-length capsular polysaccharides can be used. For serotype Ia, it is preferred to use polysaccharides with a MW in the range of 150-300kDa, particularly 175-275 kDa. Typically, a serotype Ia saccharide with MW about 200 kDa or about 260 kDa is used. For serotype Ib, it is preferred to use polysaccharides with a MW in the range of 150-300kDa, particularly 175-250 kDa. Typically, a serotype Ib saccharide with MW about 200 kDa or about 230 kDa is used. For serotype III, it is preferred to use polysaccharides with a MW in the range of 50-200kDa, particularly 80-150kDa. Typically, a serotype III saccharide with MW about 100 kDa or about 140kDa is used. For serotype V, it is also preferred to use polysaccharides with a MW up to ~50kDa. Typically, a serotype V saccharide with MW about 100 kDa is used. These molecular masses can be measured by gel filtration relative to dextran standards, such as those available from Polymer Standard Service [18].

The saccharide may be chemically modified relative to the capsular saccharide as found in nature. For example, the saccharide may be de-O-acetylated (partially or fully), de-N-acetylated (partially or fully), N-propionated (partially or fully), *etc.* De-acetylation may occur before, during or after conjugation, but preferably occurs before conjugation. Depending on the particular saccharide, de-acetylation may or may not affect immunogenicity. The relevance of O-acetylation on GBS saccharides in various serotypes is discussed in reference 19, and in some embodiments O-acetylation of sialic acid residues at positions 7, 8 and/or 9 is retained before, during and after conjugation *e.g.* by protection/deprotection, by re-acetylation, *etc.* However, typically the GBS saccharide used in the present invention has substantially no O-acetylation of sialic acid residues at positions 7, 8 and/or 9. In particular, when the GBS saccharide has been purified by base extraction as described below, then O-acetylation is typically lost (ref. 19). The effect of de-acetylation *etc.* can be assessed by routine assays.

Capsular saccharides can be purified by known techniques, as described in the references herein such as refs. 2 and 20. A typical process involves base extraction, centrifugation, filtration, RNase/DNase treatment, protease treatment, concentration, size exclusion chromatography, ultrafiltration, anion exchange chromatography, and further ultrafiltration. Treatment of GBS cells with the enzyme mutanolysin, which cleaves the bacterial cell wall to free the cell wall components, is also useful.

As an alternative, the purification process described in reference 21 can be used. This involves base extraction, ethanol/CaCl$_2$ treatment, CTAB precipitation, and re-solubilisation. A further alternative process is described in reference 22.

The invention is not limited to saccharides purified from natural sources, however, and the saccharides may be obtained by other methods, such as total or partial synthesis.

*Conjugation*

The invention involves conjugates that are capsular saccharides from GBS serotypes Ia, Ib, III and optionally V conjugated to a carrier protein. In general, covalent conjugation of saccharides to carriers enhances the immunogenicity of saccharides as it converts them from T-independent antigens to T-dependent antigens, thus allowing priming for immunological memory. Conjugation is particularly useful for paediatric vaccines [*e.g.* ref. 23] and is a well known technique [*e.g.* reviewed in refs. 24 to 32]. Thus the processes of the invention may include the further step of conjugating the purified saccharide to a carrier molecule.

Conjugation of GBS saccharides has been widely reported *e.g.* see references 1 to 9. The typical prior art process for GBS saccharide conjugation typically involves reductive amination of a purified saccharide to a carrier protein such as tetanus toxoid (TT) or CRM197 [2]. The reductive amination involves an amine group on the side chain of an amino acid in the carrier and an aldehyde group in the saccharide. As GBS capsular saccharides do not include an aldehyde group in their natural form then this is typically generated before conjugation by oxidation (e.g. periodate oxidation) of a portion (*e.g.* between 5 and 40%, particularly between 10 and 30%, preferably about 20%) of the saccharide's sialic acid residues [2,33]. Conjugate vaccines prepared in this manner have been shown to be safe and immunogenic in humans for each of GBS serotypes Ia, Ib, II, III, and V [10]. Typically, all of the conjugates in the immunogenic compositions of the present invention have been prepared in this manner. However, when the invention uses a serotype V capsular saccharide that is desialylated, then an aldehyde group may be generated in this saccharide before conjugation by oxidation (*e.g.* periodate oxidation) of a portion (*e.g.* between 5 and 40%, particularly between 10 and 30%, preferably about 20%) of the saccharide's galactose residues [14]. An alternative conjugation process involves the use of -NH$_2$ groups in the saccharide (either from de-N-acetylation, or after introduction of amines) in conjunction with bifunctional linkers, as described in ref. 34. In some embodiments, one or more of the conjugates in the immunogenic compositions of the present invention have been prepared in this manner. A further alternative process is described in refs. 15 and 16. In this process, the free aldehydes groups of terminal 2,5-anhydro-D-mannose residues from depolymerization of type II or type III capsular saccharides by mild deaminative cleavage are used for conjugation by reductive amination. In some embodiments, one or more of the conjugates in the immunogenic compositions of the present invention have been prepared in this manner.

The invention involves the use of carrier molecules, which are diphtheria toxoid or CRM197. Also described are carrier proteins including other bacterial toxins or toxoids, such as tetanus toxoid. Fragments of toxins or toxoids are also described *e.g.* fragment C of tetanus toxoid [35]. The CRM197 mutant of diphtheria toxin [36-38] is particularly useful with the invention. Also described herein are carrier proteins including the *N.meningitidis* outer membrane protein [39], synthetic peptides [40,41], heat shock proteins [42,43], pertussis proteins [44,45], cytokines [46], lymphokines [46], hormones [46], growth factors [46], human serum albumin (preferably recombinant), artificial proteins comprising multiple human CD4$^+$ T cell epitopes from various pathogen-derived antigens [47] such as N19 [48], protein D from *H.iuflueuzae* [49,50], pneumococcal surface protein PspA [51], pneumolysin [52], iron-uptake proteins [53], toxin A or B from *C.difficile* [54], recombinant *Pseudomonas aeruginosa* exoprotein A (rEPA) [55], a GBS protein (see below; particularly GBS67) [204], *etc.*

Attachment to the carrier is preferably via a -NH$_2$ group *e.g.* in the side chain of a lysine residue in a carrier protein, or of an arginine residue, or at the N-terminus. Attachment may also be via a -SH group *e.g.* in the side chain of a cysteine residue.

It is possible to use more than one carrier protein *e.g.* to reduce the risk of carrier suppression. Thus herein described is the use of different carrier proteins for different GBS serotypes *e.g.* serotype Ia saccharides might be conjugated to CRM197 while serotype Ib saccharides might be conjugated to tetanus toxoid. It is also possible to use more than one carrier protein for a particular saccharide antigen *e.g.* serotype III saccharides might be in two groups, with some conjugated to CRM197 and others conjugated to tetanus toxoid. In general, however, it is preferred to use the same carrier protein for all saccharides.

A single carrier protein might carry more than one saccharide antigen [56,57]. For example, a single carrier protein might have conjugated to it saccharides from serotypes Ia and Ib. To achieve this goal, different saccharides can be mixed prior to the conjugation reaction. In general, however, it is preferred to have separate conjugates for each serogroup, with the different saccharides being mixed after conjugation. The separate conjugates may be based on the same carrier.

Conjugates with a saccharide:protein ratio (w/w) of between 1:5 (*i.e.* excess protein) and 5:1 (*i.e.* excess saccharide) are typically used, in particular ratios between 1:5 and 2:1. When the invention uses a conjugate that is a capsular saccharide from GBS serotype Ia conjugated to a carrier protein, then the saccharide:protein ratio (w/w) is typically between about 1:1 to 1:2, particularly about 1:1.3. Similarly, when the invention uses a conjugate that is a capsular saccharide from GBS serotype Ib conjugated to a carrier protein, then the ratio is typically between about 1:1 to 1:2, particularly about 1:1.3. When the invention uses a conjugate that is a capsular saccharide from GBS serotype III conjugated to a carrier protein, then the saccharide:protein ratio (w/w) is typically between about 3:1 to 1:1, particularly about 2:1. However, GBS serotype III conjugated to a carrier protein with a saccharide:protein ratio (w/w) of about 1:1 to 1:5, particularly about 1:3.3, may also be used. Finally, when the invention uses a conjugate that is a capsular saccharide from GBS serotype V conjugated to a carrier protein, then the ratio is typically between about 2:1 to 1:1, particularly about 1.1:1. Thus a weight excess of saccharide is typical, particularly with longer saccharide chains.

Compositions may include a small amount of free carrier [58]. When a given carrier protein is present in both free and conjugated form in a composition of the invention, the unconjugated form is preferably no more than 5% of the total amount of the carrier protein in the composition as a whole, and more preferably present at less than 2% by weight.

After conjugation, free and conjugated saccharides can be separated. There are many suitable methods, including hydrophobic chromatography, tangential ultrafiltration, diafiltration *etc.* [see also refs. 59 & 60, *etc.*]. A preferred method is described in reference 61.

Where the composition of the invention includes a depolymerised oligosaccharide, it is preferred that depolymerisation precedes conjugation.


***Combinations of conjugates and other antigens***

The immunogenic compositions of the invention may comprise one or more further antigens.

The further antigen(s) may comprise further GBS conjugates. The different GBS conjugates may include different types of conjugate from the same GBS serotype and/or conjugates from different GBS serotypes. The composition will typically be produced by preparing separate conjugates (*e.g.* a different conjugate for each serotype) and then combining the conjugates.

The further antigen(s) may comprise GBS amino acid sequences, as set out below.

The further antigen(s) may comprise antigens from non-GBS pathogens. Thus the compositions of the invention may further comprise one or more non-GBS antigens, including additional bacterial, viral or parasitic antigens. These may be selected from the following:

- a protein antigen from *N.meningitidis* serogroup B, such as those in refs. 62 to 68, with protein '287' (see below) and derivatives (e.g. 'ΔG287') being particularly preferred.
- an outer-membrane vesicle (OMV) preparation from *N.meningitidis* serogroup B, such as those disclosed in refs. 69, 70, 71, 72 *etc.*
- a saccharide antigen from *N.meningitidis* serogroup A, C, W135 and/or Y, such as the oligosaccharide disclosed in ref. 73 from serogroup C or the oligosaccharides of ref. 74.
- a saccharide antigen from *Streptococcus pneumoniae* [*e.g.* refs. 75-77; chapters 22 & 23 of ref. 84].
- an antigen from hepatitis A virus, such as inactivated virus [*e.g.* 78, 79; chapter 15 of ref. 84].
- an antigen from hepatitis B virus, such as the surface and/or core antigens [*e.g.* 79,80; chpater 16 of ref. 84].
- an antigen from hepatitis C virus [*e.g.* 81].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 82 & 83; chapter 21 of ref. 84].
- a diphtheria antigen, such as a diphtheria toxoid [*e.g.* chapter 13 of ref. 84].
- a tetanus antigen, such as a tetanus toxoid [*e.g.* chapter 27 of ref. 84].
- a saccharide antigen from *Haemophilus influenzae* B [*e.g.* chapter 14 of ref. 84]
- an antigen from *N.gouorrhoeae* [*e.g.* 62, 63, 64].
- an antigen from *Chlamydia pueumouiae* [*e.g.* 85, 86, 87, 88, 89, 90, 91].
- an antigen from *Chlamydia trachomatis* [*e.g.* 92].
- an antigen from *Porphyromouas gingivalis* [*e.g.* 93].
- polio antigen(s) [*e.g.* 94, 95; chapter 24 of ref. 84] such as IPV.
- rabies antigen(s) [*e.g.* 96] such as lyophilised inactivated virus [*e.g.*97, RabAvert™].
- measles, mumps and/or rubella antigens [*e.g.* chapters 19, 20 and 26 of ref. 84].
- influenza antigen(s) [*e.g.* chapters 17 & 18 of ref. 84], such as the haemagglutinin and/or neuraminidase surface proteins.
- an antigen from *Moraxella catarrhalis* [*e.g.* 98].
- an antigen from *Streptococcus pyogenes* (group A streptococcus) [*e.g.* 99, 100, 101].
- an antigen from *Staphylococcus aureus* [*e.g.* 102].

Where a saccharide or carbohydrate antigen is used, it is preferably conjugated to a carrier in order to enhance immunogenicity. Conjugation of *H.influeuzae* B, meningococcal and pneumococcal saccharide antigens is well known.

Toxic protein antigens may be detoxified where necessary (*e.g.* detoxification of pertussis toxin by chemical and/or genetic means [83]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

One type of preferred composition includes further antigens from sexually-transmitted pathogens, such as: herpesvirus; *N.gouorrhoeae*; *C.trachomatis*; *etc.* Another type of preferred composition includes further antigens that affect the elderly and/or the immunocompromised, and so the GBS antigens of the invention can be combined with one or more antigens from the following non-GBS pathogens: influenza virus, *Euterococcus faecalis, Staphylococcus aureus, Staphylococcus epidermis, Pseudomonas aeruginosa, Legionella pneumophila, Listeria monocytogenes, Neisseria meningitidis,* and parainfluenza virus.

Antigens in the composition will typically be present at a concentration of at least 1µg/ml each. In general, the concentration of any given antigen will be sufficient to elicit an immune response against that antigen.

As an alternative to using proteins antigens in the composition of the invention, nucleic acid encoding the antigen may be used [*e.g.* refs. 103 to 111]. Protein components of the compositions of the invention may thus be replaced by nucleic acid (preferably DNA *e.g.* in the form of a plasmid) that encodes the protein.

In practical terms, there may be an upper limit to the number of antigens included in compositions of the invention. The number of antigens (including GBS antigens) in a composition of the invention may be less than 20, less than 19, less than 18, less than 17, less than 16, less than 15, less than 14, less than 13, less than 12, less than 11, less than 10, less than 9, less than 8, less than 7, less than 6, less than 5, less than 4, less than 3, or less than 2. The number of GBS antigens in a composition of the invention may be less than 6, less than 5, less than 4, less than 3, or less than 2.

### *Pharmaceutical methods and uses*

The immunogenic compositions of the invention may further comprise a pharmaceutically acceptable carrier. Typical 'pharmaceutically acceptable carriers' include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, sucrose [112], trehalose [113], lactose, and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. Sterile pyrogen-free, phosphate-buffered physiologic saline is a typical carrier. A thorough discussion of pharmaceutically acceptable excipients is available in reference 114.

Compositions of the invention may be in aqueous form (*i.e.* solutions or suspensions) or in a dried form (*e.g.* lyophilised). If a dried vaccine is used then it will be reconstituted into a liquid medium prior to injection. Lyophilisation of conjugate vaccines is known in the art *e.g.* the Menjugate™ product is presented in lyophilised form. When the immunogenic compositions of the invention include conjugates comprising capsular saccharides from more than one GBS serotypes, it is typical for the conjugates to be prepared separately, mixed and then lyophilised. In this way, lyophilised compositions comprising two, three or four *etc.* conjugates as described herein may be prepared. To stabilise conjugates during lyophilisation, it may be preferred to include a sugar alcohol (*e.g.* mannitol) and/or a disaccharide (*e.g.* sucrose or trehalose) *e.g.* at between 1mg/ml and 30mg/ml (*e.g.* about 25 mg/ml) in the composition. The use of sucrose has been recommended as a stabiliser for GBS conjugate vaccines (ref. 115). However, it is typical for the stabiliser of the present invention to be mannitol. When the dried vaccine is reconstituted into a liquid medium prior to injection, the concentration of residual mannitol will typically be about 2-20mg/ml, *e.g.* 3.75mg/ml, 7.5mg/ml or 15mg/ml. The use of mannitol is advantageous because mannitol is chemically distinct from the monosaccharide subunits of the GBS capsular saccharides. This means that detection of the capsular saccharides, *e.g.* for quality control analysis, can be based on the presence of the subunits of the saccharides without intereference from the mannitol. In contrast, a stabiliser like sucrose contains glucose, which may interfere with the detection of glucose subunits in the saccharides.

Compositions may be presented in vials, or they may be presented in ready-filled syringes. The syringes may be supplied with or without needles. A syringe will include a single dose of the composition, whereas a vial may include a single dose or multiple doses.

Aqueous compositions of the invention are also suitable for reconstituting other vaccines from a lyophilised form. Where a composition of the invention is to be used for such extemporaneous reconstitution, the invention provides a kit, which may comprise two vials, or may comprise one ready-filled syringe and one vial, with the contents of the syringe being used to reactivate the contents of the vial prior to injection.

Compositions of the invention may be packaged in unit dose form or in multiple dose form. For multiple dose forms, vials are preferred to pre-filled syringes. Effective dosage volumes can be routinely established, but a typical human dose of the composition has a volume of 0.5ml *e.g.* for for intramuscular injection.

The pH of the composition is preferably between 6 and 8, preferably about 7. Stable pH may be maintained by the use of a buffer. The immunogenic compositions of the invention typically comprise a potassium dihydrogen phosphate buffer. The potassium dihydrogen phosphate buffer may comprise about 1-10 mM potassium dihydrogen phosphate, *e.g.* 1.25 mM, 2.5 mM or 5.0 mM. The composition may be sterile and/or pyrogen-free. Compositions of the invention may be isotonic with respect to humans.

Compositions of the invention are immunogenic, and are more preferably vaccine compositions. Vaccines according to the invention may either be prophylactic (*i.e.* to prevent infection) or therapeutic (*i.e.* to treat infection), but will typically be prophylactic. Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

Within each dose, the quantity of an individual saccharide antigen will generally be between 0.1-50 $\mu$g (measured as mass of saccharide), particularly between 1-50 $\mu$g or 0.5-25$\mu$g, more particularly 2.5-7.5$\mu$g, *e.g.* about 1$\mu$g, about 2.5$\mu$g, about 5$\mu$g, about 10$\mu$g, about 15$\mu$g, about 20$\mu$g or about 25 $\mu$g. Within each dose, the total quantity of GBS capsular saccharides will generally be $\leq$ 70 $\mu$g (measured as mass of saccharide), *e.g.* $\leq$ 60 $\mu$g. In particular, the the total quantity may be $\leq$ 40 $\mu$g (*e.g.* $\leq$ 30 $\mu$g) or $\leq$ 20 $\mu$g (*e.g.* $\leq$ 15 $\mu$g). The inventors have found that these total quantities are effective, particularly when the immunogenic composition comprises: a) a conjugate that is a capsular saccharide from GBS serotype Ia conjugated to a carrier protein; b) a conjugate that is a capsular saccharide from GBS serotype Ib conjugated to a carrier protein; and c) a conjugate that is a capsular saccharide from GBS serotype III conjugated to a carrier protein. These total quantities are therefore preferred for use in the invention, particularly for this embodiment. It may be advantageous to minimise the total quantity of capsular saccharide(s) per unit dose in order to reduce potential toxicity. Accordingly, a total quantity of $\leq$ 20 $\mu$g is preferred, *e.g.* $\leq$ 15 $\mu$g, $\leq$ 7.5 $\mu$g or $\leq$ 1.5 $\mu$g.

GBS affects various areas of the body and so the compositions of the invention may be prepared in various forms. For example, the compositions may be prepared as injectables, either as liquid solutions or suspensions. The composition may be prepared for pulmonary administration *e.g.* as an inhaler, using a fine powder or a spray. The composition may be prepared as a suppository or pessary. The composition may be prepared for nasal, aural or ocular administration *e.g.* as spray, drops, gel or powder [*e.g.* refs 116 & 117]. Success with nasal administration of pneumococcal saccharides [118,119], Hib saccharides [120], MenC saccharides [121], and mixtures of Hib and MenC saccharide conjugates [122] has been reported.

Compositions of the invention may include an antimicrobial, particularly when packaged in multiple dose format.

Compositions of the invention may comprise detergent *e.g.* a Tween (polysorbate), such as Tween 80. Detergents are generally present at low levels *e.g.* <0.01%.

Compositions of the invention may include sodium salts (*e.g.* sodium chloride) to give tonicity. A concentration of $10\pm2$mg/ml NaCl is typical. In some embodiments, a concentration of 4-10mg/ml NaCl may be used, *e.g.* 9.0, 7.0, 6.75 or 4.5 mg/ml.

Compositions of the invention will generally include a buffer. A phosphate buffer is typical.

Compositions of the invention may be administered in conjunction with other immunoregulatory agents. Compositions may include one or more adjuvants but does not contain an aluminium salt adjuvant. Such adjuvants include, but are not limited to:

*A. Mineral-containing compositions*

Mineral containing compositions suitable for use as adjuvants in the invention include mineral salts, such as calcium salts (or mixtures thereof). Calcium salts include calcium phosphate (*e.g.* the "CAP" particles disclosed in ref. 123). Adsorption to these salts is preferred. The mineral containing compositions may also be formulated as a particle of metal salt [124].

*B. Oil Emulsions*

Oil emulsion compositions suitable for use as adjuvants in the invention include squalene-water emulsions, such as MF59 (5% Squalene, 0.5% Tween 80, and 0.5% Span 85, formulated into submicron particles using a microfluidizer) [; see also refs. 125-127]. MF59 is used as the adjuvant in the FLUAD™ influenza virus trivalent subunit vaccine.

Particularly preferred adjuvants for use in the compositions are submicron oil-in-water emulsions. Preferred submicron oil-in-water emulsions for use herein are squalene/water emulsions optionally containing varying amounts of MTP-PE, such as a submicron oil-in-water emulsion containing 4-5% w/v squalene, 0.25-1.0% w/v Tween 80 (polyoxyelthylenesorbitan monooleate), and/or 0.25-1.0% Span 85 (sorbitan trioleate), and, optionally, N-acetylmuramyl-L-alanyl-D-isogluatminyl-L-alanine-2-(1'-2'-dipalmitoyl-sn-glycero-3-hydroxyphosphophoryloxy)-ethylamine (MTP-PE). Submicron oil-in-water emulsions, methods of making the same and immunostimulating agents, such as muramyl peptides, for use in the compositions, are described in detail in references 125 & 128-129. Complete Freund's adjuvant (CFA) and incomplete Freund's adjuvant (IFA) may also be used as adjuvants in the invention.

C. Saponin formulations

Saponin formulations may also be used as adjuvants in the invention. Saponins are a heterologous group of sterol glycosides and triterpenoid glycosides that are found in the bark, leaves, stems, roots and even flowers of a wide range of plant species. Saponins isolated from the bark of the *Quillaia sapouaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 130. Saponin formulations may also comprise a sterol, such as cholesterol [131].

Combinations of saponins and cholesterols can be used to form unique particles called immunostimulating complexs (ISCOMS). ISCOMs typically also include a phospholipid such as phosphatidylethanolamine or phosphatidylcholine. Any known saponin can be used in ISCOMs. Preferably, the ISCOM includes one or more of QuilA, QHA and QHC. ISCOMs are further described in refs. 131-133. Optionally, the ISCOMS may be devoid of additional detergent(s) [134].

A review of the development of saponin based adjuvants can be found in refs. 135 & 136.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qβ-phage (such as coat proteins), GA-phage, fr-phage, AP205 phage, and Ty (such as retrotransposon Ty protein p1). VLPs are discussed further in refs. 137-142. Virosomes are discussed further in, for example, ref. 143

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 144. Such "small particles" of 3dMPL are small enough to be sterile filtered through a 0.22μm membrane [144]. Other non-toxic LPS derivatives include monophosphoryl lipid A mimics, such as aminoalkyl glucosaminide phosphate derivatives e.g. RC-529 [145,146].

Lipid A derivatives include derivatives of lipid A from *Escherichia coli* such as OM-174. OM-174 is described for example in refs. 147 & 148.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 149, 150 and 151 disclose possible analog substitutions e.g. replacement of guanosine with 2'-deoxy-7-deazaguanosine. The adjuvant effect of CpG oligonucleotides is further discussed in refs. 152-157.

The CpG sequence may be directed to TLR9, such as the motif GTCGTT or TTCGTT [158]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 159-161. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 158 & 162-164.

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 165 and as parenteral adjuvants in ref. 166. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivaties thereof, particularly LT-K63 and LT-R72, as adjuvants can be found in refs. 167-174. Numerical reference for amino acid substitutions is preferably based on the alignments of the A and B subunits of ADP-ribosylating toxins set forth in ref. 175, specifically incorporated herein by reference in its entirety.

### F. Human immunomodulators

Human immunomodulators suitable for use as adjuvants in the invention include cytokines, such as interleukins (e.g. IL-1, IL-2, IL-4, IL-5, IL-6, IL-7, IL-12 [176], etc.) [177], interferons (e.g. interferon-y), macrophage colony stimulating factor, and tumor necrosis factor.

### G. Bioadhesives and Mucoadhesives

Bioadhesives and mucoadhesives may also be used as adjuvants in the invention. Suitable bioadhesives include esterified hyaluronic acid microspheres [178] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [179].

*H. Microparticles*

Microparticles may also be used as adjuvants in the invention. Microparticles (*i.e.* a particle of ~100nm to ~150$\mu$m in diameter, more preferably ~200nm to ~30$\mu$m in diameter, and most preferably ~500nm to ~10$\mu$m in diameter) formed from materials that are biodegradable and non-toxic (*e.g.* a poly($\alpha$-hydroxy acid), a polyhydroxybutyric acid, a polyorthoester, a polyanhydride, a polycaprolactone, *etc.*), with poly(lactide-co-glycolide) are preferred, optionally treated to have a negatively-charged surface (*e.g.* with SDS) or a positively-charged surface (*e.g.* with a cationic detergent, such as CTAB).

I. Liposomes

Examples of liposome formulations suitable for use as adjuvants are described in refs. 180-182.

*J. Polyoxyethylene ether and polyoxyethylene ester formulations*

Adjuvants suitable for use in the invention include polyoxyethylene ethers and polyoxyethylene esters [183]. Such formulations further include polyoxyethylene sorbitan ester surfactants in combination with an octoxynol [184] as well as polyoxyethylene alkyl ethers or ester surfactants in combination with at least one additional non-ionic surfactant such as an octoxynol [185]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

*K. Polyphosphazene (PCPP)*

PCPP formulations are described, for example, in refs. 186 and 187.

*L. Muramyl peptides*

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE).

*M. Imidazoguinolone Compounds.*

Examples of imidazoquinolone compounds suitable for use adjuvants in the invention include Imiquamod and its homologues (*e,g.* "Resiquimod 3M"), described further in refs. 188 and 189.

*N. Thiosemicarbazone Compounds.*

Examples of thiosemicarbazone compounds, as well as methods of formulating, manufacturing, and screening for compounds all suitable for use as adjuvants in the invention include those described in ref. 190. The thiosemicarbazones are particularly effective in the stimulation of human peripheral blood mononuclear cells for the production of cytokines, such as TNF-$\alpha$.

*O. Tryptanthrin Compounds.*

Examples of tryptanthrin compounds, as well as methods of formulating, manufacturing, and screening for compounds all suitable for use as adjuvants in the invention include those described in ref. 191. The tryptanthrin compounds are particularly effective in the stimulation of human peripheral blood mononuclear cells for the production of cytokines, such as TNF-$\alpha$.

The invention may also comprise combinations of aspects of one or more of the adjuvants identified above. For example, the following combinations may be used as adjuvant compositions in the invention: (1) a saponin and an oil-in-water emulsion [192]; (2) a saponin (*e.g.* QS21) + a non-toxic LPS derivative (*e.g.* 3dMPL) [193]; (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) [194]; (5) combinations of 3dMPL with, for example, QS21 and/or oil-in-water emulsions [195]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts + a non-toxic derivative of LPS (such as 3dMPL).

Not all conjugates need to be adsorbed *i.e.* some or all can be free in solution.

### *Methods of treatment*

The invention also provides immunogenic compositions for use in a method for raising an immune response in a mammal, comprising administering a pharmaceutical composition of the invention to the mammal. The immune response is preferably protective and preferably involves antibodies. The method may raise a booster response.

The mammal is preferably a human. Where the vaccine is for prophylactic use, the human is preferably a child (*e.g.* a toddler or infant) or a teenager; where the vaccine is for therapeutic use, the human is preferably an adult. A vaccine intended for children may also be administered to adults *e.g.* to assess safety, dosage, immunogenicity, *etc.* A preferred class of humans for treatment are females of child-bearing age (*e.g.* teenagers and above). Another preferred class is pregnant females. Elderly patients (*e.g.* those above 50, 60, 70, 80 or 90 *etc.* years of age, particularly over 65 years of age), especially those living in nursing homes where the risk of GBS infection may be increased ([196]), are another preferred class of humans for treatment. In some embodiments, the human has an undetectable level of antibodies against capsular saccharide from GBS serotype Ia prior to administration of the pharmaceutical composition. In other embodiments, the human has an undetectable level of antibodies against capsular saccharide from GBS serotype Ib prior to administration of the pharmaceutical composition. In other embodiments, the human has an undetectable level of antibodies against capsular saccharide from GBS serotype III prior to administration of the pharmaceutical composition. In particular, the human may have an undetectable level of antibodies against capsular saccharide from GBS serotype Ia and an undetectable level of antibodies against capsular saccharide from GBS serotype Ib prior to administration of the pharmaceutical composition. Alternatively or in addition, the human may have an undetectable level of antibodies against capsular saccharide from GBS serotype III prior to administration of the pharmaceutical composition. The level(s) of antibodies against the capsular saccharide(s) may be determined using the ELISA described in *Human study (1)* below. The level(s) of antibodies may be as of one month prior to administration, particularly within one month prior to administration (*e.g.* within two weeks, within one week or on the day of administration). Women with these undetectable level(s) of antibodies against the capsular saccharide(s) may have higher rates of GBS infection in their newborns. This is because higher levels of maternal antibodies against GBS capsular saccharides are correleated with reduced risk of disease in newborns [refs. 197 and 198]. Accordingly, administration to these women is specifically envisaged in the present invention.

In some embodiments, the patient has been pre-immunised with a diphtheria toxoid or derivative thereof, *e.g.* as described below with respect to the second aspect of the invention in the section *The pre-immunised patient.* In these embodiments, it is preferred for at least one conjugate in the immunogenic composition to be a capsular saccharide from GBS conjugated to a diphtheria toxoid or derivative thereof. The inventors have found that the immune response to the capsular saccharide may be improved by presenting the saccharide on a diphtheria toxoid or derivative thereof, when the patient has been pre-immunised with a diphtheria toxoid or derivative thereof. The capsular saccharide conjugated to the diphtheria toxoid or derivative thereof in the composition may for example be from GBS serotype Ia, Ib or III. In particular, the capsular saccharide may be from GBS serotype III (as exemplified below). In these embodiments, it is typical for all of the capsular saccharides from GBS in the composition to be conjugated to a diphtheria toxoid or derivative thereof. Where the carrier or pre-immunisation antigen is a derivative of a diphtheria toxoid then that derivative preferably remains immunologically cross-reactive with Dt, and is preferably CRM197.

The invention also provides a composition of the invention for use as a medicament. The medicament is preferably able to raise an immune response in a mammal (*i.e.* it is an immunogenic composition) and is more preferably a vaccine.

The invention also provides the use of a composition of the invention in the manufacture of a medicament for raising an immune response in a mammal.

These uses and methods are preferably for the prevention and/or treatment of a disease caused by *S.agalactiae e.g.* neonatal sepsis or bacteremia, neonatal pneumonia, neonatal meningitis, endometritis, osteomyelitis, septic arthritis, *etc.*

The subject in which disease is prevented may not be the same as the subject that receives the conjugate of the invention. For instance, a conjugate may be administered to a female (before or during pregnancy) in order to protect offspring (so-called 'maternal immunisation' [199-201]).

One way of checking efficacy of therapeutic treatment involves monitoring GBS infection after administration of the composition of the invention. One way of checking efficacy of prophylactic treatment involves monitoring immune responses against the GBS antigens after administration of the composition.

Preferred compositions of the invention can confer an antibody titre in a patient that is superior to the criterion for seroprotection for each antigenic component for an acceptable percentage of human subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration to the thigh or the upper arm is preferred. Injection may be via a needle (*e.g.* a hypodermic needle), but needle-free injection may alternatively be used. A typical intramuscular dose is 0.5 ml.

The invention may be used to elicit systemic and/or mucosal immunity.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

### *GBS protein antigens*

As mentioned above, GBS proteins can be included in compositions of the invention. These may be used as carrier proteins for conjugates of the invention, carrier proteins for other conjugates, or as unconjugated protein antigens.

GBS protein antigens for use with the invention include those disclosed in references 99 and 202-204. Two preferred GBS protein antigens for use with the invention are known as: GBS67; and GBS80 [see ref. 99]. A further preferred GBS protein antigen for use with the invention is known as Spb1 [see ref. 205]. Further details of these three antigens are given below.

The full-length sequences for these three GBS proteins are SEQ ID NOs 1 to 3 herein. Compositions of the invention may thus include (a) a polypeptide comprising an amino acid sequence selected from SEQ ID NOs 1 to 3, and/or (b) a polypeptide comprising (i) an amino acid sequence that has sequence identity to one or more of SEQ ID NOs 1 to 3 and/or (ii) a fragment of SEQ ID NOs 1 to 3.

Compositions of the invention may also comprise mixtures of these GBS protein antigens.

In particular, compositions of the invention may include:

($a_1$) a polypeptide comprising an amino acid sequence of SEQ ID NO 1, and/or ($b_1$) a polypeptide comprising (i) an amino acid sequence that has sequence identity to SEQ ID NO 1 and/or (ii) a fragment of SEQ ID NO 1; and

($a_2$) a polypeptide comprising an amino acid sequence of SEQ ID NO 2, and/or ($b_2$) a polypeptide comprising (i) an amino acid sequence that has sequence identity to SEQ ID NO 2 and/or (ii) a fragment of SEQ ID NO 2.

Similarly, compositions of the invention may include:

($a_1$) a polypeptide comprising an amino acid sequence of SEQ ID NO 1, and/or ($b_1$) a polypeptide comprising (i) an amino acid sequence that has sequence identity to SEQ ID NO 1 and/or (ii) a fragment of SEQ ID NO 1; and

($a_2$) a polypeptide comprising an amino acid sequence of SEQ ID NO 3, and/or ($b_2$) a polypeptide comprising (i) an amino acid sequence that has sequence identity to SEQ ID NO 3 and/or (ii) a fragment of SEQ ID NO 3.

In the same way, compositions of the invention may include:

($a_1$) a polypeptide comprising an amino acid sequence of SEQ ID NO 2, and/or ($b_1$) a polypeptide comprising (i) an amino acid sequence that has sequence identity to SEQ ID NO 2 and/or (ii) a fragment of SEQ ID NO 2; and

($a_2$) a polypeptide comprising an amino acid sequence of SEQ ID NO 3, and/or ($b_2$) a polypeptide comprising (i) an amino acid sequence that has sequence identity to SEQ ID NO 3 and/or (ii) a fragment of SEQ ID NO 3.

Compositions of the invention may include:

(a$_1$) a polypeptide comprising an amino acid sequence of SEQ ID NO 1, and/or (b$_1$) a polypeptide comprising (i) an amino acid sequence that has sequence identity to SEQ ID NO 1 and/or (ii) a fragment of SEQ ID NO 1;

(a$_2$) a polypeptide comprising an amino acid sequence of SEQ ID NO 2, and/or (b$_2$) a polypeptide comprising (i) an amino acid sequence that has sequence identity to SEQ ID NO 2 and/or (ii) a fragment of SEQ ID NO 2; and

(a$_3$) a polypeptide comprising an amino acid sequence of SEQ ID NO 3, and/or (b$_3$) a polypeptide comprising (i) an amino acid sequence that has sequence identity to SEQ ID NO 3 and/or (ii) a fragment of SEQ ID NO 3.

Three other preferred GBS protein antigens for use with the invention are known as: GBS104; GBS276; and GBS322 [see ref. 99]. The wild-type GBS104 amino acid sequence from serotype V isolated strain 2603 V/R is given in reference 21 as SEQ ID NO: 3 therein. Where embodiments of the present invention are defined herein by reference to SEQ ID NO: 1, the references to SEQ ID NO: 1 may be substituted by references to SEQ ID NO: 3 from reference 21. The wild-type GBS276 amino acid sequence from serotype V isolated strain 2603 V/R is given in reference 21 as SEQ ID NO: 4 therein. Where embodiments of the present invention are defined herein by reference to SEQ ID NO: 2, the references to SEQ ID NO: 2 may be substituted by references to SEQ ID NO: 4 from reference 21. The wild-type GBS322 amino acid sequence from serotype V isolated strain 2603 V/R is given in reference 21 as SEQ ID NO: 5 therein. Where embodiments of the present invention are defined herein by reference to SEQ ID NO: 3, the references to SEQ ID NO: 3 may be substituted by references to SEQ ID NO: 5 from reference 21.

Depending on the particular SEQ ID NO, the degree of sequence identity in (i) is preferably greater than 50% (*e.g.* 60%, 70%, 75%, 80%, 85%, 90%, 91%, 92%, 93%, 94%, 95%, 96%, 97%, 98%, 99% or more). These polypeptides include homologs, orthologs, allelic variants and functional mutants. Typically, 50% identity or more between two polypeptide sequences is considered to be an indication of functional equivalence. Identity between polypeptides is preferably determined by the Smith-Waterman homology search algorithm as implemented in the MPSRCH program (Oxford Molecular), using an affine gap search with parameters *gap open penalty=12* and *gap extension penalty=1.*

Depending on the particular SEQ ID NO, the fragments of (ii) should comprise at least *n* consecutive amino acids from the sequences and, depending on the particular sequence, *n* is 7 or more (*e.g.* 8, 10, 12, 14, 16, 18, 20, 22, 24, 26, 28, 30, 35, 40, 45, 50, 60, 70, 80, 90, 100 or more). The fragment may comprise at least one T-cell or, preferably, a B-cell epitope of the sequence. T- and B-cell epitopes can be identified empirically (*e.g.* using PEPSCAN [206,207] or similar methods), or they can be predicted (*e.g.* using the Jameson-Wolf antigenic index [208], matrix-based approaches [209], TEPITOPE [210], neural networks [211], OptiMer & EpiMer [212, 213], ADEPT [214], Tsites [215], hydrophilicity [216], antigenic index [217] or the methods disclosed in reference 218 *etc.*). Other preferred fragments are SEQ ID NOs 1 to 3 without their N-terminal amino acid residue or without their N-terminal signal peptide. Removal of one or more domains, such as a leader or signal sequence region, a transmembrane region, a cytoplasmic region or a cell wall anchoring motif can be used. Preferred fragments of a particular protein can bind to an antibody that can bind to the full-length particular protein *e.g.* can bind to an antibody that binds to SEQ ID NO: 1, 2 or 3. Some useful fragments are given below (SEQ ID NOs 4 to 13).

These polypeptides may, compared to SEQ ID NOs 1 to 3, include one or more (*e.g.* 1, 2, 3, 4, 5, 6, 7, 8, 9, 10, *etc.*) conservative amino acid replacements *i.e.* replacements of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cystine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may also include one or more (*e.g.* 1, 2, 3, 4, 5, 6, 7, 8, 9, 10, *etc.*) single amino acid deletions relative to SEQ ID NOs 1 to 3. The polypeptides may also include one or more (*e.g.* 1, 2, 3, 4, 5, 6, 7, 8, 9, 10, *etc.*) insertions (*e.g.* each of 1, 2, 3, 4 or 5 amino acids) relative to the SEQ ID NOs 1 to 3.

Polypeptides of the invention can be prepared in many ways e.g. by chemical synthesis (in whole or in part), by digesting longer polypeptides using proteases, by translation from RNA, by purification from cell culture (*e.g.* from recombinant expression), from the organism itself (*e.g.* after bacterial culture, or direct from patients), *etc.* A preferred method for production of peptides <40 amino acids long involves *in vitro* chemical synthesis [219,220]. Solid-phase peptide synthesis is particularly preferred, such as methods based on tBoc or Fmoc [221] chemistry. Enzymatic synthesis [222] may also be used in part or in full. As an alternative to chemical synthesis, biological synthesis may be used *e.g.* the polypeptides may be produced by translation. This may be carried out *in vitro* or *in vivo.* Biological methods are in general restricted to the production of polypeptides based on L-amino acids, but manipulation of translation machinery (*e.g.* of aminoacyl tRNA molecules) can be used to allow the introduction of D-amino acids (or of other non natural amino acids, such as iodotyrosine or methylphenylalanine, azidohomoalanine, etc.) [223]. Where D-amino acids are included, however, it is preferred to use chemical synthesis. Polypeptides of the invention may have covalent modifications at the C-terminus and/or N-terminus.

If these GBS proteins are included in compositions of the invention then they can take various forms (*e.g.* native, fusions, glycosylated, non-glycosylated, lipidated, non-lipidated, phosphorylated, non-phosphorylated, myristoylated, non-myristoylated, monomeric, multimeric, particulate, denatured, *etc*.). They are preferably used in purified or substantially purified form *i.e.* substantially free from other polypeptides (*e.g.* free from naturally-occurring polypeptides), particularly from other GBS or host cell polypeptides).

*GBS67*

Nucleotide and amino acid sequence of GBS67 sequenced from serotype V strain 2603 V/R are set forth in ref. 99 as SEQ ID NOs 3745 & 3746. The amino acid sequence is SEQ ID NO:1 herein:

```
MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE
NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV
MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGI IPMTGGKGILS
FILIGGAMMSIAGGIYIWKRYKKSSDMSIKKD
```

GBS67 contains a C-terminus transmembrane region which is indicated by the underlined region closest to the C-terminus of SEQ ID NO: 1 above. One or more amino acids from the transmembrane region may be removed, or the amino acid may be truncated before the transmembrane region. An example of such a GBS67 fragment is set forth below as SEQ ID NO: 4.

```
MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE

NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV
MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGIIPMTGGKGILS
```

GBS67 contains an amino acid motif indicative of a cell wall anchor, shown in italics in SEQ ID NO: 1 above. In some recombinant host cell systems, it may be preferable to remove this motif to facilitate secretion of a recombinant GBS67 protein from the host cell. Accordingly, in one preferred fragment of GBS67 for use in the invention, the transmembrane and the cell wall anchor motif are removed from GBS67. An example of such a GBS67 fragment is set forth below as SEQ ID NO: 5.

```
MRKYQKFSKILTLSLFCLSQIPLNTNVLGESTVPENGAKGKLVVKKTDDQNKPLSKATFVLKTTAHPESKIEKVTAELT
GEATFDNLIPGDYTLSEETAPEGYKKTNQTWQVKVESNGKTTIQNSGDKNSTIGQNQEELDKQYPPTGIYEDTKESYKL
EHVKGSVPNGKSEAKAVNPYSSEGEHIREIPEGTLSKRISEVGDLAHNKYKIELTVSGKTIVKPVDKQKPLDVVFVLDN
SNSMNNDGPNFQRHNKAKKAAEALGTAVKDILGANSDNRVALVTYGSDIFDGRSVDVVKGFKEDDKYYGLQTKFTIQTE
NYSHKQLTNNAEEIIKRIPTEAPKAKWGSTTNGLTPEQQKEYYLSKVGETFTMKAFMEADDILSQVNRNSQKIIVHVTD
GVPTRSYAINNFKLGASYESQFEQMKKNGYLNKSNFLLTDKPEDIKGNGESYFLFPLDSYQTQIISGNLQKLHYLDLNL
NYPKGTIYRNGPVKEHGTPTKLYINSLKQKNYDIFNFGIDISGFRQVYNEEYKKNQDGTFQKLKEEAFKLSDGEITELM
RSFSSKPEYYTPIVTSADTSNNEILSKIQQQFETILTKENSIVNGTIEDPMGDKINLQLGNGQTLQPSDYTLQGNDGSV
MKDGIATGGPNNDGGILKGVKLEYIGNKLYVRGLNLGEGQKVTLTYDVKLDDSFISNKFYDTNGRTTLNPKSEDPNTLR
DFPIPKIRDVREYPTITIKNEKKLGEIEFIKVDKDNNKLLLKGATFELQEFNEDYKLYLPIKNNNSKVVTGENGKISYK
DLKDGKYQLIEAVSPEDYQKITNKPILTFEVVKGSIKNIIAVNKQISEYHEEGDKHLITNTHIPPKGI
```

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

Three pilin motifs, containing conserved lysine residues have been identified in GBS67. Conserved lysine residues are at amino acid residues 478 and 488, at amino acid residues 340 and 342, and at amino acid residues 703 and 717. The pilin sequences, in particular the conserved lysine residues, are thought to be important for the formation of oligomeric, pilus-like structures of GBS67. Preferred fragments of GBS 67 include at least one conserved lysine residue. Two E boxes containing conserved glutamic residues have also been identified in GBS67. Preferred fragments of GBS 67 include at least one conserved glutamic acid residue. GBS67 contains several regions predicted to form alpha helical structures. Such alpha helical regions are likely to form coiled-coil structures and may be involved in oligomerization of GBS67. GBS67 also contains a region which is homologous to the Cna_B domain of the *S.aureus* collagen-binding surface protein (pfam05738). This may form a beta sandwich structure. GBS67 contains a region which is homologous to a von Willebrand factor (vWF) type A domain.

The amino acid sequence of GBS67 sequenced from serotype Ib strain H36B is set forth in ref. 224 as SEQ ID NO 20906. The amino acid sequence is SEQ ID NO: 24 herein:

```
MRKYQKFSKILTLSLFCLSQIPLNTNVLGESTVPENGAKGKLVVKKTDDQNKPLSKATFVLKPTSHSESKVEKVTTEVT
GEATFDNLTPGDYTLSEETAPEGYKKTTQTWQVKVESNGKTTIQNSDDKKSIIEQRQEELDKQYPLTGAYEDTKESYNL
EHVKNSIPNGKLEAKAVNPYSSEGEHIREIQEGTLSKRISEVNDLDHNKYKIELTVSGKSIIKTINKDEPLDVVFVLDN
SNSMKNNGKNNKAKKAGEAVETIIKDVLGANVENRAALVTYGSDIFDGRTVKVIKGFKEDPYYGLETSFTVQTNDYSYK
KFTNIAADIIKKIPKEAPEAKWGGTSLGLTPEKKREYDLSKVGETFTMKAFMEADTLLSSIQRKSRKIIVHLTDGVPTR

SYAINSFVKGSTYANQFERIKEKGYLDKNNYFITDDPEKIKGNGESYFLFPLDSYQTQIISGNLQKLHYLDLNLNYPKG
TIYRNGPVREHGTPTKLYINSLKQKNYDIFNFGIDISGFRQVYNEDYKKNQDGTFQKLKEEAFELSDGEITELMNSFSS
KPEYYTPIVTSADVSNNEILSKIQQQFEKILTKENSIVNGTIEDPMGDKINLHLGNGQTLQPSDYTLQGNDGSIMKDSI
ATGGPNNDGGILKGVKLEYIKNKLYVRGLNLGEGQKVTLTYDVKLDDSFISNKFYDTNGRTTLNPKSEEPDTLRDFPIP
KIRDVREYPTITIKNEKKLGEIEFTKVDKDNNKLLLKGATFELQEFNEDYKLYLPIKNNNSKVVTGENGKISYKDLKDG
KYQLIEAVSPKDYQKITNKPILTFEVVKGSIQNIIAVNKQISEYHEEGDKHLITNTHIPPKGI*IPMTGG*KGILS̲F̲I̲L̲I̲G̲
G̲A̲M̲M̲S̲I̲A̲G̲G̲I̲Y̲I̲W̲KRHKKSSDASIEKD
```

In some embodiments, this variant of GBS67 may be used. Accordingly, where embodiments of the present invention are defined herein by reference to SEQ ID NO: 1, the references to SEQ ID NO: 1 may be substituted by references to SEQ ID NO: 24.

Like GBS67 sequenced from serotype V strain 2603 V/R, GBS67 sequenced from serotype Ib strain H36B contains a C-terminus transmembrane region which is indicated by the underlined region closest to the C-terminus of SEQ ID NO: 24 above. One or more amino acids from the transmembrane region may be removed, or the amino acid may be truncated before the transmembrane region. An example of such a GBS67 fragment is set forth below as SEQ ID NO: 25.

```
MRKYQKFSKILTLSLFCLSQIPLNTNVLGESTVPENGAKGKLVVKKTDDQNKPLSKATFVLKPTSHSESKVEKVTTEVT
GEATFDNLTPGDYTLSEETAPEGYKKTTQTWQVKVESNGKTTIQNSDDKKSIIEQRQEELDKQYPLTGAYEDTKESYNL
EHVKNSIPNGKLEAKAVNPYSSEGEHIREIQEGTLSKRISEVNDLDHNKYKIELTVSGKSIIKTINKDEPLDVVFVLDN
SNSMKNNGKNNKAKKAGEAVETIIKDVLGANVENRAALVTYGSDIFDGRTVKVIKGFKEDPYYGLETSFTVQTNDYSYK
KFTNIAADIIKKIPKEAPEAKWGGTSLGLTPEKKREYDLSKVGETFTMKAFMEADTLLSSIQRKSRKIIVHLTDGVPTR
SYAINSFVKGSTYANQFERIKEKGYLDKNNYFITDDPEKIKGNGESYFLFPLDSYQTQIISGNLQKLHYLDLNLNYPKG
TIYRNGPVREHGTPTKLYINSLKQKNYDIFNFGIDISGFRQVYNEDYKKNQDGTFQKLKEEAFELSDGEITELMNSFSS
KPEYYTPIVTSADVSNNEILSKIQQQFEKILTKENSIVNGTIEDPMGDKINLHLGNGQTLQPSDYTLQGNDGSIMKDSI
ATGGPNNDGGILKGVKLEYIKNKLYVRGLNLGEGQKVTLTYDVKLDDSFISNKFYDTNGRTTLNPKSEEPDTLRDFPIP
KIRDVREYPTITIKNEKKLGEIEFTKVDKDNNKLLLKGATFELQEFNEDYKLYLPIKNNNSKVVTGENGKISYKDLKDG
KYQLIEAVSPKDYQKITNKPILTFEVVKGSIQNIIAVNKQISEYHEEGDKHLITNTHIPPKGIIPMTGGKGILS
```

Like GBS67 sequenced from serotype V strain 2603 V/R, GBS67 sequenced from serotype Ib strain H36B contains an amino acid motif indicative of a cell wall anchor, shown in italics in SEQ ID NO: 24 above. Accordingly, in one preferred fragment of GBS67 for use in the invention, the transmembrane and the cell wall anchor motif are removed from GBS67. An example of such a GBS67 fragment is set forth below as SEQ ID NO: 26.

```
MRKYQKFSKILTLSLFCLSQIPLNTNVLGESTVPENGAKGKLVVKKTDDQNKPLSKATFVLKPTSHSESKVEKVTTEVT
GEATFDNLTPGDYTLSEETAPEGYKKTTQTWQVKVESNGKTTIQNSDDKKSIIEQRQEELDKQYPLTGAYEDTKESYNL
EHVKNSIPNGKLEAKAVNPYSSEGEHIREIQEGTLSKRISEVNDLDHNKYKIELTVSGKSIIKTINKDEPLDVVFVLDN
SNSMKNNGKNNKAKKAGEAVETIIKDVLGANVENRAALVTYGSDIFDGRTVKVIKGFKEDPYYGLETSFTVQTNDYSYK
KFTNIAADIIKKIPKEAPEAKWGGTSLGLTPEKKREYDLSKVGETFTMKAFMEADTLLSSIQRKSRKIIVHLTDGVPTR
SYAINSFVKGSTYANQFERIKEKGYLDKNNYFITDDPEKIKGNGESYFLFPLDSYQTQIISGNLQKLHYLDLNLNYPKG
TIYRNGPVREHGTPTKLYINSLKQKNYDIFNFGIDISGFRQVYNEDYKKNQDGTFQKLKEEAFELSDGEITELMNSFSS
KPEYYTPIVTSADVSNNEILSKIQQQFEKILTKENSIVNGTIEDPMGDKINLHLGNGQTLQPSDYTLQGNDGSIMKDSI
ATGGPNNDGGILKGVKLEYIKNKLYVRGLNLGEGQKVTLTYDVKLDDSFISNKFYDTNGRTTLNPKSEEPDTLRDFPIP
KIRDVREYPTITIKNEKKLGEIEFTKVDKDNNKLLLKGATFELQEFNEDYKLYLPIKNNNSKVVTGENGKISYKDLKDG
KYQLIEAVSPKDYQKITNKPILTFEVVKGSIQNIIAVNKQISEYHEEGDKHLITNTHIPPKGI
```

## GBS80

GBS80 refers to a putative cell wall surface anchor family protein. Nucleotide and amino acid sequence of GBS80 sequenced from serotype V isolated strain 2603 V/R are set forth in ref. 99 as SEQ ID NOs 8779 & 8780. The amino acid sequence is set forth below as SEQ ID NO: 2:

```
MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
```

```
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPSIPNTGGIGTAIFVAIGAAVMAFAVKGMKRRTKD
N
```

GBS80 contains a N-terminal leader or signal sequence region which is indicated by the underlined sequence above. One or more amino acids from the leader or signal sequence region of GBS80 can be removed. An example of such a GBS80 fragment is set forth below as SEQ ID NO: 6:

```
AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN
IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKGMTLVKNQDALDKATANTDDAAFLEIPVASTINEKAVLGKAIENTF
ELQYDHTPDKADNPKPSNPPRKPEVHTGGKRFVKKDSTETQTLGGAEFDLLASDGTAVKWTDALIKANTNKNYIAGEAV
TGQPIKLKSHTDGTFEIKGLAYAVDANAEGTAVTYKLKETKAPEGYVIPDKEIEFTVSQTSYNTKPTDITVDSADATPD
TIKNNKRPSIPNTGGIGTAIFVAIGAAVMAFAVKGMKRRTKDN
```

GBS80 contains a C-terminal transmembrane region which is indicated by the underlined sequence near the end of SEQ ID NO: 2 above. One or more amino acids from the transmembrane region and/or a cytoplasmic region may be removed. An example of such a fragment is set forth below as SEQ ID NO:7:

```
MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPS IPNTG
```

GBS80 contains an amino acid motif indicative of a cell wall anchor, shown in italics in SEQ ID NO: 2 above. In some recombinant host cell systems, it may be preferable to remove this motif to facilitate secretion of a recombinant GBS80 protein from the host cell. Thus the transmembrane and/or cytoplasmic regions and the cell wall anchor motif may be removed from GBS80. An example of such a fragment is set forth below as SEQ ID NO: 8.

```
MKLSKKLLFSAAVLTMVAGSTVEPVAQFATGMSIVRAAEVSQERPAKTTVNIYKLQADSYKSEITSNGGIENKDGEVIS
NYAKLGDNVKGLQGVQFKRYKVKTDISVDELKKLTTVEAADAKVGTILEEGVSLPQKTNAQGLVVDALDSKSNVRYLYV
EDLKNSPSNITKAYAVPFVLELPVANSTGTGFLSEINIYPKNVVTDEPKTDKDVKKLGQDDAGYTIGEEFKWFLKSTIP
ANLGDYEKFEITDKFADGLTYKSVGKIKIGSKTLNRDEHYTIDEPTVDNQNTLKITFKPEKFKEIAELLKGMTLVKNQD
ALDKATANTDDAAFLEIPVASTINEKAVLGKAIENTFELQYDHTPDKADNPKPSNPPRKPEVHTGGKRFVKKDSTETQT
LGGAEFDLLASDGTAVKWTDALIKANTNKNYIAGEAVTGQPIKLKSHTDGTFEIKGLAYAVDANAEGTAVTYKLKETKA
PEGYVIPDKEIEFTVSQTSYNTKPTDITVDSADATPDTIKNNKRPS
```

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

In one embodiment, the leader or signal sequence region, the transmembrane and cytoplasmic regions and the cell wall anchor motif are removed from the GBS80 sequence. An example of such a GBS80 fragment is set forth below as SEQ ID NO: 9:

```
AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN

IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKGMTLVKNQDALDKATANTDDAAFLEIPVASTINEKAVLGKAIENTF
ELQYDHTPDKADNPKPSNPPRKPEVHTGGKRFVKKDSTETQTLGGAEFDLLASDGTAVKWTDALIKANTNKNYIAGEAV
TGQPIKLKSHTDGTFEIKGLAYAVDANAEGTAVTYKLKETKAPEGYVIPDKEIEFTVSQTSYNTKPTDITVDSADATPD
TIKNNKRPS
```

A particularly immunogenic fragment of GBS80 is located towards the N-terminus of the protein, and is given herein as SEQ ID NO: 10:

```
AEVSQERPAKTTVNIYKLQADSYKSEITSNGGIENKDGEVISNYAKLGDNVKGLQGVQFKRYKVKTDISVDELKKLTTV
EAADAKVGTILEEGVSLPQKTNAQGLVVDALDSKSNVRYLYVEDLKNSPSNITKAYAVPFVLELPVANSTGTGFLSEIN
IYPKNVVTDEPKTDKDVKKLGQDDAGYTIGEEFKWFLKSTIPANLGDYEKFEITDKFADGLTYKSVGKIKIGSKTLNRD
EHYTIDEPTVDNQNTLKITFKPEKFKEIAELLKG
```

*Spb1*

The wild-type SpbI sequence from serotype III strain COH1 is SEQ ID NO: 3 herein:

26

```
MKKKMIQSLLVASLAFGMAVSPVTPIAFAAETGTITVQDTQKGATYKAYKVFDAEIDNANVSDSNKDGASYLIPQGKEA
EYKASTDFNSLFTTTTNGGRTYVTKKDTASANEIATWAKSISANTTPVSTVTESNNDGTEVINVSQYGYYYVSSTVNNG
AVIMVTSVTPNATIHEKNTDATWGDGGGKTVDQKTYSVGDTVKYTITYKNAVNYHGTEKVYQYVIKDTMPSASVVDLNE
GSYEVTITDGSGNITTLTQGSEKATGKYNLLEENNNFTITIPWAATNTPTGNTQNGANDDFFYKGINTITVTYTGVLKS
GAKPGSADLPENTNIATINPNTSNDDPGQKVTVRDGQITIKKIDGSTKASLQGAIFVLKNATGQFLNFNDTNNVEWGTE
ANATEYTTGADGIITITGLKEGTYYLVEKKAPLGYNLLDNSQKVILGDATDTTNSDNLLVNPTVENNKGTELPSTGGI
GTTIFYIIGAILVIGAGIVLVARRRLRS
```

Wild-type SpbI contains a N-terminal leader or signal sequence region which is indicated by the underlined sequence above (aa 1-29). One or more amino acids from the leader or signal sequence region of SpbI can be removed. An example of such a SpbI fragment is set forth below as SEQ ID NO: 11:

```
AETGTITVQDTQKGATYKAYKVFDAEIDNANVSDSNKDGASYLIPQGKEAEYKASTDFNSLFTTTTNGGRTYVTKKDTA
SANEIATWAKSISANTTPVSTVTESNNDGTEVINVSQYGYYYVSSTVNNGAVIMVTSVTPNATIHEKNTDATWGDGGGK
TVDQKTYSVGDTVKYTITYKNAVNYHGTEKVYQYVIKDTMPSASVVDLNEGSYEVTITDGSGNITTLTQGSEKATGKYN
LLEENNNFTITIPWAATNTPTGNTQNGANDDFFYKGINTITVTYTGVLKSGAKPGSADLPENTNIATINPNTSNDDPGQ
KVTVRDGQITIKKIDGSTKASLQGAIFVLKNATGQFLNFNDTNNVEWGTEANATEYTTGADGIITITGLKEGTYYLVEK
KAPLGYNLLDNSQKVILGDATDTTNSDNLLVNPTVENNKGTELPSTGGIGTTIFYIIGAILVIGAGIVLVARRRLRS
```

The wild-type SpbI sequence contains an amino acid motif indicative of a cell wall anchor (LPSTG). In some recombinant host cell systems, it may be preferable to remove this motif to facilitate secretion of a recombinant SpbI protein from the host cell. Thus the cell wall anchor motif and sequence C-terminal to this motif may be removed from SpbI. An example of such a fragment is set forth below as SEQ ID NO: 12:

```
MKKKMIQSLLVASLAFGMAVSPVTPIAFAAETGTITVQDTQKGATYKAYKVFDAEIDNANVSDSNKDGASYLIPQGKEA
EYKASTDFNSLFTTTTNGGRTYVTKKDTASANEIATWAKSISANTTPVSTVTESNNDGTEVINVSQYGYYYVSSTVNNG
AVIMVTSVTPNATIHEKNTDATWGDGGGKTVDQKTYSVGDTVKYTITYKNAVNYHGTEKVYQYVIKDTMPSASVVDLNE
GSYEVTITDGSGNITTLTQGSEKATGKYNLLEENNNFTITIPWAATNTPTGNTQNGANDDFFYKGINTITVTYTGVLKS
GAKPGSADLPENTNIATINPNTSNDDPGQKVTVRDGQITIKKIDGSTKASLQGAIFVLKNATGQFLNFNDTNNVEWGTE
ANATEYTTGADGIITITGLKEGTYYLVEKKAPLGYNLLDNSQKVILGDATDTTNSDNLLVNPTVENNKGTE
```

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

In one embodiment, the leader or signal sequence region, the cell wall anchor motif and sequence C-terminal to this motif are removed from SpbI. An example of such a SpbI fragment is set forth below as SEQ ID NO: 13:

```
AETGTITVQDTQKGATYKAYKVFDAEIDNANVSDSNKDGASYLIPQGKEAEYKASTDFNSLFTTTTNGGRTYVTKKDTA
SANEIATWAKSISANTTPVSTVTESNNDGTEVINVSQYGYYYVSSTVNNGAVIMVTSVTPNATIHEKNTDATWGDGGGK
TVDQKTYSVGDTVKYTITYKNAVNYHGTEKVYQYVIKDTMPSASVVDLNEGSYEVTITDGSGNITTLTQGSEKATGKYN
LLEENNNFTITIPWAATNTPTGNTQNGANDDFFYKGINTITVTYTGVLKSGAKPGSADLPENTNIATINPNTSNDDPGQ
KVTVRDGQITIKKIDGSTKASLQGAIFVLKNATGQFLNFNDTNNVEWGTEANATEYTTGADGIITITGLKEGTYYLVEK
KAPLGYNLLDNSQKVILGDATDTTNSDNLLVNPTVENNKGTE
```

An E box containing a conserved glutamic residue has also been identified in SpbI (underlined), with a conserved glutamic acid at residue 423 (bold). The E box motif may be important for the formation of oligomeric pilus-like structures, and so useful fragments of SpbI may include the conserved glutamic acid residue.

The wild-type Spb1 sequence includes an internal methionine codon (Met-162) that has an upstream 12-mer TAATGGAGCTGT sequence (SEQ ID NO: 14) that includes the core sequence (underlined) of a Shine-Dalgarno sequence. This Shine-Dalgarno sequence has been found to initiate translation of a truncated Spb1 sequence. To prevent translation initiation at this site the Shine-Dalgarno sequence can be disrupted in a Spb1-coding sequence used for expression. Although any suitable nucleotide can be mutated to prevent ribosome binding, the sequence includes a GGA glycine codon that is both part of the Shine-Dalgarno core and in-frame with the internal methionine codon. The third base in this codon can be mutated to C, G or T without changing the encoded glycine, thereby avoiding any change in Spb1 sequence.

Compositions of the invention may also include a polypeptide defined in refererence [225] by the amino acid sequence $NH_2$-W-X-L-Y-Z-$CO_2H$, wherein: X is a Spb1 sequence; L is an optional linker; and Y is a GBS80 sequence;

W is an optional N-terminal sequence; and Z is an optional C-terminal sequence. Further details of this polypeptide are given below.

These compositions may also comprise one or more of the GBS protein antigens described above. In particular, compositions of the invention may include (a) a polypeptide of amino acid sequence $NH_2$-W-X-L-Y-Z-$CO_2$H; and (b$_1$) a polypeptide comprising an amino acid sequence of SEQ ID NO 1 as described above, and/or (b$_2$) a polypeptide comprising (i) an amino acid sequence that has sequence identity to SEQ ID NO 1 as described above and/or (ii) a fragment of SEQ ID NO 1 as described above,

*Polypeptide $NH_2$-W-X-L-Y-Z-$CO_2$H*

Typically, the polypeptide comprises an amino acid sequence X-L-Y, wherein: X is a Spb1 sequence; L is an optional linker; and Y is a GBS80 sequence.

X: Spb1 sequence

The X moiety is a Spb1 sequence. This Spb1 sequence will, when administered to a subject, elicit an antibody response comprising antibodies that bind to wild-type Spb1 protein *e.g.* to the *S.agalactiae* protein having amino acid sequence SEQ ID NO: 3 (the full-length wild-type sequence from strain COH1).

The Spb1 sequence may comprise an amino acid sequence having at least *a*% identity to SEQ ID NO: 13. The value of *a* may be selected from 50, 60, 70, 80, 85, 90, 95, 96, 97, 98, 99 or more. The Spb1 sequence may comprise SEQ ID NO: 13.

The Spb1 sequence may comprise a fragment of SEQ ID NO: 3 and/or of SEQ ID NO:13. The fragment will usually include at least *b* amino acids of SEQ ID NO: 3/13, wherein *b* is selected from 5, 6, 7, 8, 9, 10, 11, 12, 13, 14, 15, 16, 17, 18, 19, 20, 22, 24, 26, 28, 30, 40, 50, 60, 70, 80, 90, 100, 110, 120, 130, 140, 150, 175, 200 or more. The fragment will usually include at least one T-cell or, preferably, a B-cell epitope of SEQ ID NO: 3/13. T- and B- cell epitopes can be identified by the methods described above. SEQ ID NO: 13 is itself a fragment of SEQ ID NO: 3, as explained above.

The Spb1 sequence may comprise an amino acid sequence that has both at least *a*% identity to SEQ ID NO: 13 and comprises a fragment of SEQ ID NO: 13, as defined above.

The X moiety will usually be at least c amino acids long, where c is selected from 50, 60, 70, 80, 90, 100, 120, 140, 160, 180, 200, 220, 240, 260, 280, 300, 320, 340, 360, 380, 400 or more.

The X moiety will usually be no longer than *d* amino acids long, where *d* is selected from 500, 480, 460, 440, 420, 400, 380, 360, 340, 320, 300, 280, 260, 240, 220, 200 or less.

The X moiety will usually be between 300-500 amino acids long *e.g.* 350-480, 400-460, 430-450.

The wild-type SpbI sequence from serotype III strain COH1 is SEQ ID NO: 3 above. The specific derivatives thereof described in section *"SpbI"* above are applicable to the Spb1 sequence of this embodiment of the invention.

Y: GBS80 sequence

The Y moiety is a GBS80 sequence. This GBS80 sequence will, when administered to a subject, elicit an antibody response comprising antibodies that bind to wild-type GBS80 protein *e.g.* to the *S.agalactiae* protein having amino acid sequence SEQ ID NO: 2 (the full-length wild-type sequence from strain 2603V/R).

The GBS80 sequence may comprise an amino acid sequence having at least *e*% identity to SEQ ID NO: 9. The value of *e* may be selected from 50, 60, 70, 80, 85, 90, 95, 96, 97, 98, 99 or more. The GBS80 sequence may comprise SEQ ID NO: 9.

The GBS80 sequence may comprise a fragment of SEQ ID NO: 2 or of SEQ ID NO: 9. The fragment will usually include at least *f* amino acids of SEQ ID NO: 2/9, wherein *f* is selected from 5, 6, 7, 8, 9, 10, 11, 12, 13, 14, 15, 16, 17, 18, 19, 20, 22, 24, 26, 28, 30, 40, 50, 60, 70, 80, 90, 100, 110, 120, 130, 140, 150, 175, 200 or more. The fragment will usually include at least one T-cell or, preferably, B-cell epitope of SEQ ID NO: 2/9. SEQ ID NO: 9 is itself a fragment of SEQ ID NO: 2, as explained above.

The GBS80 sequence may comprise an amino acid sequence that has both at least *e*% identity to SEQ ID NO: 9 and comprises a fragment of SEQ ID NO: 9, as defined above.

The Y moiety will usually be at least g amino acids long, where g is selected from 50, 60, 70, 80, 90, 100, 120, 140, 160, 180, 200, 220, 240, 260, 280, 300, 320, 340, 360, 380, 400, 420, 440, 460, 480, 500, 520, 540, 560, 580, 600 or more.

The Y moiety will usually be no longer than *h* amino acids long, where *h* is selected from 600, 580, 560, 540, 520, 500, 480, 460, 440, 420, 400, 380, 360, 340, 320, 300, 280, 260, 240, 220, 200 or less.

The Y moiety will usually be between 350-550 amino acids long *e.g.* 400-520, 450-500, 470-490.

The wild-type GBS80 sequence from serotype V isolated strain 2603 V/R is SEQ ID NO: 2 above. The specific derivatives thereof described in section "*GBS80*" above are applicable to the GBS80 sequence of this embodiment of the invention.

L: linker

The polypeptide optionally includes a L moiety to link the X and Y moieties. The L moiety is typically a short amino acid sequence *e.g.* in the range of 2-40 amino acids *e.g.* consisting of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 amino acids.

Linkers will usually contain at least one glycine residue, thereby facilitating structural flexibility. The linker may contain, for example, 1, 2, 3, 4, 5, 6, 7, 8, 9, 10 or more glycine residues. The glycines may be arranged to include at least two consecutive glycines in a Gly-Gly dipeptide sequence, or a longer oligo-Gly sequence *i.e.* $Gly_n$ where n = 2, 3, 4, 5, 6, 7, 8, 9, 10 or more, *e.g.* SEQ ID NO: 15: GGGG

Linkers may be encoded by codons found in the recognition sequences of restriction enzymes. For example, a 6-mer sequence that is the target of a particular restriction enzyme can code for a dipeptide. Thus the recognition sequence for *Bam*HI (GGATCC) encodes Gly-Ser, and so a linker may include a Gly-Ser dipeptide sequence. Such sequences facilitate cloning and manipulation.

Useful linker sequences include SEQ ID NO 15 above and SEQ ID NOs 16, 17 and 18 below:

GGGGSGGGGSGGGG (SEQ ID NO:16)
GGGGSGGGGSGGGGSEL (SEQ ID NO:17)
GSGGGG (SEQ ID NO:18)

However, preferred linkers do not include a sequence that shares 10 or more contiguous amino acids in common with a human polypeptide sequence. For instance, one glycine-rich linker sequence that can be used with the invention is the 14mer SEQ ID NO: 16. However, this 14mer is also found in a human RNA binding protein (gi: 8051631) and so it is preferably avoided within the L moiety.

W: N-terminal sequence

The X moiety may be at the N-terminus of the polypeptide, but it is also possible to have amino acids upstream of X. These optional amino acids form a W moiety.

The W moiety is typically a short amino acid sequence *e.g.* in the range of 2-40 amino acids *e.g.* consisting of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 amino acids.

Examples of W moieties are leader sequences to direct protein trafficking, or comprise short peptide sequences which facilitate cloning or purification (*e.g.* histidine tags *i.e.* $His_n$ where $n$ = 3, 4, 5, 6, 7, 8, 9, 10 or more). Other suitable N-terminal amino acid sequences will be apparent to those skilled in the art.

In a nascent polypeptide the W moiety can provide the polypeptide's N-terminal methionine (formyl-methionine, fMet, in bacteria). One or more amino acids may be cleaved from the N-terminus of a nascent W moiety, however, such that the W moiety in a polypeptide of the invention does not necessarily include a N-terminal methionine.

Useful W moieties include SEQ ID NO 19:
MAS

Z: C-terminal sequence

The Y moiety may be at the C-terminus of the polypeptide, but it is also possible to have amino acids downstream of Y. These optional amino acids form a Z moiety.

The Z moiety is typically a short amino acid sequence *e.g.* in the range of 2-40 amino acids *e.g.* consisting of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 amino acids.

Examples of Z moieties include sequences to direct protein trafficking, short peptide sequences which facilitate cloning or purification (*e.g.* comprising histidine tags *i.e.* $His_n$ where $n$ = 3, 4, 5, 6, 7, 8, 9, 10 or more), or sequences which enhance protein stability. Other suitable C-terminal amino acid sequences will be apparent to those skilled in the art, such as a glutathione-S-transferase, thioredoxin, 14kDa fragment of *S.aureus* protein A, a biotinylated peptide, a maltose-binding protein, an enterokinase flag, *etc.* One useful Z moiety comprises SEQ ID NO 20:

HHHHHH

Useful combinations

Of the various X, Y and L moieties, useful combinations include, but are not limited to:

| SEQ ID | W | X | L | Y | Z |
|---|---|---|---|---|---|
| 21 | 19 | 13 | 17 | 9 | - |
| 22 | 19 | 13 | 17 | 9 | 20 |
| 23 | 19 | 13 | 18 | 9 | - |

```
MASAETGTITVQDTQKGATYKAYKVFDAEIDNANVSDSNKDGASYLIPQGKEAEYKASTDFNSLFTTTTNGGRTYVTKK
DTASANEIATWAKSISANTTPVSTVTESNNDGTEVINVSQYGYYYVSSTVNNGAVIMVTSVTPNATIHEKNTDATWGDG
GGKTVDQKTYSVGDTVKYTITYKNAVNYHGTEKVYQYVIKDTMPSASVVDLNEGSYEVTITDGSGNITTLTQGSEKATG
KYNLLEENNNFTITIPWAATNTPTGNTQNGANDDFFYKGINTITVTYTGVLKSGAKPGSADLPENTNIATINPNTSNDD
PGQKVTVRDGQITIKKIDGSTKASLQGAIFVLKNATGQFLNFNDTNNVEWGTEANATEYTTGADGIITITGLKEGTYYL
VEKKAPLGYNLLDNSQKVILGDATDTTNSDNLLVNPTVENNKGTEGGGGSGGGGSGGGGSELAEVSQERPAKTTVNIY
KLQADSYKSEITSNGGIENKDGEVISNYAKLGDNVKGLQGVQFKRYKVKTDISVDELKKLTTVEAADAKVGTILEEGVS
LPQKTNAQGLVVDALDSKSNVRYLYVEDLKNSPSNITKAYAVPFVLELPVANSTGTGFLSEINIYPKNVVTDEPKTDKD
VKKLGQDDAGYTIGEEFKWFLKSTIPANLGDYEKFEITDKFADGLTYKSVGKIKIGSKTLNRDEHYTIDEPTVDNQNTL
KITFKPEKFKEIAELLKGMTLVKNQDALDKATANTDDAAFLEIPVASTINEKAVLGKAIENTFELQYDHTPDKADNPKP
SNPPRKPEVHTGGKRFVKKDSTETQTLGGAEFDLLASDGTAVKWTDALIKANTNKNYIAGEAVTGQPIKLKSHTDGTFE
IKGLAYAVDANAEGTAVTYKLKETKAPEGYVIPDKEIEFTVSQTSYNTKPTDITVDSADATPDTIKNNKRPS (SEQ
ID NO:21)
```

```
MASAETGTITVQDTQKGATYKAYKVFDAEIDNANVSDSNKDGASYLIPQGKEAEYKASTDFNSLFTTTTNGGRTYVTKK
DTASANEIATWAKSISANTTPVSTVTESNNDGTEVINVSQYGYYYVSSTVNNGAVIMVTSVTPNATIHEKNTDATWGDG
GGKTVDQKTYSVGDTVKYTITYKNAVNYHGTEKVYQYVIKDTMPSASVVDLNEGSYEVTITDGSGNITTLTQGSEKATG
KYNLLEENNNFTITIPWAATNTPTGNTQNGANDDFFYKGINTITVTYTGVLKSGAKPGSADLPENTNIATINPNTSNDD
PGQKVTVRDGQITIKKIDGSTKASLQGAIFVLKNATGQFLNFNDTNNVEWGTEANATEYTTGADGIITITGLKEGTYYL
VEKKAPLGYNLLDNSQKVILGDATDTTNSDNLLVNPTVENNKGTEGGGGSGGGGSGGGGSELAEVSQERPAKTTVNIY
KLQADSYKSEITSNGGIENKDGEVISNYAKLGDNVKGLQGVQFKRYKVKTDISVDELKKLTTVEAADAKVGTILEEGVS
LPQKTNAQGLVVDALDSKSNVRYLYVEDLKNSPSNITKAYAVPFVLELPVANSTGTGFLSEINIYPKNVVTDEPKTDKD
VKKLGQDDAGYTIGEEFKWFLKSTIPANLGDYEKFEITDKFADGLTYKSVGKIKIGSKTLNRDEHYTIDEPTVDNQNTL
KITFKPEKFKEIAELLKGMTLVKNQDALDKATANTDDAAFLEIPVASTINEKAVLGKAIENTFELQYDHTPDKADNPKP
SNPPRKPEVHTGGKRFVKKDSTETQTLGGAEFDLLASDGTAVKWTDALIKANTNKNYIAGEAVTGQPIKLKSHTDGTFE
IKGLAYAVDANAEGTAVTYKLKETKAPEGYVIPDKEIEFTVSQTSYNTKPTDITVDSADATPDTIKNNKRPSHHHHHH
```

(SEQ ID NO:22)

```
MASAETGTITVQDTQKGATYKAYKVFDAEIDNANVSDSNKDGASYLIPQGKEAEYKASTDFNSLFTTTTNGGRTYVTKK
DTASANEIATWAKSISANTTPVSTVTESNNDGTEVINVSQYGYYYVSSTVNNGAVIMVTSVTPNATIHEKNTDATWGDG
GGKTVDQKTYSVGDTVKYTITYKNAVNYHGTEKVYQYVIKDTMPSASVVDLNEGSYEVTITDGSGNITTLTQGSEKATG
KYNLLEENNNFTITIPWAATNTPTGNTQNGANDDFFYKGINTITVTYTGVLKSGAKPGSADLPENTNIATINPNTSNDD
PGQKVTVRDGQITIKKIDGSTKASLQGAIFVLKNATGQFLNFNDTNNVEWGTEANATEYTTGADGIITITGLKEGTYYL
VEKKAPLGYNLLDNSQKVILGDATDTTNSDNLLVNPTVENNKGTEGSGGGGELAEVSQERPAKTTVNIYKLQADSYKS
EITSNGGIENKDGEVISNYAKLGDNVKGLQGVQFKRYKVKTDISVDELKKLTTVEAADAKVGTILEEGVSLPQKTNAQG
LVVDALDSKSNVRYLYVEDLKNSPSNITKAYAVPFVLELPVANSTGTGFLSEINIYPKNVVTDEPKTDKDVKKLGQDDA
GYTIGEEFKWFLKSTIPANLGDYEKFEITDKFADGLTYKSVGKIKIGSKTLNRDEHYTIDEPTVDNQNTLKITFKPEKF
KEIAELLKGMTLVKNQDALDKATANTDDAAFLEIPVASTINEKAVLGKAIENTFELQYDHTPDKADNPKPSNPPRKPEV
HTGGKRFVKKDSTETQTLGGAEFDLLASDGTAVKWTDALIKANTNKNYIAGEAVTGQPIKLKSHTDGTFEIKGLAYAVD
ANAEGTAVTYKLKETKAPEGYVIPDKEIEFTVSQTSYNTKPTDITVDSADATPDTIKNNKRPS (SEQ ID NO:23)
```

The polypeptide may comprise an amino acid sequence having at least *i*% sequence identity to SEQ ID NO: 21. The value of *i* may be selected from 50, 60, 70, 80, 85, 90, 95, 96, 97, 98, 99 or more. The polypeptide may comprise SEQ ID NO: 21.

The polypeptide may comprise an amino acid sequence having at least *i*% sequence identity to SEQ ID NO: 23. The value of *i* may be selected from 50, 60, 70, 80, 85, 90, 95, 96, 97, 98, 99 or more. The polypeptide may comprise SEQ ID NO: 23.

A polypeptide used with the invention may comprise an amino acid sequence that:

(a) is identical (*i.e.* 100% identical) to SEQ ID NO: 21 or 23;

(b) shares sequence identity SEQ ID NO: 21 or 23;

(c) has 1, 2, 3, 4, 5, 6, 7, 8, 9 or 10 (or more) single amino acid alterations (deletions, insertions, substitutions), which may be at separate locations or may be contiguous, as compared to the sequences of (a) or (b); and

(d) when aligned SEQ ID 21 or 23 using a pairwise alignment algorithm, each moving window of *x* amino acids from N-terminus to C-terminus (such that for an alignment that extends to *p* amino acids, where *p>x*, there are *p-x+1* such windows) has at least *x·y* identical aligned amino acids, where: *x* is selected from 20, 25, 30, 35, 40, 45, 50, 60, 70, 80, 90, 100, 150, 200; *y* is selected from 0.50, 0.60, 0.70, 0.75, 0.80, 0.85, 0.90, 0.91, 0.92, 0.93, 0.94, 0.95, 0.96, 0.97, 0.98, 0.99; and if *x·y* is not an integer then it is rounded up to the nearest integer. The preferred pairwise alignment algorithm is the Needleman-Wunsch global alignment algorithm [226], using default parameters (*e.g.* with Gap opening penalty = 10.0, and with Gap extension penalty = 0.5, using the EBLOSUM62 scoring matrix). This algorithm is conveniently implemented in the *needle* tool in the EMBOSS package [227].

Within group (c), deletions or substitutions may be at the N-terminus and/or C-terminus, or may be between the two termini. Thus a truncation is an example of a deletion. Truncations may involve deletion of up to 40 (or more) amino acids at the N-terminus and/or C-terminus.

The Spb1 and GBS80 sequences in the polypeptides may be derived from one or more GBS strains. For instance, SEQ ID NOs: 21 and 23 include Spb1 sequence from strain COH1 and GBS80 sequence from strain 2603V/R.

Polypeptides

The polypeptides, or individual moieties, may, compared to SEQ ID NOs: 2, 3, 9, 10, 13, 21 or 23, include one or more (*e.g.* 1, 2, 3, 4, 5, 6, 7, 8, 9, 10, *etc.*) conservative amino acid replacements *i.e.* replacements of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may have one or more (*e.g.* 1, 2, 3, 4, 5, 6, 7, 8, 9, 10, *etc.*) single amino acid deletions relative to a reference sequence. The polypeptides may also include one or more (*e.g.* 1, 2, 3, 4, 5, 6, 7, 8, 9, 10, *etc.*) insertions (*e.g.* each of 1, 2, 3, 4 or 5 amino acids) relative to a reference sequence.

The polypeptides can be prepared in many ways, as described above. The polypeptides can also take various forms (*e.g.* native, fusions, glycosylated, non-glycosylated, lipidated, non-lipidated, phosphorylated, non-phosphorylated, myristoylated, non-myristoylated, monomeric, multimeric, particulate, denatured, *etc.*), as described above. The polypeptides are preferably provided in purified or substantially purified form, as described above.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.*), as well as other modifications known in the art. Polypeptides can occur as single chains or associated chains. The polypeptides can be naturally or non-naturally glycosylated (*i.e.* the polypeptide has a glycosylation pattern that differs from the glycosylation pattern found in the corresponding naturally occurring polypeptide).

The polypeptides may be at least 40 amino acids long (*e.g.* at least 40, 50, 60, 70, 80, 90, 100, 120, 140, 160, 180, 200, 220, 240, 260, 280, 300, 350, 400, 450, 500 or more). The polypeptides may be shorter than 1100 amino acids.

*Pre-immunisation*

In a second aspect, the invention provides a method for immunising a patient against infection by GBS comprising the step of administering to the patient a conjugate that is a capsular saccharide from GBS conjugated to a diphtheria toxoid or derivative thereof, wherein the patient has been pre-immunised with a diphtheria toxoid or derivative thereof. The GBS conjugates are those of the immunogenic composition of the first aspect of the invention, as described above. In other words, immunogenic compositions of the first aspect of the invention is used in the second aspect of the invention. The capsular saccharide conjugated to the diphtheria toxoid or derivative thereof in the composition is from GBS serotypes Ia, Ib and III. In this aspect all of the capsular saccharides from GBS in the composition are conjugated to a diphtheria toxoid or derivative thereof. Where the carrier or pre-immunisation antigen is a derivative of a diphtheria toxoid then that derivative preferably remains immunologically cross-reactive with Dt, and is preferably CRM197. The inventors have found that conjugates that are capsular saccharides from GBS conjugated to a diphtheria toxoid or derivative thereof do not seem to suffer from carrier-induced epitopic suppression (or "carrier suppression", as it is generally known), particularly suppression arising from carrier priming. As discussed below, "carrier suppression" is the phenomenon whereby pre-immunisation of an animal with a carrier protein prevents it from later eliciting an immune response against a new antigenic epitope that is presented on that carrier [228]. In contrast to this known phenomenon, the inventors have found that the immune response to GBS capsular saccharide-diphtheria toxoid or derivative thereof conjugates may in fact be improved by pre-immunisation with the diphtheria toxoid or derivative thereof.

As reported in reference 229, where several vaccine antigens contain the same protein component (being used as an immunogen and/or as a carrier protein in a conjugate) then there is the potential for interference between those antigens. In reference 229, the immune response against an antigen that was conjugated to a tetanus toxoid (Tt) carrier was suppressed by pre-existing immunity against Tt.

Reference 230 reports how a combination of D-T-P vaccines with a Hib conjugate vaccine was adversely affected where the carrier for the Hib conjugate was the same as the tetanus antigen from the D-T-P vaccine. The authors concludes that this "carrier suppression" phenomenon, arising from interference by a common protein carrier, should be taken into account when introducing vaccines that include multiple conjugates.

In contrast to references 229 and 230, reference 231 reported that priming with tetanus toxoid had no negative impact on the immune response against a subsequently-administered Hib-Tt conjugate, but suppression was seen in patients with maternally acquired anti-Tt antibodies. In reference 232, however, an "epitopic suppression" effect was reported for a Tt-based peptide conjugate in patients having existing anti-Tt antibodies resulting from tetanus vaccination.

In reference 233, it was suggested that a conjugate having CRM197 (a detoxified mutant of diphtheria toxin) as the carrier may be ineffective in children that had not previously received diphtheria toxin as part of a vaccine (*e.g.* as part of a D-T-P or D-T vaccine). This work was further developed in reference 234, where a carrier priming effect by D-T immunisation was seen to persist for subsequent immunisation with Hib conjugates.

In reference 235, the authors found that pre-immunisation with a diphtheria or tetanus toxoid carrier protein reduced the increase in anti-Hib antibody levels after a subsequent immunisation with the Hib capsular saccharide conjugated to those carriers, with IgG1 and IgG2 being equally affected. Responses to the carrier portions of the conjugates were also suppressed. Furthermore, a more general non-epitope-specific suppression was seen, as pre-immunisation with one conjugate was seen to affect immune responses against both the carrier and saccharide portions of a second conjugate that was administered four weeks later.

The use of different carrier proteins in a single multivalent pneumococcal conjugate vaccine is reported in reference 236, with multiple carriers being used in order to avoid carrier suppression. The authors predict that there is a maximum load of a carrier protein that can be tolerated in a multivalent conjugate vaccine without giving rise to negative interference. In reference 237 it was reported that pneumococcal conjugate vaccines including mixed carrier proteins elicited, in parallel to the anti-pneumococcus response, unintentional booster responses to the carriers.

In reference 238, an investigation of whether diphtheria and tetanus boosters could be administered with monovalent meningococcal serogroup C conjugates, it was found that titres against the meningococcal conjugate were reduced where the carrier was tetanus toxoid carrier and the patient had received prior immunisation with a tetanus-containing vaccine.

In addition to the problem of priming with a carrier having a negative impact on immune responses against saccharide conjugates, the reverse can also occur *i.e.* immunisation with a conjugate can have a negative impact on immune responses against the carrier [239].

This second aspect of the invention therefore provides a method for immunising a patient against infection by GBS comprising the step of administering to the patient a conjugate that is a capsular saccharide from GBS conjugated to a diphtheria toxoid or derivative thereof, wherein the patient has been pre-immunised with a diphtheria toxoid or derivative thereof. This aspect also provides a conjugate that is a capsular saccharide from GBS conjugated to a diphtheria toxoid or derivative thereof for use in immunising a patient against infection by GBS, wherein the patient has been pre-immunised with a diphtheria toxoid or derivative thereof. This aspect further provides the use of a conjugate that is a capsular saccharide from GBS conjugated to a diphtheria toxoid or derivative thereof in the manufacture of a medicament for immunising a patient against infection by GBS, wherein the patient has been pre-immunised with a diphtheria toxoid or derivative thereof.

*The pre-immunised patient*

This patient to be immunised has been pre-immunised with a diphtheria toxoid or derivative thereof. The diphtheria toxoid or derivative thereof may have been administered as the carrier in a conjugate of a capsular saccharide of an organism other than GBS and a diphtheria toxoid or derivative thereof. Typical pre-immunisation will have included: a diphtheria toxoid antigen; a Hib capsular saccharide conjugate using a diphtheria toxoid or CRM197 carrier; and/or a pneumococcal capsular saccharide conjugate using a diphtheria toxoid or CRM197 carrier.

The patient will have received at least one (*e.g.* 1, 2, 3 or more) dose of the pre-immunisation antigen(s), and that dose (or the earliest of multiple doses) will have been administered to the patient at least six (*e.g.* 6, 9, 12, 15, 18, 21, 24, 36, 48, 60, 120, 180, 240, 300 or more) months before the immunisation with the GBS conjugates according to this aspect of invention. In a preferred group of patients, the pre-immunisation took place within 3 years of birth *e.g.* within 2 years of birth, within 1 year of birth, within 6 months of birth, or even within 3 months, 2 months or 1 month of birth. Suitable patients to be immunised according to this aspect of the invention are described above in the section *Methods of treatment.*

Where the pre-immunisation antigen is a diphtheria toxoid then the patient will typically have received the toxoid as the 'D' antigen in a D-T-P or a D-T pre-immunisation. Such immunisations are typically given to newborn children at ages 2, 3, and 4 months. Where the immunisation includes a pertussis vaccine, that vaccine may be a whole cell or cellular pertussis vaccine ('Pw'), but is preferably an acellular pertussis vaccine ('Pa'). Pre-immunisation Pa vaccines will generally include one, two or three of the following well-known and well-characterised *B.pertussis* antigens: (1) pertussis toxoid ('PT'), detoxified either by chemical means or by site-directed mutagenesis *e.g.* the '9K/129G' mutant [240]; (2) filamentous haemagglutinin ('FHA'); (3) pertactin (also known as '69 kiloDalton outer membrane protein'). Acellular pertussis vaccines may also include agglutinogen 2 and/or agglutinogen 3. The 'T' antigen in a D-T-P pre-immunisation is typically a tetanus toxoid.

Where the pre-immunisation antigen is a diphtheria toxoid then the patient may also or alternatively have received the toxoid as the carrier protein of a protein-saccharide conjugate. Such conjugates include the 'PRP-D' Hib conjugate [see Table 14-7 of ref. [241] *e.g.* the ProHIBIT™ product.

Where the pre-immunisation antigen is CRM197 then the patient will typically have been pre-immunised with a Hib conjugate and/or a multivalent pneumococcal conjugate. Such immunisations are typically given to newborn children at ages 2, 3, and 4 months. Hib conjugates that use a CRM197 carrier include the 'HbOC' conjugates [Table 14-7 of ref. 241] *e.g.* the HibTITER™ product. Pneumococcal conjugates that use a CRM197 carrier include the 7-valent PCV7 mixtures *e.g.* the PrevNar™ vaccine [242]. The patient may also have been pre-immunised with a serogroup C meningococcal ('MenC') conjugate. MenC conjugates that use CRM197 carrier include Meninvact™/Menjugate™ [243] and Meningitec™.

Where pre-immunisation was with a conjugated antigen then the patient will almost inevitably have also received a small amount of free diphtheria toxoid (or derivative) as a result of low-level contamination of the conjugate (*e.g.* caused by hydrolysis of the conjugate during storage), but this small amount will not typically have been adequate to provide a significant immune response.

Diphtheria toxoid is a well known and well characterised protein [*e.g.* see chapter 13 of ref. 241] that can be obtained by treating the ADP-ribosylating exotoxin of *Coryuebacterium diphtheriae* with an inactivating chemical, such as formalin or formaldehyde. CRM197 is also well known and well characterised [244-247], and has been widely used as a carrier in conjugated saccharide vaccines. CRM197 and Dt share many carrier epitopes.

The result of the pre-immunisation is that the patient's immune system has been exposed to the pre-immunisation antigens. For pre-immunisation with diphtheria toxoid (Dt), this generally means that the patient will have raised an anti-Dt antibody response (typically to give an anti-Dt titer >0.01 IU/ml) and will possess memory B and/or T lymphocytes specific for Dt *i.e.* pre-immunisation with Dt is typically adequate to elicit an anamnestic anti-Dt immune response in the patient. For pre-immunisation where Dt (or derivative) is a carrier for a saccharide within a conjugate then the pre-immunisation will have raised an anti-saccharide response and the patient will possess memory B and/or T lymphocytes specific for the saccharide *i.e.* the pre-immunisation is typically adequate to elicit an anamnestic anti-saccharide immune response in the patient. The pre-immunisation was preferably adequate to elicit protective immunity in the patient *e.g.* against diphtheria disease.

Thus the patients to be immunised according to this aspect of the invention are distinct from patients in general, as they are members of a subset of the general population whose immune systems have already mounted an immune response to the pre-immunisation antigens, such that immunisation according to this aspect with a GBS conjugate that includes a diphtheria toxoid (or derivative thereof) carrier elicits a different immune response in the subset than in patients who have not previously mounted an immune response to the pre-immunisation antigens. Patients who have been pre-immunised with Dt (or derivative) as the carrier of a conjugate (particularly of a Hib conjugate) are preferred. Particularly preferred patients have been pre-immunised with Dt (or derivative) as the carrier of a conjugate and also with Dt as an unconjugated immunogen.

As well as having been pre-immunised with a diphtheria toxoid (or derivative), in conjugated or non-conjugated form, the patient may have been pre-immunised with other antigens. Such antigens include, but are not limited to: pertussis antigen(s) - see above; tetanus toxoid - see above; *Haemophilus influenzae* type B - see above; hepatitis B surface antigen (HBsAg); poliovirus, such as an inactivated poliovirus vaccine (IPV); *Streptococcus pneumoniae* - see above; influenza virus; BCG; hepatitis A virus antigens; measles virus; mumps virus; rubella virus; varicella virus; *etc.*

The patient may or may not have been pre-immunised with one or more GBS conjugate(s). In some preferred embodiments, at the time when a patient first receives a GBS conjugate, they have already been pre-immunised with Dt (or derivative). In other embodiments, a GBS conjugate is administered to a patient who has already been pre-immunised with both (i) Dt or a derivative and (ii) a GBS conjugate.

*Tetanus toxoid carriers*

Also described herein is a method for immunising a patient against infection by GBS comprising the step of administering to the patient a conjugate that is a capsular saccharide from GBS conjugated to a tetanus toxoid or derivative thereof, wherein the patient has been pre-immunised with a tetanus toxoid or derivative thereof. This method uses a conjugate that is a capsular saccharide from GBS conjugated to a tetanus toxoid or derivative thereof for use in immunising a patient against infection by GBS, wherein the patient has been pre-immunised with a tetanus toxoid or derivative thereof. This method further provides the use of a conjugate that is a capsular saccharide from GBS conjugated to a tetanus toxoid or derivative thereof in the manufacture of a medicaument for immunising a patient against infection by GBS, wherein the patient has been pre-immunised with a tetanus toxoid or derivative thereof. Conjugates that are capsular saccharides from GBS conjugated to a tetanus toxoid or derivative thereof may not suffer from carrier suppression, particularly suppression arising from carrier priming. The immune response to GBS capsular saccharide-tetanus toxoid or derivative thereof conjugates may in fact be improved by pre-immunisation with the tetanus toxoid or derivative thereof.

Tetanus toxoid is a well known protein [*e.g.* see chapter 27 of ref. 241], and can be obtained by inactivating the ADP-ribosylating exotoxin of *Clostridium tetani.* Patients will typically have received tetanus toxoid as the 'T' antigen in a D-T-P or a D-T pre-immunisation, or as the carrier protein in a conjugate. Such conjugates include the 'PRP-T' Hib conjugate [see Table 14-7 of ref. 241] *e.g.* the ActHIB™, OmniHIB™ and HIBERIX™ products.

***General***

The term "comprising" encompasses "including" as well as "consisting" *e.g.* a composition "comprising" X may consist exclusively of X or may include something additional *e.g.* X + Y.

The term "about" in relation to a numerical value $x$ means, $x \pm 10\%$.

The word "substantially" does not exclude "completely" *e.g.* a composition which is "substantially free" from Y may be completely free from Y. Where necessary, the word "substantially" may be omitted from the definition of the invention.

It will be appreciated that sugar rings can exist in open and closed form and that, whilst closed forms are shown in structural formulae herein, open forms are also encompassed by the invention. Similarly, it will be appreciated that sugars can exist in pyranose and furanose forms and that, whilst pyranose forms are shown in structural formulae herein, furanose forms are also encompassed. Different anomeric forms of sugars are also encompassed.

Unless specifically stated, a process comprising a step of mixing two or more components does not require any specific order of mixing. Thus components can be mixed in any order. Where there are three components then two components can be combined with each other, and then the combination may be combined with the third component, *etc.*

Antibodies will generally be specific for their target. Thus they will have a higher affinity for the target than for an irrelevant control protein, such as bovine serum albumin.

Unless otherwise stated, identity between polypeptide sequences is preferably determined by the Smith-Waterman homology search algorithm as implemented in the MPSRCH program (Oxford Molecular), using an affine gap search with parameters *gap open penalty=12* and *gap extension penalty=1*.

**BRIEF DESCRIPTION OF DRAWINGS**

Figure 1 shows the difference between the repeating structures in GBS serotypes Ia and III.

Figure 2 shows the repeating structures of capsular saccharides in GBS serotypes Ia, Ib, II, III & V.

Figure 3 shows the repeating structure of the desialylated form of the capsular saccharide from GBS serotype V.

Figure 4 shows the effect of priming with CRM197 prior to administration of a conjugate of GBS serotype III capsular saccharide and CRM197, with and without adjuvant.

## MODES FOR CARRYING OUT THE INVENTION

### *Conjugate production*

Purified capsular saccharides from Streptococcus agalactiae serotypes Ia, Ib and III were conjugated to a carrier protein by periodate oxidation followed by reductive amination (ref. 2). Purified, desialylated capsular saccharide from *Streptococcus agalactiae* serotype V was conjugated to a carrier protein by periodate oxidation followed by reductive amination (ref. 14). The carrier protein in most cases was CRM197. Tetanus toxoid was used as a carrier protein where specifically indicated.

### *Mouse study (1).*

In this study, the effect of the adjuvant on the efficacy and immunogenicity of GBS serotype Ia, Ib and III conjugates, either as monovalent or combination vaccines, was evaluated in an active maternal-neonatal challenge mouse model.

The maternal-neonatal challenge mouse model, adapted from the reference 248, is used to to assess the efficacy in neonates of specific antibodies acquired transplacentally from actively vaccinated dams. Specifically, female CD-1 mice, aged between 5-6 weeks from Charles River Laboratories (Calco, Italy), are vaccinated by intra-peritoneal injection with two or three immunizations on days 1, 21 and eventually 35, with or without adjuvant. After the last immunization, mice are bred and kept until delivery. An inoculum of a GBS strain (0.05 mL of Todd-Hewitt broth), lethal for 90% of non-immunized pups (100-1000 fold LD50), is used to challenge the neonatal mice. Challenge is by the intra-peritoneal route within 48 hours of birth. The number of surviving pups at 72 hours is recorded and survival rates are compared in all treated groups using the Fisher's exact test. In a control group, dams receive PBS by the same route and using the same dosing schedule. Two weeks after the last immunization, blood samples are collected for immunogenicity assessment using two *in vitro* assays (the ELISA and Opsonophagocytosis assays described below).

The ELISA assay is performed to determine the titer of GBS-specific antibodies produced following immunization. ELISA is also used to quantify the total IgG against each capsular saccharde antigen. Serum from each individual mouse is analyzed and the Geometric Mean Titer (GMT) calculated for each group. Antibody titers for capsular saccharde types Ia, Ib and III are expressed as Mouse ELISA Unit (MEU) and are calculated based on the Reference Line Assay Method.

The Opsonophagocytosis assay (OPA) is performed to evaluate the titer of vaccine-induced antibodies capable of complement-mediated GBS killing (using the approach described in reference 249). The assay is performed by combining the following components: bacteria, phagocytic cells (PMNs extracted from human blood or the differentiated HL-60 cell line), complement and immune sera. Aliquots of the reaction mix are plated before and after a 1h incubation at 37 °C to determine the remaining colony forming units (CFU). The amount of opsonophagocytic killing (log kill) is determined by subtracting the log of the surviving colony number from the log of the CFU number present at the initial time-point. A pre-immune serum, and heat-inactivated complement without PMNs, is used as negative control. Bactericidal titer is expressed as reciprocal serum dilution leading to reduction in 50% of bacteria.

In this study, female CD1 mice were immunized with two doses (1 $\mu$g each) of the three different conjugates in the presence of adjuvant (aluminum hydroxide or MF59) on days 0 and 21. The neonates were challenged with type specific strains as shown below in Table 1.

*Table 1: Determination of the protection level and antibody titers obtained with GBS serotype Ia, Ib and III/CRM197 conjugates in the presence of aluminum hydroxide or MF59 in the active maternal-neonatal challenge mouse model.*

| Antigen | Adjuvant | GMT titers | Challenge Strain (type) | Alive/Treated | Survival (%) |
|---|---|---|---|---|---|
| CRM-Ia | Al-H | 281 | A909 (Ia) | 77/80 | 96 |
| CRM-Ia | MF59 | 1253 | A909 (Ia) | 66/75 | 88 |
| PBS | - | - | A909 (Ia) | 1/65 | 1 |
| CRM-Ib | Al-H | 1097 | 7357B (Ib) | 65/70 | 93 |
| CRM-Ib | MF59 | 7843 | 7357B (Ib) | 47/60 | 78 |

(continued)

| Antigen | Adjuvant | GMT titers | Challenge Strain (type) | Alive/Treated | Survival (%) |
|---|---|---|---|---|---|
| PBS | - | - | 7357B (Ib) | 6/70 | 8 |
| CRM-III | Al-H | 234 | COH1 (III) | 44/45 | 98 |
| CRM-III | MF59 | 898 | COH1 (III) | 68/80 | 85 |
| PBS | - | - | COH1 (III) | 0/68 | 0 |
| - Not applicable | | | | | |

High levels of protection were achieved with the monovalent vaccines for all three serotypes with both aluminum hydroxide and MF59. However, slightly lower survival rates were obtained with the adjuvant MF59 even in presence of higher antibody titers.

In an additional experiment, mice were immunized with three doses of combinations at 1 μg of each conjugate in the presence of adjuvant on days 0, 21 and 35. The neonates were challenged with type specific strains as shown below in Table 2.

*Table 2: Determination of the protection level and antibody titers obtained with combinations of GBS serotype Ia, Ib and III/CRM197 conjugates in the presence or absence of adjuvant in the active maternal-neonatal challenge mouse model.*

| Antigen | Adjuvant | GMT titers | Challenge Strain (type) | Alive/Treated | Survival (%) |
|---|---|---|---|---|---|
| Combo | Al-H | 1279 | 090 (Ia) | 27/30 | 90 |
| Combo | MF59 | 4592 | 090 (Ia) | 61/65 | 94 |
| Combo | No-Adjuvant | 218 | 090 (Ia) | 59/77 | 77 |
| PBS | - | - | 090 (Ia) | 0/70 | 0 |
| Combo | Al-H | 2086 | H36B (Ib) | 64/70 | 91 |
| Combo | MF59 | 5921 | H36B (Ib) | 65/80 | 81 |
| Combo | No-Adjuvant | 386 | H36B (Ib) | 56/70 | 80 |
| PBS | - | - | H36B (Ib) | 6/79 | 7 |
| Combo | Al-H | 596 | M781 (III) | 30/40 | 75 |
| Combo | MF59 | 1978 | M781 (III) | 70/70 | 100 |
| Combo | No-Adjuvant | 163 | M781 (III) | 60/79 | 76 |
| PBS | - | - | M781 (III) | 3/77 | 4 |
| Combo = CRM197-Ia + CRM197-Ib + CRM197-III; - : Not applicable | | | | | |

High levels of protection were achieved with the combination vaccines in all three formulations in the presence or absence of adjuvant, although lower antibody titers were achieved in the absence of adjuvant.

### Mouse study (2)

In this study, the effect of lyophilization on the efficacy and immunogenicity of the serotype Ia, Ib and III/CRM197 conjugates in the active maternal-neonatal challenge mouse model was evaluated. Mice were immunized with two doses (1 μg each) of the three different conjugates, in the presence or absence of adjuvant on days 0, and 21. The neonates were challenged with type specific strains as shown below in Table 3.

*Table 3: Determination of the protection level, antibody titers and bactericidal titers achieved by the serotype Ia, Ib and III/CRM197 conjugates, when administered to mice as liquid or lyophilized antigen in the presence or absence of aluminum hydroxide in the active maternal-neonatal challenge mouse model.*

| Antigen | Adjuvant | GMT titer | Bactericidal titer | Challenge Strain (type) | Alive/ Treated | Survival (%) |
|---|---|---|---|---|---|---|
| CRM-Ia Lyophilized | PBS | 48 | < 100 | 090 (Ia) | 46/78 | 59 |
| CRM-Ia Lyophilized | Al-H | 1201 | 567 | 090 (Ia) | 47/51 | 92 |
| CRM-Ia Liquid | PBS | 14 | < 100 | 090 (Ia) | 11/60 | 18 |
| CRM-Ia Liquid | Al-H | 901 | 436 | 090 (Ia) | 58/60 | 96 |
| PBS | Al-H | - | - | 090 (Ia) | 1/57 | 2 |
| CRM-Ib Lyophilized | PBS | 23 | 366 | H36B (Ib) | ND | ND |
| CRM-Ib Lyophilized | Al-H | 172 | 2146 | H36B (Ib) | ND | ND |
| CRM-Ib Liquid | PBS | 27 | 375 | H36B (Ib) | 12/40 | 30 |
| CRM-Ib Liquid | Al-H | 169 | 1756 | H36B (Ib) | 73/78 | 93 |
| PBS | Al-H | - | - | H36B (Ib) | 6/72 | 8 |
| CRM-III Lyophilized | PBS | 59 | 419 | M781 (III) | ND | ND |
| CRM-III Lyophilized | Al-H | 429 | 1861 | M781 (III) | ND | ND |
| CRM-III Liquid | PBS | 127 | 1707 | M781 (III) | 48/50 | 96 |
| CRM-III Liquid | Al-H | 198 | 1100 | M781 (III) | 44/45 | 98 |
| PBS | Al-H | - | - | M781 (III) | 5/66 | 7 |
| - Not applicable; ND: not determined | | | | | | |

The lyophilization process did not affect the immunogenicity of the GBS conjugates. Antibody titers and bactericidal titers were comparable in mice that received both liquid and lyophilized formulations.

**Mouse study (3)**

In this study, the effect of lyophilization on the efficacy of the serotype V/CRM197 conjugate in the active maternal-neonatal challenge mouse model was evaluated. Mice were immunized with two doses (1, 5 or 10 μg each) of the conjugates in the presence or absence of adjuvant on days 0 and 21. The neonates were challenged with a type V strain. The results are shown in Table 4 below.

*Table 4: Determination of the protection level achieved by the serotype V/CRM197 conjugate, when administered to mice as liquid or lyophilized antigen in presence or absence of aluminum hydroxide in the active maternal-neonatal challenge mouse model.*

| Antigen | No adjuvant | Aluminum hydroxide |
|---|---|---|
| | Dead/treated (% survival) | |
| CRM-V Lyophilized (1μg) | 20/30 (33) | 13/44 (70) |
| CRM-V Lyophilized (5μg) | 28/39 (28) | 31/40 (22) |
| CRM-V Lyophilized (10μg) | 40/54 (26) | 33/49 (33) |
| CRM-V Liquid (1μg) | 63/70 (10) | 19/47 (59) |
| CRM-V Liquid (5μg) | 29/40 (27) | 37/60 (38) |
| CRM-V Liquid (10μg) | 46/52 (11) | 46/70 (34) |

(continued)

| | No adjuvant | Aluminum hydroxide |
|---|---|---|
| **Antigen** | Dead/treated (% survival) | |
| Placebo Lyophilized | 108/119 (9) | 70/88 (20) |

The lyophilization process did not affect the immunogenicity of the GBS conjugate. Survial rates were comparable in mice that received both liquid and lyophilized formulations.

### Mouse study (4)

In this study, the effect of different doses on the efficacy of a mixture of the GBS serotype Ia, Ib, III and V conjugates was evaluated in the active maternal-neonatal challenge mouse model. Mice were immunized with two doses of the combination at 0.2, 1 or 5 $\mu$g of each of the GBS serotype Ia, Ib, III and V conjugates without adjuvant on days 0 and 21. The neonates were challenged with type specific strains as shown below in Table 5 below.

*Table 5: Determination of the protection level obtained with combinations of GBS serotype Ia, Ib, III and V/CRM197 conjugates in the active maternal-neonatal challenge mouse model.*

| | | Challenge Strain (type) | | | |
|---|---|---|---|---|---|
| **Antigen** | **Doses** | 090 (Ia) | H36B (Ib) | M781 (III) | CJB111 (V) |
| | | Dead/treated (% survival) | | | |
| Combo | 5 $\mu$g each | 17/60 (72) | 13/70 (81) | 18/70 (74) | 20/68 (70) |
| Combo | 1 $\mu$g each | 23/70 (67) | 18/70 (74) | 6/60 (90) | 44/66 (33) |
| Combo | 0.2 $\mu$g each | 14/51 (72) | 25/79 (68) | 18/78 (77) | 41/60 (32) |
| PBS | 0 | 57/58 (2) | 49/50 (2) | 49/50 (2) | 45/50 (10) |
| Combo = CRM197-Ia + CRM197-Ib + CRM197-III + CRM197-V | | | | | |

Higher dosage of GBS serotype V conjugate raised the level of protection.

### Mouse study (5)

In this study, the effect of different numbers of doses on the efficacy of a mixture of the GBS serotype Ia, Ib, III and V conjugates was evaluated in the active maternal-neonatal challenge mouse model. Mice were immunized with one, two or three doses (1 $\mu$g of each conjugate) of the combination in the presence of an alum adjuvant on days 0, 21 and 35 as appropriate. The neonates were challenged with type specific strains as shown below in Table 6 below.

*Table 6: Determination of the protection level obtained with combinations of GBS serotype Ia, Ib, III and V/CRM197 conjugates in the presence of adjuvant in the active maternal-neonatal challenge mouse model.*

| | | Challenge Strain (type) | | | |
|---|---|---|---|---|---|
| **Antigen** | **Doses** | 090 (Ia) | H36B (Ib) | M781 (III) | CJB111 (V) |
| | | Dead/treated (% survival) | | | |
| Combo | 3 | 5/80 (94) | 4/60 (93) | 1/50 (98) | 32/70 (54) |
| Combo | 2 | 6/80 (92) | 11/60 (82) | 1/70 (98) | 42/57 (26) |
| Combo | 1 | 61/90 (32) | 21/50 (58) | 4/60 (93) | 52/58 (10) |
| PBS | 3 | 50/50 (0) | 49/50 (2) | 52/58 (10) | 59/60 (2) |
| Combo = CRM197-Ia + CRM197-Ib + CRM197-III + CRM197-V | | | | | |

Bactericidial titers were measured following administration of the mixture of serotype Ia, Ib, III and V conjugates in this study. OPA titers are shown below:

|  | Ia | Ib | III | V |
|---|---|---|---|---|
| **Post-3** | 515 | >900 | 1174 | 135 |
| **Post-2** | <100 | 455 | 525 | <100 |
| **Post-1** | <100 | 182 | 358 | <100 |

The number of immunizations strongly affected the immune response to the GBS serotype V conjugate.

*Mouse study (6)*

In this study, the efficacy of a mixture of the GBS serotype Ia, Ib, III and V conjugates compared to the GBS serotype V conjugate alone was evaluated in the active maternal-neonatal challenge mouse model. Mice were immunized with three doses of combinations at 1 μg of each conjugate or GBS serotype V conjugate at 1 μg in the presence of alum adjuvant on days 0, 21 and 35. The neonates were challenged with the CJB 111 and 2603 V/R type V strains. The results are shown in Table 7 below.

*Table 7: Determination of the protection level obtained with combinations of GBS serotype Ia, Ib, III and V/CRM197 conjugates or GBS serotype V conjugate alone in the presence of adjuvant in the active maternal-neonatal challenge mouse model.*

| Antigen | Challenge Strain (type) | |
|---|---|---|
|  | CJB111 (V) | 2603 V/R (V) |
|  | Dead/treated | (% survival) |
| CRM197-V | 78/253 (69) | 9/117 (92) |
| Combo | 218/583 (63) | 32/118 (73) |
| PBS | 333/350 (5) | 138/149 (6) |
| Combo = CRM197-Ia + CRM197-Ib + CRM197-III + CRM197-V | | |

The immune response to the capsular saccharide from GBS serotype V was diminished when GBS serotype Ia, Ib and III conjugates were also present in the composition.

*Mouse study (7)*

In this study, the effect of adjuvant on the immunogenicity and efficacy of a mixture of the GBS serotype Ia, Ib, III and V conjugates compared to the GBS serotype V conjugate alone was evaluated in the active maternal-neonatal challenge mouse model. Mice were immunized with three doses of combinations at 1 μg of each conjugate or GBS serotype V conjugate at 1 μg in the presence or absence of adjuvant on days 0, 21 and 35. The neonates were challenged with the CJB111 type V strain. The results are shown in Table 8 below.

*Table 8: Determination of the protection level, antibody titers and bactericidal titers obtained with combinations of GBS serotype Ia, Ib, III and V/CRM197 conjugates or GBS serotype V conjugate alone in the presence or absence of adjuvant in the active maternal-neonatal challenge mouse model.*

| Antigen | Adjuvant | GMT titer | Bactericidal titer | Dead/treated (% survival) |
|---|---|---|---|---|
| CRM197-V | PBS | 83 | 838 | 21/68 (69) |
| Combo | PBS | 22 | 251 | 62/130 (52) |
| CRM197-V | Alum | 130 | 1430 | 30/80 (62) |
| Combo | Alum | 59 | <100 | 66/148 (55) |
| PBS | Alum | - | - | 122/131 (7) |
| Combo = CRM197-Ia + CRM197-Ib + CRM197-III + CRM197-V; - Not applicable | | | | |

Once again, the immune response to the capsular saccharide from GBS serotype V was diminished when GBS serotype Ia, Ib and III conjugates were also present in the composition. Survival was improved by addition of adjuvant, even though addition of adjuvant to the GBS serotype V conjugate alone did not have this effect in this experiment.

***Mouse study (8)***

In this study, the effect of increasing the dose of GBS serotype V conjugate on the efficacy of a mixture of the GBS serotype Ia, Ib, III and V conjugates was evaluated in the active maternal-neonatal challenge mouse model. Mice were immunized with two doses of combinations at 1 μg of each conjugate or two doses of combinations at 1 μg of the the GBS serotype Ia, Ib, III conjugates and 5 μg of the GBS serotype V conjugate in the presence or absence of adjuvant on days 0 and 21. The neonates were challenged with type specific strains as shown below in Table 9 below.

**Table 9:** *Determination of the protection level obtained with combinations of GBS serotype Ia, Ib, III and V/CRM197 conjugates in the presence of adjuvant at with different doses of GBS serotype V conjugate in the active maternal-neonatal challenge mouse model.*

| Antigen | Adjuvant | Challenge Strain (type) | | | |
|---|---|---|---|---|---|
| | | 090 (Ia) | H36B (Ib) | M781 (III) | CJB111 (V) |
| | | Dead/treated (% survival) | | | |
| Combo | PBS | 20/59 (66) | 12/50 (76) | 1/40 (97) | 49/50 (2) |
| Combo | Alum | 36/50 (28) | 10/30 (67) | 1/40 (97) | 22/40 (45) |
| Combo plus | PBS | 40/40 (0) | 23/26 (11) | 37/40 (7) | 31/38 (18) |
| Combo plus | Alum | 13/45 (71) | 15/40 (62) | 0/50 (100) | 26/50 (48) |
| Combo = CRM197-Ia + CRM197-Ib + CRM197-III + CRM197-V (all at 1 μg) | | | | | |
| Combo plus = CRM197-Ia + CRM197-Ib + CRM197-III + CRM197-V (all at 1 μg, except for CRM197-V at 5 μg) | | | | | |

In this experiment, the immune response to the capsular saccharide from GBS serotype V in the mixture was once again improved by addition of adjuvant. The response was also improved by increasing the dose of this capsular saccharide in the composition. However, the presence of a high dose of capsular saccharide from GBS serotype V seemed to reduce the response to the capsular saccharides from GBS serotype Ia, Ib and III. This consequence was reduced by addition of adjuvant.

***Mouse study (9)***

In this study, the efficacy of a mixture of the GBS serotype Ia, Ib and III conjugates with GBS67 and GBS80 proteins was evaluated in the active maternal-neonatal challenge mouse model. Mice were immunized with combinations in the presence or absence of various different adjuvants. The neonates were challenged with type specific strains as shown below in Table 10 below.

**Table 10:** *Determination of the protection level obtained with combinations of GBS serotype Ia, Ib and III/CRM197 conjugates and GBS67 and GBS80 proteins in the active maternal-neonatal challenge mouse model.*

| Ag | Adjuvant | Challenge Strain (type) | | | | | |
|---|---|---|---|---|---|---|---|
| | | 090 (Ia) | H36B (Ib) | 3050 (II) | M781 (III) | CJB111 (V) | JM9130013 (VIII) |
| | | Dead/treated (% survival) | | | | | |
| Combo | Alum hydroxide/ saline | 9/60 (85) | 6/60 (90) | 12/58 (79) | 0/60 (100) | 11/55 (80) | 28/56 (50) |
| Combo | Alum hydroxide/ PBS | 19/78 (76) | 5/57 (91) | 16/66 (76) | 4/53 (92) | | 55/80 (31) |
| Combo | MF59 | 4/60 (93) | 12/57 (79) | 18/60 (70) | 3/77 (96) | | 45/70 (36) |
| Combo | None | 13/80 (84) | 11/70 (84) | 28/60 (53) | 14/77 (82) | | 47/59 (20) |
| PBS | Alum hydroxide/ saline | 60/60 (0) | 74/77 (4) | 36/56 (36) | 73/80 (9) | 86/99 (13) | 53/69 (23) |

(continued)

| Ag | Adjuvant | Challenge Strain (type) | | | | | |
|---|---|---|---|---|---|---|---|
| | | 090 (Ia) | H36B (Ib) | 3050 (II) | M781 (III) | CJB111 (V) | JM9130013 (VIII) |
| | | Dead/treated (% survival) | | | | | |
| PBS | None | 70/70 (0) | 73/79 (7) | 42/54 (22) | 74/77 (4) | | 63/74 (15) |
| Combo = CRM197-Ia + CRM197-Ib + CRM197-III + GBS67 + GBS80 | | | | | | | |

Antibody titers were measured following administration of the mixture of serotype Ia, Ib, III and V conjugates and GBS67 and GBS80 proteins in this study. Results from five separate experiments are shown below:

| | Aluminium Hydroxide/saline | Aluminium Hydroxide/ PBS | MF 59 | No Adjuvant |
|---|---|---|---|---|
| GBS 80 | 45395 | 50277 | 15626 | 3358 |
| GBS 67 | 25846 | 29513 | 9616 | 4232 |
| Ps Ia | 811 | 711 | 2190 | 404 |
| Ps Ib | 1929 | 1277 | 2571 | 691 |
| Ps III | 862 | 1043 | 1314 | 275 |

### Mouse study (10)

In this study, the efficacy of a mixture of the GBS serotype Ia, Ib and III conjugates with GBS67 and GBS80 proteins was evaluated in the active maternal-neonatal challenge mouse model. Mice were immunized with three doses of combinations in the presence or absence of various different adjuvants at days 0, 21 and 35. The neonates were challenged with type specific strains as shown below in Table 11 below.

**Table 11:** *Determination of the protection level obtained with combinations of GBS serotype Ia, Ib and III/CRM197 conjugates and GBS67 and GBS80 proteins in the active maternal-neonatal challenge mouse model.*

| Antigen | Adjuvant | Challenge Strain (type) | |
|---|---|---|---|
| | | 090 (Ia) | M781 (III) |
| | | Dead/treated (% survival) | |
| Combo | Alum | 4/48 (92) | 6/50 (88) |
| Combo | Alum + CpG | 6/78 (92) | 7/8 (91) |
| Combo | MF59 | 4/69 (94) | 13/55 (76) |
| Combo | MF59 + CpG | 5/66 (92) | 6/70 (91) |
| Combo | PBS | 22/60 (63) | 13/55 (76) |
| Combo | PBS + CpG | 5/59 (91) | 11/66 (83) |
| PBS | - | 69/69 (0) | 60/65 (7) |
| Combo = CRM197-Ia + CRM197-Ib + CRM197-III + CRM197-V | | | |

$$\text{Combo} = \text{CRM197-Ia} + \text{CRM197-Ib} + \text{CRM197-III} + \text{GBS67} + \text{GBS80}$$

### Mouse study (11)

In this study, the efficacy of a mixture of the GBS serotype Ia, Ib, III and V conjugates with GBS67 protein and a SpbI-GBS80 fusion protein was evaluated in the active maternal-neonatal challenge mouse model. Mice were immunized with the combination in the presence of adjuvant. The neonates were challenged with type specific strains as shown below in Table 12 below.

*Table 12: Determination of the protection level obtained with combinations of GBS serotype Ia, Ib, III and V/CRM197 conjugates, GBS67 protein and a SpbI-GBS80 fusion protein protein in the active maternal-neonatal challenge mouse model.*

| Ag | Adjvnt | Challenge Strain (type) | | | | | | | |
|---|---|---|---|---|---|---|---|---|---|
| | | 090 (Ia) | H36B (Ib) | 3050 (II) | COH1 (III) | M781 (III) | M732 (III) | CJB111 (V) | JM913 (VII) |
| | | Dead/treated (% survival) | | | | | | | |
| Combo | Alum | 16/70 (77) | 10/70 (86) | 7/70 (90) | 4/77 (95) | 0/60 (100) | 19/30 (36) | 20/58 (65) | 29/50 (42) |
| PBS | Alum | 68/68 (0) | 37/50 (26) | 25/40 (37) | 65/69 (6) | 37/40 (8) | 32/60 (47) | 36/40 (10) | 33/40 (17) |
| Combo = CRM197-Ia + CRM197-Ib + CRM197-III + GBS67 protein + SpbI-GBS80 fusion protein | | | | | | | | | |

### Mouse study (12)

In this study, the efficacy of GBS serotype Ia, Ib, III and V conjugates was evaluated in the active maternal-neonatal challenge mouse model. Mice were immunized at 1 µg of each conjugate. The neonates were challenged with type specific strains as shown below in Table 13 below. The experiment was repeated twice.

*Table 13: Determination of the protection level obtained with GBS serotype Ia, Ib, III and V/CRM197 conjugates in the active maternal-neonatal challenge mouse model.*

| Expt | Antigen | Challenge Strain (type) | | | |
|---|---|---|---|---|---|
| | | 090 (Ia) | H36B (Ib) | M781 (III) | CJB111 (V) |
| | | Dead/treated (% survival) | | | |
| 1 | CRM197-Ia | 10/70 (86) | 64/70 (9) | 76/80 (5) | |
| | CRM197-Ib | 48/94 (51) | 12/99 (88) | | |
| | CRM 197-III | 69/70 (1) | | 3/60 (95) | 60/68 (12) |
| | CRM197-V | | | 80/89 (10) | 5/100 (95) |
| | PBS/Alum | 50/50 (0) | 39/40 (2) | 61/69 (12) | 10/10 (0) |
| 2 | CRM197-Ia | 14/78 (82) | 68/70 (2) | 41/42 (2) | |
| | CRM197-Ib | 66/110 (40) | 2/110 (98) | | |
| | CRM197-III | 70/80 (12) | | 1/60 (98) | |
| | CRM197-V | | | | 38/192 (80) |
| | PBS/Alum | 45/45 (0) | 57/58 (2) | 32/36 (11) | 50/58 (14) |

The conjugate comprising capsular saccharide from GBS serotype Ib conferred protection against GBS serotype Ia in addition to GBS serotype Ib.

### Mouse study (13)

In this study, capsular saccharide conjugated to either tetanus toxoid (TT) carrier protein or CRM197 carrier protein were tested and compared for their immunogenicity. Female CD1 mice were immunized with two doses of 1 µg each of GBS serotype Ia, Ib and III conjugates with aluminum hydroxide adjuvant on days 0 and 21. The neonates were challenged with specific strain types as shown below in Table 14.

*Table 14: Determination of the protection achieved by GBS serotype Ia, Ib and III capsular saccharide conjugated to TT or CRM in the active maternal-neonatal challenge mouse model.*

| CPS type | Challenge strain (type) | Carrier protein Tetanus Toxoid* | Carrier protein Crm* | PBS* |
|---|---|---|---|---|
| Ia | 090 (Ia) | 78 (52/67) | 86 (54/63) | 0 (0/59) |
| Ib | 7357B (Ib) | 62 (50/80) | 73 (71/97) | 0 (0/38) |
| III | COH1 (III) | 97 (37/38) | 93 (95/102) | 2 (1/48) |
| *% Survival (Alive/treated) | | | | |

The survival rates in the groups immunized with CRM197 conjugates of all three serotypes were comparable to the survival rates observed in the immunized group with TT conjugates. Based on these results, CRM197 was selected as the carrier protein for further development.

**Mouse study (14)**

In this study, the impact of the level of capsular saccharide oxidation during the covalent conjugation process on immunogenicity was evaluated. Several batches of GBS serotype Ia, Ib and III conjugates were obtained with the saccharides prepared at different percentages of oxidation conjugated either to TT and/or to CRM197 and tested in female CD1 mice for their immunogenicity. Mice were immunized with two doses (1 $\mu$g each) of the three different conjugates in the presence of aluminum hydroxide adjuvant on days 0 and 21. The neonates were challenged with type specific strains as shown below in Table 15.

*Table 15: Determination of the protection level, antibody titers and bactericidal titers of GBS serotype Ia and III capsular saccharide conjugated either to TT or CRM197 and different percentage of oxidation using the active maternal-neonatal challenge mouse model.*

| Antigen | Oxidation level (%) | GMT titers | Challenge Strain (type) | Alive/ Treated | Survival (%) | Bactericidal Titer |
|---|---|---|---|---|---|---|
| CRM-Ia | 5,1 | ND | A909 (Ia) | 42/80 | 52 | ND |
| CRM-Ia | 14,2 | ND | A909 (Ia) | 58/60 | 97 | ND |
| CRM-Ia | 44,7 | ND | A909 (Ia) | 48/78 | 61 | ND |
| CRM-Ia | 79 | ND | A909 (Ia) | 6/50 | 12 | ND |
| PBS | - | - | A909 (Ia) | 11/87 | 13 | - |
| TT- III | 3,9 | 5135 | COH1 (III) | 66/78 | 85 | 575 |
| TT-III | 16 | 7662 | COH1 (III) | 55/59 | 93 | 470 |
| TT-III | 20 | 6850 | COH1 (III) | 47/48 | 98 | 1320 |
| TT-III | 55 | 13290 | COH1 (III) | 64/70 | 91 | 1320 |
| PBS | - | - | COH1 (III) | 1/79 | 1 | - |
| CRM-III | 4,3 | 972 | COH1 (III) | 64/100 | 64 | 127 |
| CRM-III | 17,5 | 812 | COH1 (III) | 77/83 | 93 | 150 |
| CRM-III | 40,9 | 2484 | COH1 (III) | 98/107 | 91 | 183 |
| CRM-III | 61,8 | 8690 | COH1 (III) | 75/85 | 88 | 140 |
| CRM-III | 78,9 | 58629 | COH1 (III) | 67/80 | 84 | 150 |
| PBS | - | - | COH1 (III) | 0/73 | 0 | - |
| - not applicable | | | | | | |

The survival rates reached a peak when the dams were immunized with conjugates prepared from saccharides oxidized at 15-20%. The antibody titers increased with increasing levels of oxidation without any impact on the function. The bactericidal titers did not increase with higher antibody titers. Based on these results and on additional experiments (data not shown), the optimal percentage of CPS oxidation for all three serotypes was defined as being between 10 and 30%.

*Reproductive and developmental toxicology studies in rabbits and rats*

Results from two species showed no effect of the serotype Ia, Ib and III/CRM197 conjugates on embryonic or fetal development.

In rabbits, a combination of the three conjugates with aluminum hydroxide adjuvant was administered by intramuscular injection at a clinical dose of 20/20/20 $\mu$g (based on mass of each saccharide) on days -35, -21 and -7 relative to mating on day 0 (pre-mating period) and on gestation days 7 and 20 or on gestation days 7 or 20 only. Treatment resulted in neither maternal toxicity, effects on mating nor evidence of embryo lethality, fetotoxicity or teratogenicity at any dose level.

In rats, there was similarly no maternal toxicity or evidence of effects on reproductive function and embryo-fetal development when the combination was administered, and no difference was noted between groups administered the combination in saline versus the combination in aluminum hydroxide adjuvant in 3 (during gestation only) or 6 (prior to gestation and during gestation) injections. Injections were given on days -35, -21 and -7 relative to mating on day 0 as well as on days 6, 12 and 17 of gestation or only on days 6, 12 and 17 of gestation. There was no effect on the F1 generation pup survival, clinical condition or body weight during the pre-weaning period.

*Human study (1)*

This study investigated a monovalent GBS serotype Ia capsular saccharide-CRM197 conjugate vaccine. Test groups of 10 subjects were administered 1 or 2 injections at 5, 10 or 20 $\mu$g (measured as mass of saccharide) doses. Placebo groups of 3 and 2 subjects received 1 and 2 injections of saline respectively. Blood was drawn from each subject at screening and a month after the first injection for analysis by ELISA. Additionally, at 3 months into the study, the 2-injection groups had a blood draw at the time they received the second injection, and then returned a month later for another blood draw. Further blood draws were carried out at 6, 12 and 24 months after the last injection the subject had received.

The ELISA measures the concentration of specific antibodies against GBS Ia (or Ib and III in the studies described below) capsular saccharides. Microtiter plates were coated with 1 $\mu$g/ml of the appropriate GBS saccharide (conjugated to HSA) and were incubated with sera from study subjects for 1h at 37°C. After 3 washes, the plates were incubated with an alkaline phosphatase (AP) labeled anti-human IgG secondary antibody for 90 min at 37°C followed by additional 3 washes. The substrate (pNPP) was added to the plate and incubated for 30 min at room temperature. The AP catalyzes the hydrolysis of the substrate generating a colorimetric reaction which can be quantified by an ELISA reader at 405 nm (reference filter 650 nm). The evaluation of the antibody concentration was done using a standard curve. A summary of the geometric mean concentration ($\mu$g/ml) of anti-Ia antibodies for each group is given in table 16 below:

EP 2 616 099 B1

*Table 16:* *Geometric mean concentrations (and geometric mean ratios) for monovalent GBS serotype Ia capsular saccharide-CRM197 conjugate vaccine study.*

| | Placebo N=3 | Placebo+ N=2 | GBS Ia 5 N=10 | GBS Ia 5+ N=10 | GBS Ia10 N=10 | GBS Ia10+ N=10 | GBS Ia20 N=10 | GBS Ia20+ N=10 |
|---|---|---|---|---|---|---|---|---|
| Baseline (visit 0) | 0.84 (0.12-5.86) | 0.16 (0.015-1.77) | 1.05 (0.36-3.05) | 0.42 (0.14-1.29) N=9 | 1.25 (0.38-4.1) N=8 | 0.37 (0.1-1.32) N=7 | 0.54 (0.19-1.57) | 0.2 (0.07-0.59) |
| 1 month after last immunization | 0.8 (0.034-19) | 0.16(0.0007-38) N=1 | 43 (7.6-240) | 5.94 (1.06-33) | 7.94 (1.41-45) | 14 (2.52-79) | 25 (4.45-140) | 6.88(1.12-42) N=9 |
| 1 month after last immunization to Baseline (visit 0) | 0.96 (0.093-9.92) | 1 (0.018-57) N=1 | 41 (11-146) | 16 (4.05-60) N=9 | 10 (2.4-42) N=8 | 53 (12-246) N=7 | 46 (13-166) | 33 (8.55-127) N=9 |
| 6 months after last immunization | 0.73 (0.05-11) | 0.16(0.0016-17) N=1 | 19 (4.43-83) | 3.08 (0.71-13) | 12 (2.66-50) | 7.03 (1.63-30) | 14 (3.22-60) | 4.21 (0.9-20) N=9 |
| 6 months after last immunization to Baseline (visit 0) | 0.87 (0.12-6.39) | 1 (0.032-31) N=1 | 18 (6.14-54) | 7.9 (2.5-25) N=9 | 13 (3.71-43) N=8 | 27 (7.2-98) N=7 | 26 (8.67-77) | 20 (6.39-64) N=9 |
| 12 months after last immunization | 0.79 (0.054-12) | 0.16(0.0016-17) N=1 | 14 (3.3-62) | 2.23 (0.51-9.68) | 7.28 (1.68-32) | 7.17 (1.53-34) N=9 | 9.96 (2.29-43) | 3.24(0.63-17) N=8 |
| 12 months after last immunization to Baseline (visit 0) | 0.94 (0.14-6.46) | 1 (0.036-28) N=1 | 14 (4.76-39) | 5.59 (1.84-17) N=9 | 8.02 (2.46-26) N=8 | 19 (5.49-68) N=7 | 18 (6.43-53) | 17 (5.31-56) N=8 |
| 24 months after last immunization | 0.66 (0.043-10) | | 3.57 (0.59-21) N=7 | 2.74 (0.56-13) N=9 | 10 (1.74-63) N=7 | 5.05 (0.84-30) N=7 | 8.63(1.78-42) N=9 | 3.46(0.65-19) N=8 |
| 24 months after last immunization to Baseline (visit 0) | 0.79 (0.11-5.95) | | 7.47 (2-28) N=7 | 6.51 (1.9-22) N=8 | 7.88 (1.9-33) N=6 | 14 (3.26-56) N=6 | 14 (4.38-45) N=9 | 16 (4.68-55) N=8 |

+ two injections

Overall the GMC data show a significant increase between baseline and later timepoints (e.g. GMC range from 6 to 43 $\mu$g/ml one month after the last vaccination) and although there was a decline over time, 24 months into the study, the group GMC were still multiple-fold higher than at baseline (the GMR ranges from 7 to 14 at 24 months). Judging by the GMC point estimate, the group receiving the 5 $\mu$g dose as a single vaccination had the highest overall response at a month after the last vaccination.

The number of subjects with antibody levels ≥ 3 $\mu$g/mL showed similar numbers of "responders" across the different doses (11, 13 and 12 out of 20 for 5, 10 and 20 doses respectively), and different vaccination schedules (18 out of 20 for both), at a month after the last vaccination (data not shown),. The percentage of subjects with antibody levels ≥ 5 $\mu$g/mL confirmed the same observations (data not shown). These cut-offs were intended to allow responses to be assessed in the context of potential serologic correlates of protection (based on ref. 250). These data suggest that there is no observable contribution by either a second vaccination or a higher vaccine dose. As no dose-response was observed, it is possible that a dose of 5 $\mu$g or lower may be an optimal dose in an adult population. No sustained advantage was observed from administering 2 injections compared to 1 injection for the groups receiving 5 and 20 $\mu$g. The group receiving 10 $\mu$g dose showed higher peak responses (at 1 month post vaccination) after two vs one injection, but this trend was reversed at subsequent (steady-state) time-points.

Safety analysis was assessed based on a number of different criteria. No safety issues stood out, and no dose dependent response was noticeable.

*Human study (2)*

This study investigated monovalent GBS serotype Ib and III capsular saccharide-CRM197 conjugate vaccines. Test groups of 10 subects were administered 1 or 2 injections at 5, 10 or 20 $\mu$g (measured as mass of saccharide) doses. Placebo groups of 3 and 2 subjects received 1 and 2 injections of saline respectively. Blood was drawn from each subject at screening and a month after the first injection for analysis by ELISA. Additionally, at 3 months into the study, the 2-injection groups had a blood draw at the time they received the second injection, and then returned a month later for another blood draw. Further blood draws were carried out at 6, 12 and 24 months after the last injection the subject had received. A summary of the geometric mean concentration of anti-Ib and III antibodies for each group is given in table 17 below.

*Table 17:* Geometric mean concentrations (and geometric mean ratios) for monovalent GBS serotype III and Ib capsular saccharide-CRM197 conjugate vaccines study.

| | | Placebo | Placebo+ | GBS Ib 5 | GBS Ib 5+ | GBS Ib10 | GBS Ib10+ | GBS Ib20 | GBS Ib20+ |
|---|---|---|---|---|---|---|---|---|---|
| | | N=2 | N=2 | N=8 | N=10 | N=9 | N=11 | N=9 | N=10 |
| **GBS Ib** | Baseline (visit 0) | 0.1 (0.0042-2.38) N=1 | 0.042 (0.0017-0.99) N=1 | 0.27 (0.066-1.13) N=5 | 0.24 (0.08-0.75) N=8 | 0.088 (0.021-0.36) N=5 | 0.44 (0.16-1.21) N=10 | 0.38 (0.11-1.25) N=7 | 0.2 (0.074-0.55) |
| | 1 month after last immunization | 0.3 (0.0019-47) N=1 | 0.53 (0.015-19) | 1.89 (0.32-11) | 11 (1.77-63) N=8 | 2.63 (0.49-14) | 18 (3.82-81) | 14 (2.6-76) | 10 (2.11-52) |
| | 1 month after last immunization to Baseline (visit 0) | | 9.64 (0.26-359) N=1 | 60 (9.78-364) N=4 | 47 (11-208) N=6 | 46 (9.13-232) N=5 | 48 (15-152) N=10 | 68 (17-267) N=7 | 56 (17-186) N=9 |
| | 6 months after last immunization | 0.091 (0.0051-1.63) | 0.3 (0.0051-18) N=1 | 1.53 (0.36-6.48) | 7.52 (2.07-27) | 5.45 (1.4-21) | 11 (3.09-36) | 6.23 (1.6-24) | 9.2 (2.53-33) |
| | 6 months after last immunization to Baseline (visit 0) | 0.42 (0.031-5.64) N=1 | | 32 (8.56-116) N=4 | 36 (14-91) N=8 | 97 (30-310) N=5 | 28 (12-63) N=10 | 26 (9.61-69) N=7 | 47 (20-112) N=9 |
| | 12 months after last immunization | 0.091 (0.0054-1.53) | 0.4 (0.0074-22) N=1 | 1.29 (0.31-5.26) | 5.77 (1.53-22) N=9 | 3.24 (0.79-13) N=8 | 9.52 (2.86-32) | 4.98 (1.32-19) | 6.67 (1.89-24) |
| | 12 months after last immunization to Baseline (visit 0) | 0.42 (0.036-4.85) N=1 | | 22 (6.52-76) N=4 | 29 (12-75) N=7 | 45 (13-152) N=4 | 26 (12-57) N=10 | 23 (8.96-57) N=7 | 34 (15-78) N=9 |
| | 24 months after last immunization | 0.091 (0.004-2.07) | 0.1 (0.0012-8.29) N=1 | 1.34 (0.28-6.4) | 3.8 (0.94-15) | 2.58 (0.59-11) | 9.49 (2.35-38) N=10 | 3.22 (0.53-20) N=6 | 3.92 (0.9-17) N=9 |
| | 24 months after last immunization to Baseline (visit 0) | | | 27 (6.49-111) N=4 | 19 (7.07-53) N=8 | 53 (15-188) N=5 | 25 (9.65-64) N=9 | 15 (3.55-61) N=4 | 22 (7.95-59) N=8 |
| | | N=3 | N=2 | N=8 | N=10 | N=10 | N=9 | N=9 | N=10 |
| **GBS III** | Baseline (visit 0) | 0.034 (0.0007-1.55) N=1 | 0.27 (0.018-3.98) | 0.27 (0.057-1.3) N=6 | 1.64 (0.46-5.86) N=9 | 0.23 (0.065-0.83) N=9 | 0.65 (0.18-2.34) | 0.89 (0.21-3.77) N=7 | 1.93 (0.58-6.47) |
| | 1 month after last immunization | 0.14 (0.0099-2.11) | 0.88 (0.033-24) | 13 (2.44-65) | 31 (7.07-134) | 2.72 (0.63-12) | 31 (6.64-147) | 22 (4.73-105) | 22 (5.03-95) |
| | 1 month after last immunization to Baseline (visit 0) | 8.82 (0.64-122) N=1 | 3.31 (0.51-21) | 44 (15-130) N=6 | 31 (12-84) N=7 | 10 (4.26-25) N=9 | 42 (16-106) N=8 | 63 (23-172) N=7 | 14 (5.94-34) N=9 |
| | 6 months after last immunization | 0.034 (0.0005-2.11) N=1 | 1.9 (0.031-118) N=1 | 4.97 (1.04-24) N=7 | 21 (5.57-76) | 2.7 (0.73-9.95) | 19 (4.89-77) | 18 (4.43-69) | 15 (3.84-60) N=9 |

| | | | | | | | |
|---|---|---|---|---|---|---|---|
| 6 months after last immunization to Baseline (visit 0) | 0.9 (0.086-9.48) N=1 | 23 (8.85-60) N=6 | 22 (8.87-52) N=7 | 11 (5-24) N=9 | 25 (11-58) N=8 | 33 (14-80) N=7 | 9.86 (4.3-23) N=8 |
| 12 months after last immunization | 0.1 (0.01-1.02) | 2 (0.036-110) N=1 | 3.7 (0.81-17) N=7 | 16 (4.39-55) | 2.56 (0.72-9.09) | 15 (3.97-57) | 13 (3.35-48) | 13 (3.05-52) N=8 |
| 12 months after last immunization to Baseline (visit 0) | 8.82 (0.75-104) N=1 | 0.95 (0.081-11) N=1 | 18 (6.41-48) N=6 | 17 (6.51-42) N=7 | 10 (4.61-24) N=9 | 20 (8.35-48) N=8 | 22 (8.81-57) N=7 | 9.54 (3.76-24) N=7 |
| 24 months after last immunization | 0.034 (0.0008-1.39) N=1 | 0.4 (0.029-5.51) | 0.33 (0.039-2.81) N=3 | 13 (3.92-41) | 0.71 (0.14-3.73) N=5 | 24 (4.62-127) N=5 | 11 (2.11-58) N=5 | 6.15 (1.66-23) N=8 |
| 24 months after last immunization to Baseline (visit 0) | 1 (0.092-11) N=1 | 1.5 (0.28-8.1) | 2.45 (0.45-13) N=2 | 14 (5.57-34) N=7 | 6.55 (2.25-19) N=5 | 23 (8.06-68) N=5 | 13 (4.39-37) N=5 | 6.21 (2.52-15) N=7 |

+ two injections

Once again, no significant dose-response or advantage from administering two injections compared to one was observed in this small study.

Safety analysis was assessed based on a number of different criteria. No safety issues stood out, and no dose dependent response was noticeable. Among the reactogenicity indicators, pain on injection site (15 occurrences out of 98 injections for serotype Ib, and 14 occurrences out of 96 injections for serotype III) was the most common complaint in the solicited local reactions for both serotypes, and headache was the one in the solicited systemic reactions, but no obvious differences between placebo and the vaccinated individuals were observed.

### Human study (3)

This study investigated a trivalent GBS serotype Ia, Ib and III capsular saccharide-CRM197 conjugate vaccine in healthy, non-pregnant women. Two different vaccine formulations were studied, each combining the three saccharides in equal proportions. Two different doses (5 $\mu$g and 20 $\mu$g, measured as mass of each saccharide, in 0.5 ml) were tested with and without alum adjuvant. The study also evaluated intramuscular 1- and 2-injection (30 days apart) schedules for each formulation. The vaccine also included 4.5 mg sodium choloride, 0.34 mg potassium dihydrogen phosphate and 7.5 mg mannitol. The study groups are summarised in Table 18 below. A placebo group (two 0.9% saline injections, 30 days apart) with 20 subjects was also tested.

*Table 18: Study groups for trivalent GBS serotype Ia, Ib and III capsular saccharide-CRM197 conjugate vaccine study*

| Variables | 1 injection | | 2 injections | |
|---|---|---|---|---|
| | 5/5/5 $\mu$g | 20/20/20 $\mu$g | 5/5/5 $\mu$g | 20/20/20 $\mu$g |
| No alum | N=40 | N=39 | N=40 | N=40 |
| Alum | N=40 | N=39 | N=40 | N=40 |

Blood was drawn from each subject at screening and a month after the first injection for analysis of immunogenicity by ELISA. The 2-injection groups received the second injection after the blood draw at the one-month timepoint. Blood was also drawn from all groups at 3 months into the study. A summary of the geometric mean concentration of anti-Ia, Ib and III antibodies for each group (adjusted for baseline antibody concentrations and excluding the placebo group) is given in table 19 below.

*Table 19:* Geometric mean concentrations (and geometric mean ratios) for trivalent GBS serotype Ia, Ib and III capsular saccharide-CRM197 conjugate vaccine study.

| | | 5 na | 5+ na | 20 na | 20+ na | 5 adv | 5+ adv | 20 adv | 20+ adv |
|---|---|---|---|---|---|---|---|---|---|
| | | N=40 | N=40 | N=38 | N=39 | N=40 | N=40 | N=39 | N=39 |
| **GBS Ia** | Screening | 0.71 (0.41-1.21) | 0.65 (0.37-1.16) N=35 | 0.48 (0.27-0.85) N=35 | 0.49 (0.28-0.87) N=37 | 0.57 (0.32-1.01) N=36 | 0.71 (0.4-1.27) N=35 | 0.59 (0.33-1.05) N=35 | 0.45 (0.26-0.79) N=37 |
| | Day 31 | 13 (7.06-25) | 12 (5.99-23) | 20 (10-41) | 18 (9.41-36) | 16 (8.02-30) | 8.26 (4.19-16) N=39 | 12 (6.12-24) | 8.88 (4.57-17) |
| | Day 31 to Screening | 23 (12-44) | 20 (10-40) N=35 | 34 (17-66) N=34 | 31 (16-61) N=36 | 27 (14-52) N=36 | 14 (7.32-28) N=35 | 21 (11-41) N=35 | 15 (7.62-28) N=37 |
| | Day 61 | 16 (9.34-28) | 15 (8.63-27) | 18 (10-33) | 23 (13-40) | 18 (10-32) | 12 (6.79-22) | 13 (7.3-23) | 11 (6.01-19) N=38 |
| | Day 61 to Screening | 28 (16-47) | 26 (15-47) N=35 | 31 (17-55) N=34 | 39 (22-69) N=36 | 31 (18-55) N=36 | 20 (11-36) N=35 | 23 (13-40) N=35 | 18 (10-32) N=36 |
| | | N=39 | N=40 | N=38 | N=39 | N=40 | N=40 | N=39 | N=37 |
| **GBS Ib** | Screening | 0.15 (0.091-0.25) N=37 | 0.12 (0.072-0.2) N=34 | 0.1 (0.063-0.17) N=36 | 0.12 (0.072-0.2) N=37 | 0.14 (0.082-0.22) N=37 | 0.11 (0.068-0.19) N=34 | 0.12 (0.074-0.21) N=34 | 0.081 (0.048-0.14) N=34 |
| | Day 31 | 4.92 (2.67-9.06) N=38 | 4.21 (2.24-7.91) N=39 | 4.59 (2.51-8.41) | 4.12 (2.23-7.6) N=35 | 3.94 (2.15-7.23) N=39 | 3.25 (1.73-6.1) N=39 | 3.31 (1.74-6.28) N=37 | 2.87 (1.48-5.6) N=36 |

| | | | | | | | | |
|---|---|---|---|---|---|---|---|---|
| Day 31 to Screening | 45 (24-85) N=36 | 35 (18-69) N=33 | 35 (19-67) N=36 | 33 (17-63) N=35 | 34 (18-64) N=36 | 27 (14-53) N=33 | 30 (15-59) N=32 | 19 (9.43-37) N=31 |
| Day 61 | 5.17 (3.17-8.45) | 5.29 (3.19-8.78) | 4.69 (2.84-7.73) | 5.35 (3.26-8.76) | 4.1 (2.52-6.68) | 4.09 (2.47-6.79) | 3.74 (2.26-6.22) | 3.39 (1.98-5.8) |
| Day 61 to Screening | 47 (29-78) N=37 | 46 (27-77) N=34 | 38 (23-64) N=35 | 45 (27-75) N=36 | 35 (21-59) N=37 | 35 (21-59) N=34 | 33 (20-56) N=34 | 24 (14-41) N=32 |
| | **N=34** | **N=36** | **N=38** | **N=36** | **N=35** | **N=36** | **N=35** | **N=36** |
| Screening | 0.3 (0.16-0.54) | 0.16 (0.088-0.29) N=34 | 0.17 (0.096-0.3) | 0.14 (0.078-0.25) | 0.18 (0.1-0.34) N=34 | 0.38 (0.21-0.69) | 0.15 (0.082-0.27) | 0.24 (0.13-0.43) |
| Day 31 | 7.82 (4.24-14) | 5.48 (3.03-9.91) | 8.13 (4.66-14) N=37 | 8.31 (4.47-15) N=34 | 5.5 (3-10) N=34 | 5.36 (2.88-10) N=34 | 8.51 (4.45-16) N=31 | 6.03 (3.41-11) |
| Day 31 to Screening | 34 (19-63) N=31 | 25 (14-45) N=32 | 36 (21-64) N=36 | 34 (19-62) N=31 | 26 (14-47) N=30 | 24 (14-44) N=32 | 35 (19-66) N=28 | 29 (16-51) N=34 |
| Day 61 | 7.5 (4.43-13) N=33 | 8.71 (5.31-14) | 8.48 (5.26-14) N=37 | 9.59 (5.72-16) N=35 | 5.35 (3.2-8.93) | 7.75 (4.58-13) | 8.23 (4.77-14) N=32 | 7.79 (4.74-13) N=35 |
| Day 61 to Screening | 33 (20-55) N=31 | 43 (26-70) N=34 | 39 (25-64) N=36 | 43 (26-71) N=33 | 26 (15-43) N=31 | 33 (20-54) N=33 | 38 (22-64) N=29 | 38 (23-62) N=33 |

(GBS III)

+ two injections

na – no adjuvant

adv – with adjuvant

The vaccine was immunogenic, inducing in between 80% and 100% of the subjects at least a 2-fold increase in GBS specific antibodies across the different serotypes. A comparison of the GMCs from the eight groups revealed a) no contribution from a second injection compared to a single injection only; b) no contribution from the inclusion of alum adjuvant compared to no adjuvant; and c) no contribution from the higher dose of 20/20/20 μg versus 5/5/5 μg.

More specifically, there was no consistent increase in antibody response among subjects receiving two vaccine injections compared to those receiving only one vaccine injection against any of the GBS serotypes (Ia, Ib or III). This lack of contribution of the second vaccine injection was observed regardless of the dose (5/5/5 or 20/20/20 μg) or the formulation (no alum or alum adjuvant). For GBS Ia, GMC measurements for each of the eight groups ranged from 7 to 20 μg/ml on day 61 of the study. From these results, no contribution of two injections (GMC range [7-16 μg/ml]) was observed compared to one injection (GMC range [9-20 μg/ml] (95% CI all overlapping)). Moreover, the ratio of one vs two injections was 1.2 [95% CI (0.7, 2.0)]. This result indicates practical equivalence of one vs two injections (p-value = 0.5). For GBS Ib, GMC measurements for each of the eight groups ranged from 2-7 μg/ml on day 61 of the study. No contribution of two injections (GMC range [2-5 μg/ml]) was observed compared to one injection (GMC range [4-7 μg/ml] (95% CI all overlapping)). This time, the ratio of one vs two injections was 1.2 [95% CI (0.7, 2.0)]. Once again, this result indicates practical equivalence (p-value = 0.5). For GBS III, measurements for each of the eight groups ranged from 5-13 μg/ml on day 61 of the study. No contribution of two injections (GMC range [5-11 μg/ml]) was observed compared to one injection (GMC range [5-13 μg/ml] (95% CI all overlapping)). The ratio of one vs two injections was 0.94 [95% CI (0.55, 1.66], indicating equivalence (p-value = 0.8).

Similarly, there was no added contribution to GMC from the inclusion of alum compared to no alum. This lack of contribution of alum adjuvant was observed regardless of the dose (5/5/5 or 20/20/20 μg) or the injection number and was seen across all three serotypes (Ia, Ib and III). For GBS Ia, the GMC across the eight groups ranged from 7-20 μg/ml on day 61 of the study and showed no contribution of alum (GMC range [7-15 μg/ml] compared to no alum (GMC range [13-16 μg/ml] (95% CI all overlapping)). The ratio of group GMC for the no alum group compared to the alum group was 1.6 [95% CI (0.9, 2.6)], which suggests that the response without alum is potentially higher relative to the vaccine formulation with alum (p-value = 0.11). For GBS Ib, GMC ranged from 2-7 μg/ml on day 61 of the study and showed no contribution of alum (GMC range [2-4 μg/ml] compared to no alum (GMC range [4-7 μg/ml] (95% CI all overlapping)). The ratio of group GMC for the no alum group compared to the alum group was 1.4 [95% CI (0.8, 2.4)] implying near equivalence in GMC values (p-value = 0.2). For GBS III, GMC ranged from 5-13 μg/ml on day 61 of the study and showed no contribution of alum (GMC range [5-11 μg/ml] compared to no alum (GMC range [5-13 μg/ml] (95% CI all overlapping)). The ratio of group GMC for the no alum group compared to the alum group was 1.09 [95% CI (0.6, 1.9)] implying near equivalence in GMC values (p-value = 0.7).

Finally, the data allows an evaluation of the two doses (5 vs 20 μg of each of the three saccharides in the conjugates). The results suggest that the higher dose (20 μg) does not induce a higher antibody response. In particular, the ratios of GMC for subjects receiving 5 μg (across all groups) and subjects receiving 20 μg (across all groups) are 1.2 [95% CI (0.7, 2.1)] for GBS Ia; 0.7 [95% CI (0.4, 1.2)] for GBS Ib and 1.4 [95% CI (0.9, 2.5)] for GBS III. These ratios are close to 1 and the p-values of the statistical test for equality to 1, are > 0.15 for all three serotypes, suggesting no discernable differences in the level of induced antibodies between the two dose regimens.

Safety was measured by the incidence of local and systemic reactogenicity, adverse events and serious adverse events, as well as clinical laboratory results. The trivalent GBS vaccine was found safe and well tolerated in all of the eight vaccine study groups when compared to placebo. Safety was evaluated by: percentages of subjects with solicited local (*i.e* injection site pain, ecchymosis, erythema, induration, and swelling) and solicited systemic (*i.e.* chills, nausea, malaise, myalgia, headache, fatigue, arthralgia, rash, fever [defined as axillary temperature ≥ 38 °C], and other) reactions occurring during the 7 days following each vaccination together with severity of reactions; all of other adverse events reported from day 1 to day 23 after each vaccination; percentages of subjects with reported serious adverse events and/or adverse events resulting in withdrawal from the study, per vaccine group for up to Day 61.

### *Human study (4)*

The responses of subjects with antibody (Ab) levels below detection at study entry (0.4, 0.084 and 0.068 μg/ml for serotypes Ia, Ib and III respectively) were of particular interest. This subset analysis was carried out on the data from *Human study (3)* above. For each serotype, data were assessed as:

(a) GMC for each injection/formulation/dose group, and the corresponding 95% CI

(b) GMC over all subjects receiving (i) 1 injection regardless of a group assignment and this was compared to the GMC of all subjects receiving 2 injections. Similarly, the GMC of subjects receiving no adjuvant compared to the GMC of subjects receiving alum, as well as GMC receiving 5/5/5 μg dose compared to the GMC of all subjects receiving 20/20/20 μg. The assessment was based on the ratio of GMC, together with the two-sided 95% CI around the calculated ratio.

(c) Proportion of subjects with at least 4-fold change from baseline, assumed as half the lowest level of detection (lld)

In general, approximately 25% and 50% of women presented with Ab levels below the limit of detection for serotypes III and Ia/Ib respectively. The percentage of subjects in this subset achieving ≥4-fold increase in Ab level at day 61 compared to baseline (where baseline value is assigned half lld) range from 64-95% (serotype Ia), 80-100% (serotype III) and 81-100% (serotype Ib).

Similarly to results from the full study cohort, subjects with undetectable Ab levels at study entry also fail to show additional benefit from 2 injections (vs 1 injection), from a higher dosage (vs lower dosage) or from inclusion of alum (vs no adjuvant). The ratio of GMC (on day 61) for all 1 injection vs all 2 injection subjects was 1.1 (0.6-1.8; serotype Ia), 0.7 (0.3-1.5; serotype III) and 0.9 (0.5-1.4; serotype Ib); for all 5 μg vs. all 20 μg dosage subjects was 1.3 (0.8-2.1; serotype Ia), 1.4 (0.7-2.8; serotype III) and 1.4 (0.9-2.3; serotype Ib); for all no adjuvant subjects vs all alum subjects was 1.4 (0.8-2.4; serotype Ia), 1 (0.5-2.0; serotype III) and 1.7 (1.1-2.7; serotype Ib)

### *Mouse study (15)*

Mice were primed with CRM197 and aluminium hydroxide adjuvant or aluminium hydroxide adjuvant alone at day 0 and then immunized with a GBS serotype III/CRM197 conjugate with or without the adjuvant aluminium hydroxide adjuvant at days 21 and 35. Blood was drawn on day 0 and before vaccination on days 21 and 35. IgG/IgM serum titers to the GBS serotype III polysaccharide and CRM197 carrier protein were measured from the blood samples.

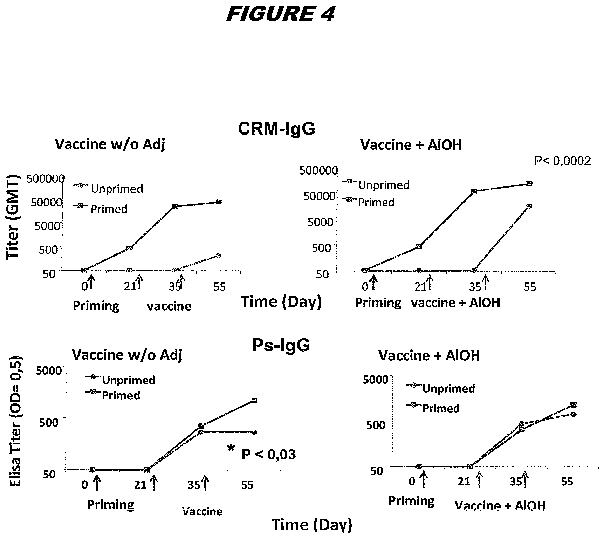

As shown in Figure 4, priming with the CRM197 carrier resulted in a significantly higher IgG antibody response to the carrier after one and two doses of the vaccine (with or without adjuvant) compared to unprimed mice (P< 0.0002). Priming also resulted in a good antibody response against the GBS serotype III polysaccharide after two doses of vaccine (with or without adjuvant). Unprimed mice required the adjuvant in order to reach an anti-polysaccharide antibody titer comparable to that observed in primed mice. In unprimed mice, when the glycoconjugate vaccine was administered without adjuvant, the antibody titer was significantly lower than in the other groups (P< 0.03).

Priming with CRM197 therefore seems to have a positive influence on the subsequent antibody response to the GBS capsular saccharide component of the conjugate, even when administered without an adjuvant.

### *Rat and rabbit studies*

Studies to assess potential reproductive and developmental toxicity of the trivalent GBS serotype Ia, Ib and III capsular saccharide-CRM197 conjugate vaccine were carried out in rats and rabbits.

The rat study was carried out according to table 20 below:

*Table 20: Rat study*

| Treatment | Dose each antigen± (μg) | SC dose volume (mL) | Dosing days relative to mating on Day 0 | Number of animals | |
|---|---|---|---|---|---|
| | | | | C section (gestation day 21) | Natural delivery |
| Control (saline) | 0/0/0 | 0.5 | -35, -21, -7, 6, 12, 17 | 24 | 24 |
| GBS vaccine | 20/20/20 | 0.5 | | 24 | 24 |
| GBS vaccine | 20/20/20 | 0.5 | 6, 12, 17 | 24 | 24 |
| GBS vaccine + alum* | 20/20/20 | 0.5 | -35, -21, -7, 6, 12, 17 | 24 | 24 |
| GBS vaccine + alum* | 20/20/20 | 0.5 | 6, 12, 17 | 24 | 24 |
| ± to serotype Ia/Ib/III<br>* aluminum hydroxide, 2 mg/mL | | | | | |

Subcutaneous administration of the trivalent vaccine to female rats on study days 1, 15, 29 (premating period) and/or on gestation days 6, 12 and 17 at a dose of 20 μg with or without aluminum hydroxide resulted in no maternal toxicity or effects on reproductive function or embryofetal development. No differences were noted between groups treated with three or six injections of the trivalent vaccine with or without aluminum hydroxide adjuvant. Additionally, there was no effect on the F1 generation pup survival, clinical condition or body weight or reproductive ability.

The rabbit study was carried out according to table 21 below:

*Table 21: Rabbit study*

| Treatment | Dose each antigen± (μg) | IM dose volume (mL) | Dosing days relative to mating on Day 0 | Number of animals | |
|---|---|---|---|---|---|
| | | | | C section (gestation day 29) | Natural delivery |
| Control (saline) | 0/0/0 | 0.5 | -35, -21, -7, 7, 20 | 23 | 25 |
| GBS vaccine + alum* | 20/20/20 | 0.5 | -35, -21, -7, 7, 20 | 23 | 25 |

# EP 2 616 099 B1

(continued)

| Treatment | Dose each antigen± (μg) | IM dose volume (mL) | Dosing days relative to mating on Day 0 | Number of animals | |
|---|---|---|---|---|---|
| | | | | C section (gestation day 29) | Natural delivery |
| GBS vaccine + alum | 20/20/20 | 0.5 | 7, 20 | 23 | 25 |
| ± to serotype Ia/Ib/III<br>* aluminum hydroxide, 2 mg/mL | | | | | |

Intramuscular administration of the trivalent vaccine plus aluminum hydroxide to female rabbits, at a dose of 20 μg on study days 1, 15 and 29 (premating period) and/or on gestation days 7 and 20, resulted in neither maternal toxicity, effects on mating nor evidence of embryolethality, fetotoxicity or teratogenicity. There were no differences between the adult F1 generation of control and vaccine-treated does.

These studies showed that the trivalent vaccine was immunogenic and did not have any prenatal or postnatal effects on pregnant rats or rabbits or their offspring.

## Stabilty study

The stability of the trivalent GBS serotype Ia, Ib and III capsular saccharide-CRM197 conjugate vaccine was measured during 1 month of storage at two different temperatures. The vaccine was formulated by pooling the three glycoconjugates, each one present at 80 μg saccharide/ml in 10 mM $KH_2PO_4$ and 3% mannitol. 3-ml single dose vials were filled with 0.3 ml of solution, partially capped with bromobuthyl siliconized rubber stopper and submitted to a freeze-drying cycle. Once the lyophilization process was over, the vials were stored at 2-8 °C or 36-38 °C. A slight increase in free saccharide content was detected (using HPAEC-PAD) upon storage at 36-38°C. However, overall the trivalent vaccine was stable upon storage up to one month at both 2-8 °C and at 36-38°C.

It will be understood that the invention has been described by way of example only and modifications may be made.

## REFERENCES

[1] Paoletti et al. (1990) J Biol Chem 265:18278-83.
[2] Wessels et al. (1990) J Clin Invest 86:1428-33.
[3] Paoletti et al. (1992) Infect Immun 60:4009-14.
[4] Paoletti et al. (1992) J Clin Invest 89:203-9.
[5] Wessels et al. (1987) Proc Natl Acad Sci USA 84:9170-4.
[6] Wang et al. (2003) Vaccine 21:1112-7.
[7] Wessels et al. (1993) Infect Immun 61:4760-6
[8] Wessels et al. (1995) J Infect Dis 171:879-84.
[9] Baker et al. (2004) J Infect Dis 189:1103-12.
[10] Paoletti & Kasper (2003) Expert Opin Biol Ther 3:975-84.
[11] Brigtsen et al. (2002) JID, 185 :1277-84.

[13] WO2006/050341
[14] Guttormsen et al. (2008) Proc Natl Acad Sci U S A. 105(15):5903-8. Epub 2008 Mar 31.
[15] WO96/40795
[16] Michon et al. (2006) Clin Vaccine Immunol. 2006 Aug;13(8):936-43.
[17] US patents 6027733 & 6274144.
[18] www.polymer.de
[19] Lewis et al. (2004) PNAS USA 101:11123-8.
[20] Wessels et al. (1989) Infect Immun 57:1089-94.
[21] WO2006/082527.
[22] US patent application US 61/008,941, entitled "FERMENTATION PROCESSES FOR CULTIVATING STREPTOCOCCI AND PURIFICATION PROCESSES FOR OBTAINING CPS THEREFROM" filed on 20th December 2007 and international patent application WO 2009/081276.

[23] Ramsay et al. (2001) Lancet 357(9251):195-196.

[24] Lindberg (1999) Vaccine 17 Suppl 2: S28-36.

[25] Buttery & Moxon (2000) J R Coll Physicians Lond 34:163-68.

[26] Ahmad & Chapnick (1999) Infect Dis Clin North Am 13:113-33, vii.

[27] Goldblatt (1998) J. Med. Microbiol. 47:563-7.

[28] European patent 0477508.

[29] US patent 5,306,492.

[30] WO98/42721.

[31] Dick et al. in Conjugate Vaccines (eds. Cruse et al.) Karger, Basel, 1989, 10:48-114.

[32] Hermanson Bioconjugae Techniques, Academic Press, San Diego (1996) ISBN: 0123423368.

[33] US patent 4356170.

[34] WO2006/082530.

[35] WO2005/000346

[36] Anonymous (Jan 2002) Research Disclosure, 453077.

[37] Anderson (1983) Infect Immun 39(1):233-238.

[38] Anderson et al. (1985) J Clin Invest 76(1):52-59.

[39] EP-A-0372501.

[40] EP-A-0378881.

[41] EP-A-0427347.

[42] WO93/17712

[43] WO94/03208.

[44] WO98/58668.

[45] EP-A-0471177.

[46] WO91/01146

[47] Falugi et al. (2001) Eur J Immunol 31:3816-24.

[48] Baraldo et al. (2004) Infect Immun 72:4884-87.

[49] EP-A-0594610.

[50] WO00/56360.

[51] WO02/091998.

[52] Kuo et al. (1995) Infect Immun 63:2706-13.

[53] WO01/72337

[54] WO00/61761.

[55] WO00/33882

[56] WO99/42130.

[57] WO2004/011027.

[58] WO96/40242.

[59] Lei et al. (2000) Dev Biol (Basel) 103:259-264.

[60] WO00/38711; US patent 6,146,902.

[61] International patent application PCT/IB2008/02690, 'CONJUGATE PURIFICATION', claiming priority from GB-0713880.3 (NOVARTIS AG), published as WO 2009/010877.

[62] WO99/24578.

[63] WO99/36544.

[64] WO99/57280.

[65] WO00/22430.

[66] Tettelin et al. (2000) Science 287:1809-1815.

[67] WO96/29412.

[68] Pizza et al. (2000) Science 287:1816-1820.

[69] WO01/52885.

[70] Bjune et al. (1991) Lancet 338(8775):1093-1096.

[71] Fukasawa et al. (1999) Vaccine 17:2951-2958.

[72] Rosenqvist et al. (1998) Dev. Biol. Stand. 92:323-333.

[73] Costantino et al. (1992) Vaccine 10:691-698.

[74] WO03/007985.

[75] Watson (2000) Pediatr Infect Dis J 19:331-332.

[76] Rubin (2000) Pediatr Clin North Am 47:269-285, v.

[77] Jedrzejas (2001) Microbiol Mol Biol Rev 65:187-207.

[78] Bell (2000) Pediatr Infect Dis J 19:1187-1188.

[79] Iwarson (1995) APMIS 103:321-326.

[80] Gerlich et al. (1990) Vaccine 8 Suppl:S63-68 & 79-80.

[81] Hsu et al. (1999) Clin Liver Dis 3:901-915.

[82] Gustafsson et al. (1996) N. Engl. J. Med. 334:349-355.

[83] Rappuoli et al. (1991) TIBTECH 9:232-238.

[84] Vaccines (2004) eds. Plotkin & Orenstein. ISBN 0-7216-9688-0.

[85] WO02/02606.

[86] Kalman et al. (1999) Nature Genetics 21:385-389.

[87] Read et al. (2000) Nucleic Acids Res 28:1397-406.

[88] Shirai et al. (2000) J. Infect. Dis. 181(Suppl 3):S524-S527.

[89] WO99/27105.

[90] WO00/27994.

[91] WO00/37494.

[92] WO99/28475.

[93] Ross et al. (2001) Vaccine 19:4135-4142.

[94] Sutter et al. (2000) Pediatr Clin North Am 47:287-308.

[95] Zimmerman & Spann (1999) Am Fam Physician 59:113-118, 125-126.

[96] Dreesen (1997) Vaccine 15 Suppl:S2-6.

[97] MMWR Morb Mortal Wkly Rep 1998 Jan 16;47(1):12, 19.

[98] McMichael (2000) Vaccine 19 Suppl 1:5101-107.

[99] WO02/34771.

[100] Dale (1999) Infect Dis Clin North Am 13:227-43, viii.

[101] Ferretti et al. (2001) PNAS USA 98: 4658-4663.

[102] Kuroda et al. (2001) Lancet 357(9264):1225-1240; see also pages 1218-1219.

[103] Robinson & Torres (1997) Seminars in Immunology 9:271-283.

[104] Donnelly et al. (1997) Annu Rev Immunol 15:617-648.

[105] Scott-Taylor & Dalgleish (2000) Expert Opin Investing Drugs 9:471-480.

[106] Apostolopoulos & Plebanski (2000) Curr Opin Mol Ther 2:441-447.

[107] Ilan (1999) Curr Opin Mol Ther 1:116-120.

[108] Dubensky et al. (2000) Mol Med 6:723-732.

[109] Robinson & Pertmer (2000) Adv Virus Res 55:1-74.

[110] Donnelly et al. (2000) Am T Respir Crit Care Med 162(4 Pt 2):S190-193.

[111] Davis (1999) Mt. Sinai J. Med. 66:84-90.

[112] Paoletti et al. (2001) Vaccine 19:2118-2126.

[113] WO00/56365.

[114] Gennaro (2000) Remington: The Science and Practice of Pharmacy. 20th edition, ISBN: 0683306472.

[115] Paoletti (2001) Vaccine 19(15-16):2118-26.

[116] Almeida & Alpar (1996) J. Drug Targeting 3:455-467.

[117] Agarwal & Mishra (1999) Indian J Exp Biol 37:6-16.

[118] WO00/53221.

[119] Jakobsen et al. (2002) Infect Immun 70:1443-1452.

[120] Bergquist et al. (1998) APMIS 106:800-806.

[121] Baudner et al. (2002) Infect Immun 70:4785-4790.

[122] Ugozzoli et al. (2002) J Infect Dis 186:1358-1361.

[123] US patent 6355271.

[124] WO00/23105.

[125] WO90/14837.

[126] Podda (2001) Vaccine 19:2673-80.

[127] Frey et al. (2003) Vaccine 21:4234-7.

[128] US Patent 6,299,884.

[129] US Patent 6,451,325.

[130] US patent 5,057,540.

[131] WO96/33739.

[132] EP-A-0109942.

[133] WO96/11711.

[134] WO00/07621.

[135] Barr et al. (1998) Advanced Drug Delivery Reviews 32:247-271.

[136] Sjolanderet et al. (1998) Advanced Drug Delivery Reviews 32:321-338.

[137] Niikura et al. (2002) Virology 293:273-280.

[138] Lenz et al. (2001) J Immunol 166:5346-5355.

[139] Pinto et al. (2003) J Infect Dis 188:327-338.

[140] Gerber et al. (2001) Virol 75:4752-4760.

[141] WO03/024480

[142] WO03/024481

[143] Gluck et al. (2002) Vaccine 20:B10-B16.

[144] EP-A-0689454.

[145] Johnson et al. (1999) Bioorg Med Chem Lett 9:2273-2278.

[146] Evans et al. (2003) Expert Rev Vaccines 2:219-229.

[147] Meraldi et al. (2003) Vaccine 21:2485-2491.

[148] Pajak et al. (2003) Vaccine 21:836-842.

[149] Kandimalla et al. (2003) Nucleic Acids Research 31:2393-2400.

[150] WO02/26757.

[151] WO99/62923.

[152] Krieg (2003) Nature Medicine 9:831-835.

[153] McCluskie et al. (2002) FEMS Immunology and Medical Microbiology 32:179-185.

[154] WO98/40100.

[155] US patent 6,207,646.

[156] US patent 6,239,116.

[157] US patent 6,429,199.

[158] Kandimalla et al. (2003) Biochemical Society Transactions 31 (part 3):654-658.

[159] Blackwell et al. (2003) J Immunol 170:4061-4068.

[160] Krieg (2002) Treads Immunol 23:64-65.

[161] WO01/95935.

[162] Kandimalla et al. (2003) BBRC 306:948-953.

[163] Bhagat et al. (2003) BBRC 300:853-861.

[164] WO03/035836.

[165] WO95/17211.

[166] WO98/42375.

[167] Beignon et al. (2002) Infect Immun 70:3012-3019.

[168] Pizza et al. (2001) Vaccine 19:2534-2541.

[169] Pizza et al. (2000) Int J Med Microbiol 290:455-461.

[170] Scharton-Kersten et al. (2000) Infect Immun 68:5306-5313.

[171] Ryan et al. (1999) Infect Immun 67:6270-6280.

[172] Partidos et al. (1999) Immunol Lett 67:209-216.

[173] Peppoloni et al. (2003) Expert Rev Vaccines 2:285-293.

[174] Pine et al. (2002) J Control Release 85:263-270.

[175] Domenighini et al. (1995) Mol Microbiol 15:1165-1167.

[176] WO99/40936.

[177] WO99/44636.

[178] Singh et al] (2001) J Cont Release 70:267-276.

[179] WO99/27960.

[180] US patent 6,090,406

[181] US patent 5,916,588

[182] EP-A-0626169.

[183] WO99/52549.

[184] WO01/21207.

[185] WO01/21152.

[186] Andrianov et al. (1998) Biomaterials 19:109-115.

[187] Payne et al. (1998) Adv Drug Delivery Review 31:185-196.

[188] Stanley (2002) Clin Exp Dermatol 27:571-577.

[189] Jones (2003) Curr Opin Investing Drugs 4:214-218.

[190] WO04/60308

[191] WO04/64759.

[192] WO99/11241.

[193] WO94/00153.

[194] WO98/57659.

[195] European patent applications 0835318, 0735898 and 0761231.

[196] Hennings et al. (2001) J Infect Dis. 183(7):1138-42. Epub 2001 Mar 1.

[197] Lin et al. (2001) J Infect Dis. 184(8):1022-8.

[198] Lin et al. (2004) J Infect Dis. 190(5):928-34

[199] Glezen & Alpers (1999) Clin. Infect. Dis. 28:219-224

[200] Madoff et al. (1994) J Clin Invest 94:286-92.

[201] Paoletti et al. (1994) Infect Immun 62:3236-43.

[202] WO03/093306.

[203] WO2004/018646.

[204] WO2004/041157.

[205] Adderson et al. (2003) Infection and Immunity 71(12):6857-6863.

[206] Geysen et al. (1984) PNAS USA 81:3998-4002.

[207] Carter (1994) Methods Mol Biol 36:207-23.

[208] Jameson, BA et al. 1988, CABIOS 4(1):181-186.

[209] Raddrizzani & Hammer (2000) Brief Bioinform 1(2):179-89.

[210] De Lalla et al. (1999) J. Immunol. 163:1725-29.

[211] Brusic et al. (1998) Bioinformatics 14(2):121-30

[212] Meister et al. (1995) Vaccine 13(6):581-91.

[213] Roberts et al. (1996) AIDS Res Hum Retroviruses 12(7):593-610.

[214] Maksyutov & Zagrebelnaya (1993) Comput Appl Biosci 9(3):291-7.

[215] Feller & de la Cruz (1991) Nature 349(6311):720-1.

[216] Hopp (1993) Peptide Research 6:183-190.

[217] Welling et al. (1985) FEBS Lett. 188:215-218.

[218] Davenport et al. (1995) Immunogenetics 42:392-297.

[219] Bodanszky (1993) Principles of Peptide Synthesis (ISBN: 0387564314).

[220] Fields et al. (1997) Meth Enzymol 289: Solid-Phase Peptide Synthesis. ISBN: 0121821900.

[221] Chan & White (2000) Fmoc Solid Phase Peptide Synthesis. ISBN: 0199637245.

[222] Kullmann (1987) Enzymatic Peptide Synthesis. ISBN: 0849368413.

[223] Ibba (1996) Biotechnol Genet Eng Rev 13:197-216.

[224] WO2006/069200

[225] UK patent application 0802503.3, entitled "HYBRID POLYPEPTIDE" filed on 11th February 2008, and international patent application WO2009/101403.

[226] Needleman & Wunsch (1970) J. Mol. Biol. 48, 443-453.

[227] Rice et al. (2000) Trends Genet 16:276-277.

[228] Herzenberg et al. (1980) Nature 285: 664-667.

[229] Schutze et al. (1985) J Immunol 135:2319-2322.

[230] Dagan et al. (1998) Infect Immun 66:2093-2098.

[231] Barington et al. (1994) Infect Immun 62:9-14.

[232] Di John et al. (1989) Lancet 2(8677):1415-8.

[233] Granoff et al. (1993) Vaccine Suppl1: S46-51.

[234] Granoff et al. (1994) JAMA 272:1116-1121.

[235] Barington et al. (1993) Infect Immun 61:432-438.

[236] Australian patent 748716 (granted from WO98/51339).

[237] Olander et al. (2001) Vaccine 20:336-341.

[238] Burrage et al. (2002) Infect Immun 70:4946-4954.

[239] Hoppenbrouwers et al. (1999) Vaccine 17:2588-98.

[240] Podda et al. (1991) Vaccine 9:741-745.

[241] Vaccines. (eds. Plotkin & Orenstein). 4th edition, 2004, ISBN: 0-7216-9688-0

[242] Darkes & Plosker (2002) Paediatr Drugs 4:609-630.

[243] Jones (2001) Curr Opin Investing Drugs 2:47-49

[244] Del Guidice et al. (1998) Molecular Aspects of Medicine 19:1-70.

[245] Anonymous (Jan 2002) Research Disclosure, 453077.

[246] Anderson (1983) Infect Immun 39(1):233-238.

[247] Anderson et al. (1985) J Clin Invest 76(1):52-59.

[248] Rodewald et al. (1992) J Infect Dis. 166(3):635-9.

[249] Baltimore et al. (1977) J Immunol. 118(2):673-8

[250] Lin et al. (2001) J Infect Dis. 184(8):1022-8.

[253] Lancaster et al. (2009), Meningitis and Septicaemia in Children and Adults, Royal Society of Medicine, London, www.meningitis.org/conference#sthash.6RAnxqST.dpuf

### Claims

1. An immunogenic composition comprising: a) a conjugate that is a capsular saccharide from Group B *Streptococcus* serotype Ia conjugated to a carrier protein; b) a conjugate that is a capsular saccharide from Group B *Streptococcus* serotype Ib conjugated to a carrier protein; and c) a conjugate that is a capsular saccharide from Group B *Streptococcus* serotype III conjugated to a carrier protein, wherein (i) each GBS capsular saccharide is present at an amount of about 5 µg, 10 µg or 20 µg per unit dose, (ii) the carrier protein in a), b) and c) is diphtheria toxoid or CRM 197 and (iii) the immunogenic composition does not contain an aluminium salt adjuvant.

2. The immunogenic composition according to claim 1, wherein the amounts of the Group B *Streptococcus* serotype Ia, Ib and III capsular saccharides per unit dose are about 5µg, 5µg and 5µg.

3. The immunogenic composition according to claim 1 or 2, wherein the ratio of the masses of the Group B *Streptococcus* serotype Ia, Ib and III capsular saccharides is 1:1:1.

4. The immunogenic composition according to any one of claims 1 to 3 further comprising:
   d) a conjugate that is a capsular saccharide from Group B *Streptococcus* serotype V conjugated to a carrier protein.

5. The immunogenic composition according to any one of claims 1 to 4, wherein the composition is for administration in a single dose.

6. The immunogenic composition according to any one of the preceding claims, wherein the conjugate(s) are obtainable by reductive amination of aldehyde groups generated before conjugation by oxidation of between 10 and 30% of the saccharides' sialic acid residues

7. The immunogenic composition according to any one of the preceding claims wherein the Group B *Streptococcus* capsular saccharide(s) have substantially no O-acetylation of sialic acid residues at positions 7, 8 and/or 9.

8. The immunogenic composition according to any one of the preceding claims, wherein the composition is for administration intramuscularly.

9. The immunogenic composition according to any one of the preceding claims, wherein the composition further comprises: (a) a polypeptide comprising an amino acid sequence selected from SEQ ID NOs 1 to 3, and/or (b) a polypeptide comprising (i) an amino acid sequence that has 90% sequence identity or more to one or more of SEQ ID NOs 1 to 3 and/or (ii) a fragment of SEQ ID NOs 1 to 3.

10. The immunogenic composition according to any one of the preceding claims, wherein the composition comprises mannitol to stabilise the conjugate(s).

11. The immunogenic composition according to any one of the preceding claims for use as a medicament.

12. The immunogenic composition according to claims 1-10 or for use according to claim 11, wherein the composition is a vaccine for use in the prevention and/or treatment of a disease caused by *S.agalactiae.*

13. The immunogenic composition according to claims 1-10 or for use according to claims 11 or 12, wherein the composition is for administration to humans selected from females of child-bearing age, pregnant females and elderly patients.

14. The immunogenic composition according to claims 1-10 or for use according to claims 11-13, wherein the composition is for administration to a patient that has been pre-immunised with a diphtheria toxoid or derivative thereof.

**Patentansprüche**

1. Immunogene Zusammensetzung, umfassend: a) ein Konjugat, das ein Kapselsaccharid von Gruppe *B-Streptococcus* Serotyp Ia, konjugiert an ein Trägerprotein, ist; b) ein Konjugat, das ein Kapselsaccharid von Gruppe B-*Streptococcus* Serotyp Ib, konjugiert an ein Trägerprotein, ist; und c) ein Konjugat, das ein Kapselsaccharid von Gruppe *B-Streptococcus* Serotyp III, konjugiert an ein Trägerprotein, ist, wobei (i) jedes GBS-Kapselsaccharid in einer Menge von etwa 5 µg, 10 µg oder 20 µg pro Einheitsdosis vorliegt, (ii) das Trägerprotein in a), b) und c) Diphtherietoxoid oder CRM 197 ist, und (iii) die immunogene Zusammensetzung kein Aluminiumsalzadjuvans enthält.

2. Immunogene Zusammensetzung gemäß Anspruch 1, wobei die Mengen der Gruppe *B-Streptococcus* Serotyp Ia-, Ib- und III-Kapselsaccharide pro Einheitsdosis etwa 5 µg, 5 µg und 5 µg sind.

3. Immunogene Zusammensetzung gemäß Anspruch 1 oder 2, wobei das Verhältnis der Massen der Gruppe B-*Streptococcus* Serotyp Ia-, Ib- und III-Kapselsaccharide 1:1:1 ist.

4. Immunogene Zusammensetzung gemäß irgendeinem der Ansprüche 1 bis 3, weiterhin umfassend: d) ein Konjugat, das ein Kapselsaccharid von Gruppe *B-Streptococcus* Serotyp V, konjugiert an ein Trägerprotein, ist.

5. Immunogene Zusammensetzung gemäß irgendeinem der Ansprüche 1 bis 4, wobei die Zusammensetzung zur Verabreichung in einer einzelnen Dosis ist.

6. Immunogene Zusammensetzung gemäß irgendeinem der vorangegangenen Ansprüche, wobei das/die Konjugat(e) erhältlich ist/sind durch reduktive Aminierung von Aldehydgruppen, erzeugt vor der Konjugation durch Oxidation von zwischen 10 und 30% der Sialinsäurereste der Saccharide.

7. Immunogene Zusammensetzung gemäß irgendeinem der vorangegangenen Ansprüche, wobei das/die Gruppe B-Streptococcus-Kapselsaccharid(e) im Wesentlichen keine 0-Acetylierung von Sialinsäureresten an den Positionen 7, 8 und/oder 9 aufweist/aufweisen.

8. Immunogene Zusammensetzung gemäß irgendeinem der vorangegangenen Ansprüche, wobei die Zusammensetzung für die intramuskuläre Verabreichung ist.

9. Immunogene Zusammensetzung gemäß irgendeinem der vorangegangenen Ansprüche, wobei die Zusammensetzung weiterhin umfasst: (a) ein Polypeptid, das eine Aminosäuresequenz, ausgewählt aus SEQ ID NOs: 1 bis 3, umfasst, und/oder (b) ein Polypeptid, das (i) eine Aminosäuresequenz mit 90% Sequenzidentität oder mehr zu einer oder mehreren von SEQ ID NOs: 1 bis 3 und/oder (ii) ein Fragment von SEQ ID NOs: 1 bis 3 umfasst.

10. Immunogene Zusammensetzung gemäß irgendeinem der vorangegangenen Ansprüche, wobei die Zusammensetzung Mannitol umfasst, um das/die Konjugat(e) zu stabilisieren.

11. Immunogene Zusammensetzung gemäß irgendeinem der vorangegangenen Ansprüche zur Verwendung als ein Medikament.

12. Immunogene Zusammensetzung gemäß den Ansprüchen 1-10 oder zur Verwendung gemäß Anspruch 11, wobei die Zusammensetzung ein Impfstoff zur Verwendung bei der Prävention und/oder Behandlung einer durch *S. agalactiae* verursachten Erkrankung ist.

13. Immunogene Zusammensetzung gemäß den Ansprüchen 1-10 oder zur Verwendung gemäß den Ansprüchen 11 oder 12, wobei die Zusammensetzung für die Verabreichung an Menschen, ausgewählt aus Frauen im gebärfähigen Alter, schwangeren Frauen und älteren Patienten, ist.

14. Immunogene Zusammensetzung gemäß den Ansprüchen 1-10 oder zur Verwendung gemäß den Ansprüchen 11-13, wobei die Zusammensetzung für die Verabreichung an einen Patienten ist, der mit einem Diphtherietoxoid oder Derivat davon vorimmunisiert wurde.

**Revendications**

1. Composition immunogène comprenant : a) un conjugué qui est un saccharide capsulaire de *Streptococcus* du groupe B de sérotype Ia conjugué à une protéine de support ; b) un conjugué qui est un saccharide capsulaire de *Streptococcus* du groupe B de sérotype Ib conjugué à une protéine de support ; et c) un conjugué qui est un saccharide capsulaire de *Streptococcus* du groupe B de sérotype III conjugué à une protéine de support, dans laquelle (i) chaque saccharide capsulaire de GBS est présent dans une quantité d'environ 5 $\mu$g, 10 $\mu$g ou 20 $\mu$g par dose unitaire, (ii) la protéine de support dans a), b) et c) est l'anatoxine diphtérique ou le CRM 197 et (iii) la composition immunogène ne contient pas un adjuvant de sel d'aluminium.

2. Composition immunogène selon la revendication 1, dans laquelle les quantités des saccharides capsulaires de *Streptococcus* du groupe B de sérotype Ia, Ib et III par dose unitaire sont d'environ 5 $\mu$g, 5 $\mu$g et 5 $\mu$g.

3. Composition immunogène selon la revendication 1 ou 2, dans laquelle le rapport des masses des saccharides capsulaires de *Streptococcus* du groupe B de sérotype Ia, Ib et III est de 1:1:1.

4. Composition immunogène selon l'une quelconque des revendications 1 à 3 comprenant en outre d) un conjugué qui est un saccharide capsulaire de *Streptococcus* du groupe B de sérotype V conjugué à une protéine de support.

**5.** Composition immunogène selon l'une quelconque des revendications 1 à 4, dans laquelle la composition est pour une administration en une dose unique.

**6.** Composition immunogène selon l'une quelconque des revendications précédentes, dans laquelle le ou les conjugué(s) peuvent être obtenus par amination réductrice des groupes aldéhyde générés avant la conjugaison par oxydation de 10 à 30 % des résidus d'acide sialique des saccharides.

**7.** Composition immunogène selon l'une quelconque des revendications précédentes dans laquelle le ou les saccharides capsulaires de *Streptococcus* du groupe B ne présentent sensiblement aucune 0-acylation des résidus d'acide sialique aux positions 7, 8 et/ou 9.

**8.** Composition immunogène selon l'une quelconque des revendications précédentes, dans laquelle la composition est pour une administration par voie intramusculaire.

**9.** Composition immunogène selon l'une quelconque des revendications précédentes, dans laquelle la composition comprend en outre : (a) un polypeptide comprenant une séquence d'acides aminés choisie parmi SEQ ID NO : 1 à 3, et/ou (b) un polypeptide comprenant (i) une séquence d'acides aminés qui présente 90 % d'identité de séquence ou plus avec une ou plusieurs de SEQ ID NO : 1 à 3 et/ou (ii) un fragment de SEQ ID NO : 1 à 3.

**10.** Composition immunogène selon l'une quelconque des revendications précédentes, dans laquelle la composition comprend du mannitol pour stabiliser le ou les conjugué(s).

**11.** Composition immunogène selon l'une quelconque des revendications précédentes pour une utilisation en tant que médicament.

**12.** Composition immunogène selon les revendications 1 à 10 ou pour une utilisation selon la revendication 11, dans laquelle la composition est un vaccin pour une utilisation dans la prévention et/ou le traitement d'une maladie provoquée par *S. agalactiae.*

**13.** Composition immunogène selon les revendications 1 à 10 ou pour une utilisation selon les revendications 11 ou 12, dans laquelle la composition est pour une administration à des êtres humains choisis parmi des femmes en âge de procréer, des femmes enceintes et des patients âgés.

**14.** Composition immunogène selon les revendications 1 à 10 ou pour une utilisation selon les revendications 11 à 13, dans laquelle la composition est pour une administration à un patient qui a été pré-immunisé avec une anatoxine diphtérique ou l'un de ses dérivés.

## FIGURE 1

**Ia**

$$[\boxed{\rightarrow 4)}\text{-}\beta\text{-Gal-}(1\rightarrow 4)\text{-}\beta\text{-Glc-}(1\rightarrow]_n$$

$$\begin{array}{c} 3 \\ \uparrow \\ 1 \\ \beta\text{-GlcNAc} \\ 4 \\ \uparrow \\ 1 \\ \beta\text{-Gal} \\ 3 \\ \uparrow \\ 2 \\ \alpha\text{-NeuNAc} \end{array}$$

**III**

$$\beta\text{-Gal-}(1\rightarrow 4)\text{-}\beta\text{-Glc-}(1\rightarrow]_n$$

$$\begin{array}{c} 3 \\ \uparrow \\ 1 \\ \boxed{\rightarrow 6)}\text{-}\beta\text{-GlcNAc} \\ 4 \\ \uparrow \\ 1 \\ \beta\text{-Gal} \\ 3 \\ \uparrow \\ 2 \\ \alpha\text{-NeuNAc} \end{array}$$

## FIGURE 2

| | |
|---|---|
| **Ia** | $[\rightarrow 4)\text{-}\beta\text{-D-Glc}p\text{-}(1\rightarrow 4)\text{-}\beta\text{-D-Gal}p\text{-}(1\rightarrow]_n$<br>3<br>↑<br>1<br>β-D-Glc$p$NAc<br>4<br>↑<br>1<br>β-D-Gal$p$<br>3<br>↑<br>2<br>α-D-Neu$p$NAc |
| **Ib** | $[\rightarrow 4)\text{-}\beta\text{-D-Glc}p\text{-}(1\rightarrow 4)\text{-}\beta\text{-D-Gal}p\text{-}(1\rightarrow]_n$<br>3<br>↑<br>1<br>β-D-Glc$p$NAc<br>3<br>↑<br>1<br>β-D-Gal$p$<br>3<br>↑<br>2<br>α-D-Neu$p$NAc |
| **II** | $[\rightarrow 4)\text{-}\beta\text{-D-Glc}p\text{NAc-}(1\rightarrow 3)\text{-}\beta\text{-D-Gal}p\text{-}(1\rightarrow 4)\text{-}\beta\text{-D-Glc}p\text{-}(1\rightarrow 3)\text{-}\beta\text{-D-Glc}p\text{-}(1\rightarrow 2)\text{-}\beta\text{-D-Gal}p\text{-}(1\rightarrow]_n$<br>6                            3<br>↑                            ↑<br>1                            2<br>β-D-Gal$p$               α-D-Neu$p$NAc |
| **III** | $\rightarrow 4)\text{-}\beta\text{-D-Glc}p\text{-}(1\rightarrow 6)\text{-}\beta\text{-D-Glc}p\text{NAc-}(1\rightarrow 3)\text{-}\beta\text{-D-Gal}p\text{-}(1\rightarrow$<br>4<br>↑<br>1<br>β-D-Gal$p$<br>3<br>↑<br>2<br>α-D-Neu$p$NAc |
| **V** | $\rightarrow 4)\text{-}\alpha\text{-D-Glc}p\text{-}(1\rightarrow 4)\text{-}\beta\text{-D-Gal}p\text{-}(1\rightarrow 4)\text{-}\beta\text{-D-Glc}p\text{-}(1\rightarrow$<br>6                  3<br>↑                  ↑<br>1                  1<br>β-D-Glc$p$NAc     β-D-Glc$p$<br>4<br>↑<br>1<br>β-D-Gal$p$<br>3<br>↑<br>2<br>α-D-Neu$p$NAc |

## FIGURE 3

→ 4)-α-D-Glc*p*-(1→4)-β-D-Glc*p*-(1→4)-β-D-Gal*p*-(1→4)-β-D-Glc*p*-(1→

6 ← 1   3 ← 1

β-D-Glc*p*NAc   β-D-Glc*p*

4 ← 1

β-D-Gal*p*

# FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006050341 A
- WO 9640795 A
- US 6027733 A
- US 6274144 B
- WO 2006082527 A
- US 61008941 B
- WO 2009081276 A
- EP 0477508 A
- US 5306492 A
- WO 9842721 A
- US 4356170 A
- WO 2006082530 A
- WO 2005000346 A
- EP 0372501 A
- EP 0378881 A
- EP 0427347 A
- WO 9317712 A
- WO 9403208 A
- WO 9858668 A
- EP 0471177 A
- WO 9101146 A
- EP 0594610 A
- WO 0056360 A
- WO 02091998 A
- WO 0172337 A
- WO 0061761 A
- WO 0033882 A
- WO 9942130 A
- WO 2004011027 A
- WO 9640242 A
- WO 0038711 A
- US 6146902 A
- WO IB200802690 A
- GB 0713880 A
- WO 2009010877 A
- WO 9924578 A
- WO 9936544 A
- WO 9957280 A
- WO 0022430 A
- WO 9629412 A
- WO 0152885 A
- WO 03007985 A
- WO 0202606 A
- WO 9927105 A
- WO 0027994 A
- WO 0037494 A
- WO 9928475 A
- WO 0234771 A
- WO 0056365 A
- WO 0053221 A
- US 6355271 B
- WO 0023105 A
- WO 9014837 A
- US 6299884 B
- US 6451325 B
- US 5057540 A
- WO 9633739 A
- EP 0109942 A
- WO 9611711 A
- WO 0007621 A
- WO 03024480 A
- WO 03024481 A
- EP 0689454 A
- WO 0226757 A
- WO 9962923 A
- WO 9840100 A
- US 6207646 B
- US 6239116 B
- US 6429199 B
- WO 0195935 A
- WO 03035836 A
- WO 9517211 A
- WO 9842375 A
- WO 9940936 A
- WO 9944636 A
- WO 9927960 A
- US 6090406 A
- US 5916588 A
- EP 0626169 A
- WO 9952549 A
- WO 0121207 A
- WO 0121152 A
- WO 0460308 A
- WO 0464759 A
- WO 9911241 A
- WO 9400153 A
- WO 9857659 A
- EP 0835318 A
- EP 0735898 A
- EP 0761231 A
- WO 03093306 A
- WO 2004018646 A
- WO 2004041157 A
- WO 2006069200 A
- GB 0802503 A
- WO 2009101403 A
- AU 748716
- WO 9851339 A

- US 61383668 B

- GB 1101665 A

**Non-patent literature cited in the description**

- **PAOLETTI et al.** *J Biol Chem,* 1990, vol. 265, 18278-83
- **WESSELS et al.** *J Clin Invest,* 1990, vol. 86, 1428-33
- **PAOLETTI et al.** *Infect Immun,* 1992, vol. 60, 4009-14
- **PAOLETTI et al.** *J Clin Invest,* 1992, vol. 89, 203-9
- **WESSELS et al.** *Proc Natl Acad Sci USA,* 1987, vol. 84, 9170-4
- **WANG et al.** *Vaccine,* 2003, vol. 21, 1112-7
- **WESSELS et al.** *Infect Immun,* 1993, vol. 61, 4760-6
- **WESSELS et al.** *J Infect Dis,* 1995, vol. 171, 879-84
- **BAKER et al.** *J Infect Dis,* 2004, vol. 189, 1103-12
- **PAOLETTI ; KASPER.** *Expert Opin Biol Ther,* 2003, vol. 3, 975-84
- **BRIGTSEN et al.** *JID,* 2002, vol. 185, 1277-84
- **GUTTORMSEN et al.** *Proc Natl Acad Sci U S A.,* 2008, vol. 105 (15), 5903-8
- **MICHON et al.** *Clin Vaccine Immunol.,* August 2006, vol. 13 (8), 936-43
- **LEWIS et al.** *PNAS USA,* 2004, vol. 101, 11123-8
- **WESSELS et al.** *Infect Immun,* 1989, vol. 57, 1089-94
- **RAMSAY et al.** *Lancet,* 2001, vol. 357 (9251), 195-196
- **LINDBERG.** *Vaccine,* 1999, vol. 17 (2), 28-36
- **BUTTERY ; MOXON.** *J R Coll Physicians Lond,* 2000, vol. 34, 163-68
- **AHMAD ; CHAPNICK.** *Infect Dis Clin North Am,* 1999, vol. 13, 113-33
- **GOLDBLATT.** *J. Med. Microbiol.,* 1998, vol. 47, 563-7
- **DICK et al.** Conjugate Vaccines. Karger, 1989, vol. 10, 48-114
- **HERMANSON.** Bioconjugae Techniques. Academic Press, 1996
- **ANONYMOUS.** *Research Disclosure,* January 2002, 453077
- **ANDERSON.** *Infect Immun,* 1983, vol. 39 (1), 233-238
- **ANDERSON et al.** *J Clin Invest,* 1985, vol. 76 (1), 52-59
- **FALUGI et al.** *Eur J Immunol,* 2001, vol. 31, 3816-24
- **BARALDO et al.** *Infect Immun,* 2004, vol. 72, 4884-87
- **KUO et al.** *Infect Immun,* 1995, vol. 63, 2706-13

- **LEI et al.** *Dev Biol (Basel),* 2000, vol. 103, 259-264
- **TETTELIN et al.** *Science,* 2000, vol. 287, 1809-1815
- **PIZZA et al.** *Science,* 2000, vol. 287, 1816-1820
- **BJUNE et al.** *Lancet,* 1991, vol. 338 (8775), 1093-1096
- **FUKASAWA et al.** *Vaccine,* 1999, vol. 17, 2951-2958
- **ROSENQVIST et al.** *Dev. Biol. Stand.,* 1998, vol. 92, 323-333
- **COSTANTINO et al.** *Vaccine,* 1992, vol. 10, 691-698
- **WATSON.** *Pediatr Infect Dis J,* 2000, vol. 19, 331-332
- **RUBIN.** *Pediatr Clin North Am,* 2000, vol. 47, 269-285
- **JEDRZEJAS.** *Microbiol Mol Biol Rev,* 2001, vol. 65, 187-207
- **BELL.** *Pediatr Infect Dis J,* 2000, vol. 19, 1187-1188
- **IWARSON.** *APMIS,* 1995, vol. 103, 321-326
- **GERLICH et al.** *Vaccine,* 1990, vol. 8, 63-68, 79-80
- **HSU et al.** *Clin Liver Dis,* 1999, vol. 3, 901-915
- **GUSTAFSSON et al.** *N. Engl. J. Med.,* 1996, vol. 334, 349-355
- **RAPPUOLI et al.** *TIBTECH,* 1991, vol. 9, 232-238
- Vaccines. 2004
- **KALMAN et al.** *Nature Genetics,* 1999, vol. 21, 385-389
- **READ et al.** *Nucleic Acids Res,* 2000, vol. 28, 1397-406
- **SHIRAI et al.** *J. Infect. Dis.,* 2000, vol. 181 (3), S524-S527
- **ROSS et al.** *Vaccine,* 2001, vol. 19, 4135-4142
- **SUTTER et al.** *Pediatr Clin North Am,* 2000, vol. 47, 287-308
- **ZIMMERMAN ; SPANN.** *Am Fam Physician,* 1999, vol. 59, 113-118, 125-126
- **DREESEN.** *Vaccine,* 1997, vol. 15, 2-6
- *MMWR Morb Mortal Wkly Rep,* 16 January 1998, vol. 47 (1), 12, , 19
- **MCMICHAEL.** *Vaccine,* 2000, vol. 19 (1), 5101-107
- **DALE.** *Infect Dis Clin North Am,* 1999, vol. 13, 227-43
- **FERRETTI et al.** *PNAS USA,* 2001, vol. 98, 4658-4663

- **KURODA et al.** *Lancet,* 2001, vol. 357 (9264), 1225-1240
- **ROBINSON ; TORRES.** *Seminars in Immunology,* 1997, vol. 9, 271-283
- **DONNELLY et al.** *Annu Rev Immunol,* 1997, vol. 15, 617-648
- **SCOTT-TAYLOR ; DALGLEISH.** *Expert Opin Investing Drugs,* 2000, vol. 9, 471-480
- **APOSTOLOPOULOS ; PLEBANSKI.** *Curr Opin Mol Ther,* 2000, vol. 2, 441-447
- **ILAN.** *Curr Opin Mol Ther,* 1999, vol. 1, 116-120
- **DUBENSKY et al.** *Mol Med,* 2000, vol. 6, 723-732
- **ROBINSON ; PERTMER.** *Adv Virus Res,* 2000, vol. 55, 1-74
- **DONNELLY et al.** *Am T Respir Crit Care Med,* 2000, vol. 162 (4), 190-193
- **DAVIS.** *Mt. Sinai J. Med.,* 1999, vol. 66, 84-90
- **PAOLETTI et al.** *Vaccine,* 2001, vol. 19, 2118-2126
- **GENNARO.** Remington: The Science and Practice of Pharmacy. 2000
- **PAOLETTI.** *Vaccine,* 2001, vol. 19 (15-16), 2118-26
- **ALMEIDA ; ALPAR.** *J. Drug Targeting,* 1996, vol. 3, 455-467
- **AGARWAL ; MISHRA.** *Indian J Exp Biol,* 1999, vol. 37, 6-16
- **JAKOBSEN et al.** *Infect Immun,* 2002, vol. 70, 1443-1452
- **BERGQUIST et al.** *APMIS,* 1998, vol. 106, 800-806
- **BAUDNER et al.** *Infect Immun,* 2002, vol. 70, 4785-4790
- **UGOZZOLI et al.** *J Infect Dis,* 2002, vol. 186, 1358-1361
- **PODDA.** *Vaccine,* 2001, vol. 19, 2673-80
- **FREY et al.** *Vaccine,* 2003, vol. 21, 4234-7
- **BARR et al.** *Advanced Drug Delivery Reviews,* 1998, vol. 32, 247-271
- **SJOLANDERET et al.** *Advanced Drug Delivery Reviews,* 1998, vol. 32, 321-338
- **NIIKURA et al.** *Virology,* 2002, vol. 293, 273-280
- **LENZ et al.** *J Immunol,* 2001, vol. 166, 5346-5355
- **PINTO et al.** *J Infect Dis,* 2003, vol. 188, 327-338
- **GERBER et al.** *Virol,* 2001, vol. 75, 4752-4760
- **GLUCK et al.** *Vaccine,* 2002, vol. 20, B10-B16
- **JOHNSON et al.** *Bioorg Med Chem Lett,* 1999, vol. 9, 2273-2278
- **EVANS et al.** *Expert Rev Vaccines,* 2003, vol. 2, 219-229
- **MERALDI et al.** *Vaccine,* 2003, vol. 21, 2485-2491
- **PAJAK et al.** *Vaccine,* 2003, vol. 21, 836-842
- **KANDIMALLA et al.** *Nucleic Acids Research,* 2003, vol. 31, 2393-2400
- **KRIEG.** *Nature Medicine,* 2003, vol. 9, 831-835
- **MCCLUSKIE et al.** *FEMS Immunology and Medical Microbiology,* 2002, vol. 32, 179-185
- **KANDIMALLA et al.** *Biochemical Society Transactions,* 2003, vol. 31, 654-658
- **BLACKWELL et al.** *J Immunol,* 2003, vol. 170, 4061-4068
- **KRIEG.** *Treads Immunol,* 2002, vol. 23, 64-65
- **KANDIMALLA et al.** *BBRC,* 2003, vol. 306, 948-953
- **BHAGAT et al.** *BBRC,* 2003, vol. 300, 853-861
- **BEIGNON et al.** *Infect Immun,* 2002, vol. 70, 3012-3019
- **PIZZA et al.** *Vaccine,* 2001, vol. 19, 2534-2541
- **PIZZA et al.** *Int J Med Microbiol,* 2000, vol. 290, 455-461
- **SCHARTON-KERSTEN et al.** *Infect Immun,* 2000, vol. 68, 5306-5313
- **RYAN et al.** *Infect Immun,* 1999, vol. 67, 6270-6280
- **PARTIDOS et al.** *Immunol Lett,* 1999, vol. 67, 209-216
- **PEPPOLONI et al.** *Expert Rev Vaccines,* 2003, vol. 2, 285-293
- **PINE et al.** *J Control Release,* 2002, vol. 85, 263-270
- **DOMENIGHINI et al.** *Mol Microbiol,* 1995, vol. 15, 1165-1167
- **SINGH et al.** *J Cont Release,* 2001, vol. 70, 267-276
- **ANDRIANOV et al.** *Biomaterials,* 1998, vol. 19, 109-115
- **PAYNE et al.** *Adv Drug Delivery Review,* 1998, vol. 31, 185-196
- **STANLEY.** *Clin Exp Dermatol,* 2002, vol. 27, 571-577
- **JONES.** *Curr Opin Investing Drugs,* 2003, vol. 4, 214-218
- **HENNINGS et al.** *J Infect Dis.,* 2001, vol. 183 (7), 1138-42
- **LIN et al.** *J Infect Dis.,* 2001, vol. 184 (8), 1022-8
- **LIN et al.** *J Infect Dis.,* 2004, vol. 190 (5), 928-34
- **GLEZEN ; ALPERS.** *Clin. Infect. Dis.,* 1999, vol. 28, 219-224
- **MADOFF et al.** *J Clin Invest,* 1994, vol. 94, 286-92
- **PAOLETTI et al.** *Infect Immun,* 1994, vol. 62, 3236-43
- **ADDERSON et al.** *Infection and Immunity,* 2003, vol. 71 (12), 6857-6863

- **GEYSEN et al.** *PNAS USA,* 1984, vol. 81, 3998-4002
- **CARTER.** *Methods Mol Biol,* 1994, vol. 36, 207-23
- **JAMESON, BA et al.** *CABIOS,* 1988, vol. 4 (1), 181-186
- **RADDRIZZANI ; HAMMER.** *Brief Bioinform,* 2000, vol. 1 (2), 179-89
- **DE LALLA et al.** *J. Immunol.,* 1999, vol. 163, 1725-29
- **BRUSIC et al.** *Bioinformatics,* 1998, vol. 14 (2), 121-30
- **MEISTER et al.** *Vaccine,* 1995, vol. 13 (6), 581-91
- **ROBERTS et al.** *AIDS Res Hum Retroviruses,* 1996, vol. 12 (7), 593-610
- **MAKSYUTOV ; ZAGREBELNAYA.** *Comput Appl Biosci,* 1993, vol. 9 (3), 291-7
- **FELLER ; DE LA CRUZ.** *Nature,* 1991, vol. 349 (6311), 720-1
- **HOPP.** *Peptide Research,* 1993, vol. 6, 183-190
- **WELLING et al.** *FEBS Lett.,* 1985, vol. 188, 215-218
- **DAVENPORT et al.** *Immunogenetics,* 1995, vol. 42, 392-297
- **BODANSZKY.** *Principles of Peptide Synthesis,* 1993, ISBN 0387564314
- **FIELDS et al.** *Meth Enzymol 289: Solid-Phase Peptide Synthesis,* 1997, ISBN 0121821900
- **CHAN ; WHITE.** *Fmoc Solid Phase Peptide Synthesis,* 2000, ISBN 0199637245
- **KULLMANN.** *Enzymatic Peptide Synthesis,* 1987, ISBN 0849368413
- **IBBA.** *Biotechnol Genet Eng Rev,* 1996, vol. 13, 197-216
- **NEEDLEMAN ; WUNSCH.** *J. Mol. Biol.,* 1970, vol. 48, 443-453
- **RICE et al.** *Trends Genet,* 2000, vol. 16, 276-277
- **HERZENBERG et al.** *Nature,* 1980, vol. 285, 664-667
- **SCHUTZE et al.** *J Immunol,* 1985, vol. 135, 2319-2322
- **DAGAN et al.** *Infect Immun,* 1998, vol. 66, 2093-2098
- **BARINGTON et al.** *Infect Immun,* 1994, vol. 62, 9-14
- **DI JOHN et al.** *Lancet,* 1989, vol. 2 (8677), 1415-8
- **GRANOFF et al.** *Vaccine,* 1993, vol. 1, 46-51
- **GRANOFF et al.** *JAMA,* 1994, vol. 272, 1116-1121
- **BARINGTON et al.** *Infect Immun,* 1993, vol. 61, 432-438
- **OLANDER et al.** *Vaccine,* 2001, vol. 20, 336-341
- **BURRAGE et al.** *Infect Immun,* 2002, vol. 70, 4946-4954
- **HOPPENBROUWERS et al.** *Vaccine,* 1999, vol. 17, 2588-98
- **PODDA et al.** *Vaccine,* 1991, vol. 9, 741-745
- **DARKES ; PLOSKER.** *Paediatr Drugs,* 2002, vol. 4, 609-630
- **JONES.** *Curr Opin Investing Drugs,* 2001, vol. 2, 47-49
- **DEL GUIDICE et al.** *Molecular Aspects of Medicine,* 1998, vol. 19, 1-70
- **RODEWALD et al.** *J Infect Dis.,* 1992, vol. 166 (3), 635-9
- **BALTIMORE et al.** *J Immunol.,* 1977, vol. 118 (2), 673-8
- **LANCASTER et al.** Meningitis and Septicaemia in Children and Adults. Royal Society of Medicine, 2009